(19) Europäisches Patentamt / European Patent Office / Office européen des brevets

(11) **EP 4 155 321 A1**

(12) **EUROPEAN PATENT APPLICATION**

(43) Date of publication:
29.03.2023  Bulletin 2023/13

(21) Application number: 22176993.8

(22) Date of filing: 02.06.2022

(51) International Patent Classification (IPC):
*C07K 16/28* (2006.01)     *A61K 39/395* (2006.01)

(52) Cooperative Patent Classification (CPC):
**A61P 43/00; A61K 39/395; C07K 16/00;**
**C07K 16/28; C07K 16/2851; C12N 5/10;**
A61K 2039/505; C07K 2317/33; C07K 2317/34

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: 04.06.2021  JP 2021094689

(71) Applicant: **CHUGAI SEIYAKU KABUSHIKI KAISHA**
**Tokyo 115-8543 (JP)**

(72) Inventors:
• **Takahashi, Noriyuki**
**138623 Synapse (SG)**
• **Kanamori, Masakazu**
**Shizuoka, 412-8513 (JP)**
• **Yang, Bo**
**138623 Synapse (SG)**

(74) Representative: **Lahrtz, Fritz**
**Simmons & Simmons LLP**
**Lehel Carré Thierschplatz 6**
**80538 München (DE)**

Remarks:
The complete document including Reference Table(s) and the Sequence Listing(s) can be downloaded from the EPO website

(54)  **ANTI-DDR2 ANTIBODIES AND USES THEREOF**

(57)     [Problems to be solved] An objective of the present invention is to provide anti-DDR2 antibodies that binds to Discoidin Domain Receptor 2 (DDR2).

[Means for solving the problem] The invention provides anti-DDR2 antibodies that binds to DDR2, and methods of using the same. The invention further provides isolated nucleic acids encoding the anti-DDR2 antibodies, host cells comprising the same, and methods of producing the anti-DDR2 antibodies. The invention further provides use of the anti-DDR2 antibodies in the manufacture of a medicament.

EP 4 155 321 A1

**Description**

[Technical Field]

The present invention relates to anti-DDR2 antibodies and methods of using the same.

[Background Art]

Discoidin Domain Receptors (DDRs) are widely expressed receptor tyrosine kinases (RTKs) in mammalian cells. Different from other RTKs, DDRs are activated by collagens, which is one of the major ECM components in tissue, but not diffusive cytokines or growth factors (NPL 1). In addition, DDRs are also unique group of RTKs as they displayed slow and sustained activation/ inactivation kinetics (NPL 1, NPL 2).

DDR family contains two members: DDR1 and DDR2 (NPL 1). The extracellular region of DDR2 composed of an N-terminal discoidin (DS) domain, followed by a DS-like (DSL) domain and an extracellular juxtamembrane (EJXM) domain (NPL 3, NPL 4). DS domain of DDR2 has been reported to be responsible for collagen binding (NPL 5). However, the biological functions of the remaining extracellular domains are still unknown.

DDR2 is implicated in cancers, osteoarthritis and various fibrotic diseases (NPL 6, NPL 7). Fibrosis is a complication of many chronic diseases and progressive fibrosis may lead to organ failure or death. Fibrosis is mainly characterized by accumulation of activated fibroblasts and the imbalanced deposition/degradation of extracellular matrix components, especially collagens (NPL 8). DDR2, as a collagen receptor, is highly expressed in mesenchymal original cells, such as fibroblasts, comparing with other types (NPL 9). Furthermore, DDR2 also plays a significant role in fibroblast proliferation, migration and activation (NPL 10, NPL 11) during fibrosis development. Antibodies against DDR2 used for cancer treatment has been obtained (PTL 1). However, whether DDR2 is an effective therapeutic target for various fibrotic diseases or not is not evaluated.

[Citation List]

[Patent Literature]

[PTL 1] WO2008070090

[Non Patent Literature]

[NPL 1] W. Vogel, G. D. Gish, F. Alves, T. Pawson, The discoidin domain receptor tyrosine kinases are activated by collagen. Molecular cell 1, 13-23 (1997).

[NPL 2] A. Shrivastava et al., An orphan receptor tyrosine kinase family whose members serve as nonintegrin collagen receptors. Molecular cell 1, 25-34 (1997).

[NPL 3] S. Baumgartner, K. Hofmann, R. Chiquet-Ehrismann, P. Bucher, The discoidin domain family revisited: new members from prokaryotes and a homology-based fold prediction. Protein science : a publication of the Protein Society 7, 1626-1631 (1998).

[NPL 4] M. A. Lemmon, J. Schlessinger, Cell signaling by receptor tyrosine kinases. Cell 141, 1117-1134 (2010).

[NPL 5] B. Leitinger, Molecular analysis of collagen binding by the human discoidin domain receptors, DDR1 and DDR2. Identification of collagen binding sites in DDR2. The Journal of biological chemistry 278, 16761-16769 (2003).

[NPL 6] C. M. Borza, A. Pozzi, Discoidin domain receptors in disease. Matrix biology : journal of the International Society for Matrix Biology 34, 185-192 (2014).

[NPL 7] B. Leitinger, Discoidin domain receptor functions in physiological and pathological conditions. Int Rev Cell Mol Biol 310, 39-87 (2014).

[NPL 8] T. A. Wynn, T. R. Ramalingam, Mechanisms of fibrosis: therapeutic translation for fibrotic disease. Nat Med 18, 1028-1040 (2012).

[NPL 9] F. Alves et al., Distinct structural characteristics of discoidin I subfamily receptor tyrosine kinases and complementary expression in human cancer. Oncogene 10, 609-618 (1995).

[NPL 10] E. Olaso et al., Discoidin Domain Receptor 2 Regulates Fibroblast Proliferation and Migration through the Extracellular Matrix in Association with Transcriptional Activation of Matrix Metalloproteinase-2 *. Journal of Biological Chemistry 277, 3606-3613 (2002).

[NPL 11] H. Zhao et al., Targeting of Discoidin Domain Receptor 2 (DDR2) Prevents Myofibroblast Activation and Neovessel Formation During Pulmonary Fibrosis. Mol Ther 24, 1734-1744 (2016).

[NPL 12] K. Roach et al., Evaluation of Pirfenidone and Nintedanib in a Human Lung Model of Fibrogenesis. Front. Pharmacol. 12, 679388 (2021).

[NPL 13] E. Conte et al., Molecular mechanisms of pirfenidone activity in human lung fibroblasts. European Respiratory Journal 44, P825 (2014).

[NPL 14] L. Wollin et al., Mode of action of nintedanib in the treatment of idiopathic pulmonary fibrosis. European Respiratory Journal 45, 1434-1445 (2015).

[NPL 15] L. Chen et al., Recent Advances in the Role of Discoidin Domain Receptor Tyrosine Kinase 1 and Discoidin Domain Receptor Tyrosine Kinase 2 in Breast and Ovarian Cancer. Front. Cell Dev. Biol 9, 747314 (2021).

[NPL 16] S. Jia et al., Discoidin Domain Receptor 2 Signaling Regulates Fibroblast Apoptosis through PDK1/Akt. American Journal of Respiratory Cell and Molecular Biology 59(3), 295-305 (2018).

[NPL 17] WA Denny et al., Inhibitors of Discoidin Domain Receptor (DDR) Kinases for Cancer and Inflammation. Biomolecules 11(11), 1671 (2021).

[NPL 18] J Tashiro et al., Exploring Animal Models that Resemble Idiopathic Pulmonary Fibrosis. Front Med 4, 118 (2017).

[NPL 19] T Liu et al., The Bleomycin Model of Pulmonary Fibrosis. Methods Mol Biol 1627, 27-42 (2017).

[NPL 20] D Toren et al., Systems biology analysis of lung fibrosis-related genes in the bleomycin mouse model. Sci Rep 11, 19269 (2021).

[Summary of Invention]

[Technical Problem]

The invention provides anti-DDR2 antibodies and methods of using the same.

[Solution to Problem]

The present disclosure provides the following:

[A1] An isolated antibody that binds to Discoidin Domain Receptor 2 (DDR2)

[A2] An isolated antibody that binds to extracellular region of DDR2.

[A3] An isolated antibody that binds to discoidin domain (DS domain), discoidin-like domain (DSL domain) or extracellular juxtamembrane domain (EJXM domain) of DDR2.

[A4] The antibody of any one of [A1] to [A3], wherein the antibody binds to human DDR2 and/or mouse DDR2.

[A5] The antibody of any one of [A1] to [A3], wherein the antibody binds to human DDR2 comprising the amino acid sequence shown in SEQ ID NO: 78 and/or mouse DDR2 comprising the amino acid sequence shown in SEQ ID NO: 77.

[A6] The antibody of any one of [A1] to [A5], wherein the antibody does not bind to DDR1.

[A7] The antibody of any one of [A1] to [A5], wherein the antibody does not bind to human DDR1a, human DDR1b, human DDR1c, human DDR1d, human DDR1e and/or mouse DDR1.

[A8] The antibody of any one of [A6] or [A7], wherein the antibody does not bind to human DDRIa comprising the amino acid sequence shown in SEQ ID NO: 81, human DDR1b comprising the amino acid sequence shown in SEQ ID NO: 80, human DDR1c comprising the amino acid sequence shown in SEQ ID NO: 82, human DDR1d comprising the amino acid sequence shown in SEQ ID NO: 83, human DDR1e comprising the amino acid sequence shown in SEQ ID NO: 84 and/or mouse DDR1 comprising the amino acid sequence shown in SEQ ID NO: 79.

[A9] The antibody of any one of [A1] to [A8], wherein the antibody binds to the region of amino acid 22 to 399 of SEQ ID NO: 78.

[A10] The antibody of any one of [A1] to [A9], wherein the antibody binds to the region of amino acid 22 to 191, or the region of amino acid 32 to 184, or the region of amino acid 30 to 185, or the region of amino acid 28-187 of SEQ ID NO: 78.

[A11] The antibody of any one of [A1] to [A9], wherein the antibody binds to the region of amino acid 200 to 367, or the region of amino acid 188 to 367 of SEQ ID NO: 78.

[A12] The antibody of any one of [A1] to [A9], wherein the antibody binds to the region of amino acid 368 to 399 of SEQ ID NO: 78.

[A13] The antibody of any one of [A1] to [A12], wherein the antibody binds to the region of amino acid 24 to 399, or the region of amino acid 22 to 399 of SEQ ID NO: 77.

[A14] The antibody of any one of [A1] to [A13], wherein the antibody binds to the region of amino acid 24 to 191, or the region of amino acid 32 to 184, or the region of amino acid 30 to 185, or the region of amino acid 28 to 187 of SEQ ID NO: 77.

[A15] The antibody of any one of [A1] to [A13], wherein the antibody binds to the region of amino acid 200 to 367, or the region of amino acid 188 to 367 of SEQ ID NO: 77.

[A16] The antibody of any one of [A1] to [A13], wherein the antibody binds to the region of amino acid 368 to 399 of SEQ ID NO: 77.

[A17] The antibody of any one of [A1] to [A13], wherein the antibody binds to the region of amino acid 201 to 393 of SEQ ID NO: 77.

[A18] The antibody of any one of [A1] to [A13] wherein the antibody binds to the region of amino acid 24 to 200 of SEQ ID NO: 77.

[A19] The antibody of any one of [A1] to [A18], wherein the antibody does not bind to the region of amino acid 22 to 415 of SEQ ID NO: 79.

[A20] The antibody of any one of [A1] to [A19], wherein the antibody does not bind to the region of amino acid 21 to 417 of SEQ ID NO: 81.

[A21] The antibody of any one of [A1] to [A20], wherein the antibody does not bind to the region of amino acid 21 to 417 of SEQ ID NO: 80.

[A22] The antibody of any one of [A1] to [A21] wherein the antibody does not bind to the region of amino acid 21 to 417 of SEQ ID NO: 82.

[A23] The antibody of any one of [A1] to [A22], wherein the antibody does not bind to the region of amino acid 21 to 417 of SEQ ID NO: 83.

[A24] The antibody of any one of [A1] to [A23], wherein the antibody does not bind to the region of amino acid 21 to 417 of SEQ ID NO: 84.

[A25] The antibody of any one of [A1] to [A24], wherein the antibody comprises any one of (a1) to (a9) below: (a1) HVR-H1 comprising the amino acid sequence of SEQ ID NO: 2, HVR-H2 comprising the amino acid sequence of SEQ ID NO:3, and HVR-H3 comprising the amino acid sequence of SEQ ID NO:4; (a2) HVR-H1 comprising the amino acid sequence of SEQ ID NO:10, HVR-H2 comprising the amino acid sequence of SEQ ID NO:11, and HVR-H3 comprising the amino acid sequence of SEQ ID NO:12; (a3) HVR-H1 comprising the amino acid sequence of SEQ ID NO:18, HVR-H2 comprising the amino acid sequence of SEQ ID NO:19, and HVR-H3 comprising the amino acid sequence of SEQ ID NO:20; (a4) HVR-H1 comprising the amino acid sequence of SEQ ID NO:26, HVR-H2 comprising the amino acid sequence of SEQ ID NO:27, and HVR-H3 comprising the amino acid sequence of SEQ ID NO:28; (a5) HVR-H1 comprising the amino acid sequence of SEQ ID NO:34, HVR-H2 comprising the amino acid sequence of SEQ ID NO:35, and HVR-H3 comprising the amino acid sequence of SEQ ID NO:36; (a6) HVR-H1 comprising the amino acid sequence of SEQ ID NO:42, HVR-H2 comprising the amino acid sequence of SEQ ID NO:43, and HVR-H3 comprising the amino acid sequence of SEQ ID NO:44; (a7) HVR-H1 comprising the amino acid sequence of SEQ ID NO:50, HVR-H2 comprising the amino acid sequence of SEQ ID NO:51, and HVR-H3 comprising the amino acid sequence of SEQ ID NO:52; (a8) HVR-H1 comprising the amino acid sequence of SEQ ID NO:58, HVR-H2 comprising the amino acid sequence of SEQ ID NO:59, and HVR-H3 comprising the amino acid sequence of SEQ ID NO:60; (a9) HVR-H1 comprising the amino acid sequence of SEQ ID NO:66, HVR-H2 comprising the amino acid sequence of SEQ ID NO:67, and HVR-H3 comprising the amino acid sequence of SEQ ID NO:68.

[A26] The antibody of [A25], further comprising any one of (b1) to (b9) below: (b1) HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8; (b2) HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16; (b3) HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24; (b4) HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32; (b5) HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40; (b6) HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48; (b7) HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56; (b8) HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; (b9) HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

[A27] The antibody of any one of [A1] to [A26], wherein the antibody is any one of (c1) to (c11) below: (c1) an antibody that comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8; (c2) an antibody that comprises HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16; (c3) an antibody that comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24; (c4) an antibody that comprises HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32; (c5) an antibody that comprises HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40; (c6) an antibody that comprises HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48; (c7) an antibody that comprises HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56; (c8) an antibody that comprises HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; (c9) an antibody that comprises HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72; (c10) an antibody that binds to the same epitope as any one of (c1) to (c9); (c11) an antibody that competes with any one of (c1) to (c9) in binding towards DDR2.

[A28] The antibody of any one of [A1] to [A27], wherein the antibody is any one of (d1) to (d10) below: (d1) an antibody comprising a VH sequence having the amino acid sequence of SEQ ID NO:1 and a VL sequence having the amino acid sequence of SEQ ID NO:5; (d2) an antibody comprising a VH sequence having the amino acid sequence of SEQ ID NO:9 and a VL sequence having the amino acid sequence of SEQ ID NO: 13; (d3) an antibody comprising a VH sequence having the amino acid sequence of SEQ ID NO: 17 and a VL sequence having the amino acid sequence of SEQ ID NO:21; (d4) an antibody comprising a VH sequence having the amino acid sequence of SEQ ID NO:25 and a VL sequence having the amino acid sequence of SEQ ID NO:29; (d5) an antibody comprising a VH sequence having the amino acid sequence of SEQ ID NO:33 and a VL sequence having the amino acid sequence of SEQ ID NO:37; (d6) an antibody comprising a VH sequence having the amino acid sequence of SEQ ID NO:41 and a VL sequence having the amino acid sequence of SEQ ID NO:45; (d7) an antibody comprising a VH sequence having the amino acid sequence of SEQ ID NO:49 and a VL sequence having the amino acid sequence of SEQ ID NO:53; (d8) an antibody comprising a VH sequence having the amino acid sequence of SEQ ID NO:57 and a VL sequence having the amino acid sequence of SEQ ID NO:61; (d9) an antibody comprising a VH sequence having the amino acid sequence of SEQ ID NO:65 and a VL sequence having the amino acid sequence of SEQ ID NO:69; (d10) an antibody comprising (i) a VH sequence having at least 95% sequence identity to any one of the amino acid sequence of SEQ ID NO:1, 9, 17, 25, 33, 41, 49, 57 and 65; (ii) a VL sequence having at least 95% sequence identity to any one of the amino acid sequence of SEQ ID NO:5, 13, 21, 29, 37, 45, 53, 61 and 69; or (iii) a VH sequence as in (i) and a VL sequence as in (ii).

[A29] The antibody of any of [A1] to [A28], wherein the antibody is any one of (e1) to (e10) below: (e1) an antibody comprising a heavy chain having the amino acid sequence of SEQ ID NO: 85 and a light chain having the amino acid sequence of SEQ ID NO: 86; (e2) an antibody comprising a heavy chain having the amino acid sequence of SEQ ID NO: 87 and a light chain having the amino acid sequence of SEQ ID NO: 88; (e3) an antibody comprising a heavy chain having the amino acid sequence of SEQ ID NO: 89 and a light chain having the amino acid sequence of SEQ ID NO: 90; (e4) an antibody comprising a heavy chain having the amino acid sequence of SEQ ID NO: 91 and a light chain having the amino acid sequence of SEQ ID NO: 92; (e5) an antibody comprising a heavy chain having the amino acid sequence of SEQ ID NO: 93 and a light chain having the amino acid sequence of SEQ ID NO: 94; (e6) an antibody comprising a heavy chain having the amino acid sequence of SEQ ID NO: 95 and a light chain having the amino acid sequence of SEQ ID NO: 96; (e7) an antibody comprising a heavy chain having the amino acid sequence of SEQ ID NO: 97 and a light chain having the amino acid sequence of SEQ ID NO: 98; (e8) an antibody comprising a heavy chain having the amino acid sequence of SEQ ID NO: 99 and a light chain having the amino acid sequence of SEQ ID NO: 100; (e9) an antibody comprising a heavy chain having the amino acid sequence of SEQ ID NO: 101 and a light chain having the amino acid sequence of SEQ ID NO: 102; (e10) an antibody comprising (i) a heavy chain having at least 95% sequence identity to any one of the amino acid sequence of SEQ ID NO: 85, 87, 89, 91, 93, 95, 97, 99, and 101; (ii) a light chain having at least 95% sequence identity to any one of the amino acid sequence of SEQ ID NO: 86, 88, 90, 92, 94, 96, 98, 100, and 102; or (iii) a heavy chain sequence as in (i) and a light chain sequence as in (ii).

[A30] The antibody of any one of [A1] to [A29], which is a monoclonal antibody.

[A31] The antibody of any one of [A1] to [A30], which is a human, humanized, or chimeric antibody.

[A32] The antibody of any one of [A1] to [A31], which is an antibody fragment that binds to DDR2.

[A33] An isolated antibody that binds to Discoidin Domain Receptor 2 (DDR2) and binds to the same epitope as an antibody comprising any one of the following:

(c1) HVR-H1 comprising the amino acid sequence of SEQ ID NO: 2, HVR-H2 comprising the amino acid sequence of SEQ ID NO:3, and HVR-H3 comprising the amino acid sequence of SEQ ID NO:4, and HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8;
(c2) HVR-H1 comprising the amino acid sequence of SEQ ID NO:10, HVR-H2 comprising the amino acid sequence of SEQ ID NO:11, and HVR-H3 comprising the amino acid sequence of SEQ ID NO:12; and HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16;
(c3) HVR-H1 comprising the amino acid sequence of SEQ ID NO:18, HVR-H2 comprising the amino acid sequence of SEQ ID NO:19, and HVR-H3 comprising the amino acid sequence of SEQ ID NO:20; and HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24;
(c4) HVR-H1 comprising the amino acid sequence of SEQ ID NO:26, HVR-H2 comprising the amino acid sequence of SEQ ID NO:27, and HVR-H3 comprising the amino acid sequence of SEQ ID NO:28; and HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32;
(c5) HVR-H1 comprising the amino acid sequence of SEQ ID NO:34, HVR-H2 comprising the amino acid sequence of SEQ ID NO:35, and HVR-H3 comprising the amino acid sequence of SEQ ID NO:36; and HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40;
(c6) HVR-H1 comprising the amino acid sequence of SEQ ID NO:42, HVR-H2 comprising the amino acid sequence of SEQ ID NO:43, and HVR-H3 comprising the amino acid sequence of SEQ ID NO:44; and HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48;
(c7) HVR-H1 comprising the amino acid sequence of SEQ ID NO:50, HVR-H2 comprising the amino acid sequence of SEQ ID NO:51, and HVR-H3 comprising the amino acid sequence of SEQ ID NO:52; and HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56;
(c8) HVR-H1 comprising the amino acid sequence of SEQ ID NO:58, HVR-H2 comprising the amino acid sequence of SEQ ID NO:59, and HVR-H3 comprising the amino acid sequence of SEQ ID NO:60; and HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64;
(c9) HVR-H1 comprising the amino acid sequence of SEQ ID NO:66, HVR-H2 comprising the amino acid sequence of SEQ ID NO:67, and HVR-H3 comprising the amino acid sequence of SEQ ID NO:68; and HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

[A34] An isolated antibody that binds to Discoidin Domain Receptor 2 (DDR2) and competes with any one of (c1) to (c9) in binding towards DDR2:

(c1) HVR-H1 comprising the amino acid sequence of SEQ ID NO: 2, HVR-H2 comprising the amino acid sequence of SEQ ID NO:3, and HVR-H3 comprising the amino acid sequence of SEQ ID NO:4, and HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8;
(c2) HVR-H1 comprising the amino acid sequence of SEQ ID NO:10, HVR-H2 comprising the amino acid sequence of SEQ ID NO:11, and HVR-H3 comprising the amino acid sequence of SEQ ID NO:12; and HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16;
(c3) HVR-H1 comprising the amino acid sequence of SEQ ID NO:18, HVR-H2 comprising the amino acid sequence of SEQ ID NO:19, and HVR-H3 comprising the amino acid sequence of SEQ ID NO:20; and HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24;

(c4) HVR-H1 comprising the amino acid sequence of SEQ ID NO:26, HVR-H2 comprising the amino acid sequence of SEQ ID NO:27, and HVR-H3 comprising the amino acid sequence of SEQ ID NO:28; and HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32;

(c5) HVR-H1 comprising the amino acid sequence of SEQ ID NO:34, HVR-H2 comprising the amino acid sequence of SEQ ID NO:35, and HVR-H3 comprising the amino acid sequence of SEQ ID NO:36; and HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40;

(c6) HVR-H1 comprising the amino acid sequence of SEQ ID NO:42, HVR-H2 comprising the amino acid sequence of SEQ ID NO:43, and HVR-H3 comprising the amino acid sequence of SEQ ID NO:44; and HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48;

(c7) HVR-H1 comprising the amino acid sequence of SEQ ID NO:50, HVR-H2 comprising the amino acid sequence of SEQ ID NO:51, and HVR-H3 comprising the amino acid sequence of SEQ ID NO:52; and HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56;

(c8) HVR-H1 comprising the amino acid sequence of SEQ ID NO:58, HVR-H2 comprising the amino acid sequence of SEQ ID NO:59, and HVR-H3 comprising the amino acid sequence of SEQ ID NO:60; and HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64;

(c9) HVR-H1 comprising the amino acid sequence of SEQ ID NO:66, HVR-H2 comprising the amino acid sequence of SEQ ID NO:67, and HVR-H3 comprising the amino acid sequence of SEQ ID NO:68; and HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

[A35] An isolated antibody that binds to Discoidin Domain Receptor 2 (DDR2) and binds to the same epitope as an antibody of the following:

(d1) an antibody comprising a VH sequence having the amino acid sequence of SEQ ID NO: 1 and a VL sequence having the amino acid sequence of SEQ ID NO:5;

(d2) an antibody comprising a VH sequence having the amino acid sequence of SEQ ID NO:9 and a VL sequence having the amino acid sequence of SEQ ID NO: 13;

(d3) an antibody comprising a VH sequence having the amino acid sequence of SEQ ID NO: 17 and a VL sequence having the amino acid sequence of SEQ ID NO:21;

(d4) an antibody comprising a VH sequence having the amino acid sequence of SEQ ID NO:25 and a VL sequence having the amino acid sequence of SEQ ID NO:29;

(d5) an antibody comprising a VH sequence having the amino acid sequence of SEQ ID NO:33 and a VL sequence having the amino acid sequence of SEQ ID NO:37;

(d6) an antibody comprising a VH sequence having the amino acid sequence of SEQ ID NO:41 and a VL sequence having the amino acid sequence of SEQ ID NO:45;

(d7) an antibody comprising a VH sequence having the amino acid sequence of SEQ ID NO:49 and a VL sequence having the amino acid sequence of SEQ ID NO:53;

(d8) an antibody comprising a VH sequence having the amino acid sequence of SEQ ID NO:57 and a VL sequence having the amino acid sequence of SEQ ID NO:61;

(d9) an antibody comprising a VH sequence having the amino acid sequence of SEQ ID NO:65 and a VL sequence having the amino acid sequence of SEQ ID NO:69.

[A36] An isolated antibody that binds to Discoidin Domain Receptor 2 (DDR2) and competes with any one of (d1) to (d9) in binding towards DDR2:

(d1) an antibody comprising a VH sequence having the amino acid sequence of SEQ ID NO: 1 and a VL sequence having the amino acid sequence of SEQ ID NO:5;

(d2) an antibody comprising a VH sequence having the amino acid sequence of SEQ ID NO:9 and a VL sequence having the amino acid sequence of SEQ ID NO: 13;

(d3) an antibody comprising a VH sequence having the amino acid sequence of SEQ ID NO: 17 and a VL sequence having the amino acid sequence of SEQ ID NO:21;

(d4) an antibody comprising a VH sequence having the amino acid sequence of SEQ ID NO:25 and a VL sequence having the amino acid sequence of SEQ ID NO:29;

(d5) an antibody comprising a VH sequence having the amino acid sequence of SEQ ID NO:33 and a VL sequence having the amino acid sequence of SEQ ID NO:37;

(d6) an antibody comprising a VH sequence having the amino acid sequence of SEQ ID NO:41 and a VL sequence having the amino acid sequence of SEQ ID NO:45;

(d7) an antibody comprising a VH sequence having the amino acid sequence of SEQ ID NO:49 and a VL sequence having the amino acid sequence of SEQ ID NO:53;

(d8) an antibody comprising a VH sequence having the amino acid sequence of SEQ ID NO:57 and a VL sequence having the amino acid sequence of SEQ ID NO:61;

(d9) an antibody comprising a VH sequence having the amino acid sequence of SEQ ID NO:65 and a VL sequence having the amino acid sequence of SEQ ID NO:69.

[A37] An isolated antibody that binds to Discoidin Domain Receptor 2 (DDR2) and binds to the same epitope as an antibody of the following:

(e1) an antibody comprising a heavy chain having the amino acid sequence of SEQ ID NO: 85 and a light chain having the amino acid sequence of SEQ ID NO: 86;

(e2) an antibody comprising a heavy chain having the amino acid sequence of SEQ ID NO: 87 and a light chain having the amino acid sequence of SEQ ID NO: 88;

(e3) an antibody comprising a heavy chain having the amino acid sequence of SEQ ID NO: 89 and a light chain having the amino acid sequence of SEQ ID NO: 90;

(e4) an antibody comprising a heavy chain having the amino acid sequence of SEQ ID NO: 91 and a light chain having the amino acid sequence of SEQ ID NO: 92;

(e5) an antibody comprising a heavy chain having the amino acid sequence of SEQ ID NO: 93 and a light chain having the amino acid sequence of SEQ ID NO: 94;

(e6) an antibody comprising a heavy chain having the amino acid sequence of SEQ ID NO: 95 and a light chain having the amino acid sequence of SEQ ID NO: 96;

(e7) an antibody comprising a heavy chain having the amino acid sequence of SEQ ID NO: 97 and a light chain having the amino acid sequence of SEQ ID NO: 98;

(e8) an antibody comprising a heavy chain having the amino acid sequence of SEQ ID NO: 99 and a light chain having the amino acid sequence of SEQ ID NO: 100;

(e9) an antibody comprising a heavy chain having the amino acid sequence of SEQ ID NO: 101 and a light chain having the amino acid sequence of SEQ ID NO: 102.

[A38] An isolated antibody that binds to Discoidin Domain Receptor 2 (DDR2) and competes with any one of (e1) to (e9) in binding towards DDR2:

(e1) an antibody comprising a heavy chain having the amino acid sequence of SEQ ID NO: 85 and a light chain having the amino acid sequence of SEQ ID NO: 86;

(e2) an antibody comprising a heavy chain having the amino acid sequence of SEQ ID NO: 87 and a light chain having the amino acid sequence of SEQ ID NO: 88;

(e3) an antibody comprising a heavy chain having the amino acid sequence of SEQ ID NO: 89 and a light chain having the amino acid sequence of SEQ ID NO: 90;

(e4) an antibody comprising a heavy chain having the amino acid sequence of SEQ ID NO: 91 and a light chain having the amino acid sequence of SEQ ID NO: 92;

(e5) an antibody comprising a heavy chain having the amino acid sequence of SEQ ID NO: 93 and a light chain having the amino acid sequence of SEQ ID NO: 94;

(e6) an antibody comprising a heavy chain having the amino acid sequence of SEQ ID NO: 95 and a light chain having the amino acid sequence of SEQ ID NO: 96;

(e7) an antibody comprising a heavy chain having the amino acid sequence of SEQ ID NO: 97 and a light chain having the amino acid sequence of SEQ ID NO: 98;

(e8) an antibody comprising a heavy chain having the amino acid sequence of SEQ ID NO: 99 and a light chain having the amino acid sequence of SEQ ID NO: 100;

(e9) an antibody comprising a heavy chain having the amino acid sequence of SEQ ID NO: 101 and a light chain having the amino acid sequence of SEQ ID NO: 102.

[A39] The antibody of [A33] or [A35] or [A37], wherein the epitope is determined by X-ray diffraction analysis.

[A40] The antibody of any of [A1] to [A39], wherein the antibody binds DDR2 at an epitope comprising all or part of a sequence defined by amino acids 31 to 179 of SEQ ID NO: 77 or 78.

[A41] The antibody of [A40], wherein the antibody binds to at least one or more, or all, of the amino acids R31, P33, S37, D64, S65, E66, Q103, H106, A107, G108, H110, S145, N146, Y148, D149, V150, F151, L152, D154 and R179 of SEQ ID NO: 77.

[A42] The antibody of [A41], wherein the antibody binds to at least one or more, or all, of the amino acids P33, S37, D64, S65, E66, Q103, H106, G108, H110, S145, N146, Y148, D149, V150, F151, and L152 of SEQ ID NO: 77.

[A43] The antibody of [A40], wherein the antibody binds to at least one or more, or all, of the amino acids R31, P33, S37, D64, S65, E66, Q103, H106, A107, G108, H110, S145, N146, Y148, D149, 1150, F151, L152, D154 and R179 of SEQ ID NO: 78.

[A44] The antibody of [A43], wherein the antibody binds to at least one or more, or all, of the amino acids P33, S37, D64, S65, E66, Q103, H106, G108, H110, S145, N146, Y148, D149, 1150, F151, and L152 of SEQ ID NO: 78.

The present disclosure also provides the following:

[B1]    Isolated nucleic acid encoding the antibody of any one of [A1] to [A44].
[B2]    A host cell comprising the nucleic acid of [B1].
[B3]    A method of producing an antibody comprising culturing the host cell of [B2] so that the antibody is produced.
[B4]    The method of [B3], further comprising recovering the antibody from the host cell.
[B5]    An immunoconjugate comprising the antibody of any one of [A1] to [A44] and a cytotoxic agent.

The present disclosure further provides the following:

[C1]    A pharmaceutical formulation comprising the antibody of any one of [A1] to [A44] and a pharmaceutically acceptable carrier.
[C2]    The antibody of any one of [A1] to [A44] for use as a medicament.
[C3]    The antibody of any one of [A1] to [A44] for use in reducing collagen type 1 alpha 1 (Col1a1) mRNA level and/or fibronectin 1 (Fnl) mRNA level in a subject.
[C4]    The antibody of any one of [A1] to [A44] for use in treating fibrotic disease.
[C5]    The antibody for use of any one of [A1] to [A44] wherein the fibrotic disease is idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis.
[C6]    The antibody for use of any one of [A1] to [A44] wherein the fibrotic disease is idiopathic pulmonary fibrosis (IPF), lung fibrosis/pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), or cystic fibrosis.
[C7]    The antibody for use of any one of [A1] to [A44] wherein the fibrotic disease is renal fibrosis, nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis.
[C8]    Use of the antibody of any one of [A1] to [A44] in the manufacture of a medicament.

(continued)

[C9] Use of the antibody of any one of [A1] to [A44] in the manufacture of a medicament for reduction of collagen type 1 alpha 1 (Col1a1) mRNA level and/or fibronectin 1 (Fnl) mRNA level in a subject.

[C10] Use of the antibody of any one of [A1] to [A44] in the manufacture of a medicament for treatment of fibrotic disease.

[C11] The use of any one of [A1] to [A44] wherein the fibrotic disease is idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis.

[C12] The use of any one of [A1] to [A44] wherein the fibrotic disease is idiopathic pulmonary fibrosis (IPF), lung fibrosis/pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), or cystic fibrosis.

[C13] The use of any one of [A1] to [A44] wherein the fibrotic disease is renal fibrosis, nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis.

[C14] A method for reducing collagen type 1 alpha 1 (Col1a1) mRNA level and/or fibronectin 1 (Fnl) mRNA level in an individual, comprising administering to the individual an effective amount of the antibody of any one of [A1] to [A44].

[C15] A method of treating an individual having fibrotic disease comprising administering to the individual an effective amount of the antibody of any one of [A1] to [A44].

[C16] The method of [C15], wherein the fibrotic disease is idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis.

[C17] The method of [C15], wherein the fibrotic disease is idiopathic pulmonary fibrosis (IPF), lung fibrosis/ pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), or cystic fibrosis.

[C18] The method of [C15], wherein the fibrotic disease is renal fibrosis, nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis.

The present disclosure also provides the following:

[D1]     An isolated antibody that binds to Discoidin Domain Receptor 2 (DDR2).

[D2]     The isolated antibody of [D1], wherein the antibody binds to the extracellular region of DDR2, in particular wherein the antibody binds to discoidin domain (DS domain), discoidin-like domain (DSL domain) or extracellular juxtamembrane domain (EJXM domain) of DDR2.

[D3]     The antibody of any one of [D1] or [D2], wherein the antibody does not bind to DDR1.

[D4]     The antibody of any one of [D1] to [D3], wherein the antibody binds DDR2 at an epitope comprising all or part of a sequence defined by amino acids 31 to 179 of SEQ ID NO: 77 or 78.

[D5]     The antibody of [D4], wherein the antibody binds to a discontinuous epitope comprising amino acids R31, P33, S37, D64, S65, E66, Q103, H106, A107, G108, H110, S145, N146, Y148, D149, V150, F151, L152, D154 and R179 of SEQ ID NO: 77, or R31, P33, S37, D64, S65, E66, Q103, H106, A107, G108, H110, S145, N146, Y148, D149, 1150, F151, L152, D154 and R179 of SEQ ID NO: 78, in particular wherein the antibody binds to a discontinuous epitope comprising amino acids P33, S37, D64, S65, E66, Q103, H106, G108, H110, S145, N146, Y148, D149, V150, F151, and L152 of SEQ ID NO: 77, or P33, S37, D64, S65, E66, Q103, H106, G108, H110, S145, N146, Y148, D149, 1150, F151, and L152 of SEQ ID NO: 78.

[D6]     The antibody of any one of [D1] to [D5], wherein the antibody comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8.

[D7]     The antibody of [D6], comprising a VH sequence having the amino acid sequence of SEQ ID NO:1 and a VL sequence having the amino acid sequence of SEQ ID NO:5.

[D8]     An antibody binding to the same epitope as the antibody of any of [D6] or [D7] or which competes with the antibody of any of [D6] or [D7] in binding towards DDR2.

[D9]     The antibody of any one of claims [D1] to [D8], which is a human, humanized, or chimeric antibody.

[D10]    An isolated nucleic acid encoding the antibody of any one of claims [D1] to [D9].

[D11]    A host cell comprising the nucleic acid of [D10].

[D12]    A method of producing an antibody comprising culturing the host cell of [D11] so that the antibody is produced.

[D13]    The antibody of any one of [D1] to [D9] for use as a medicament.

[D14]    The antibody of any one of [D1] to [D9] for use in reducing collagen type 1 alpha 1 (Col1a1) mRNA level and/or fibronectin 1 (Fnl) mRNA level in a subject.

[D15]    The antibody of any one of [D1] to [D9] for use in treating fibrotic disease.

[D16]    The antibody for use of [D15], wherein the fibrotic disease is idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis, in particular wherein the fibrotic disease is a lung fibrotic disease, more in particular idiopathic pulmonary fibrosis (IPF).

[Brief Description of Drawings]

[Fig. 1]

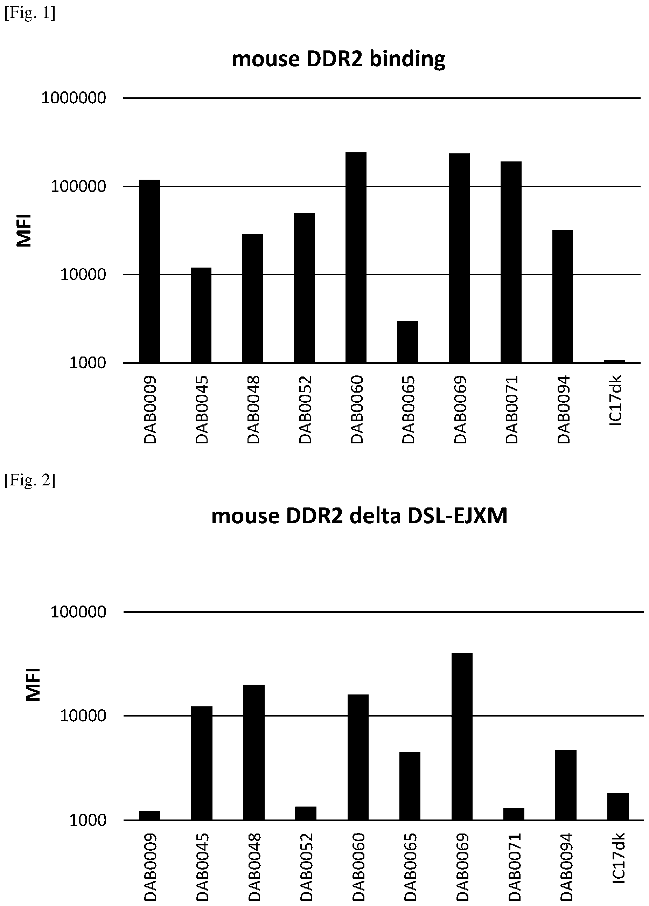

Figure 1 shows the result of binding analysis of the antibodies towards mouse DDR2. Mean Fluorescent Intensity (MFI) of each antibody was measured by flow cytometry using Ba/F3 cell line that stably expresses full length mouse DDR2. Names under the horizontal axis are names of the tested antibodies. IC17dK is an anti-KLH antibody used as a negative control.

[Fig. 2]

Figure 2 shows the result of binding analysis of the antibodies towards mouse DDR2 delta DSL-EJXM. Mean Fluorescent Intensity (MFI) of each antibody was measured by flow cytometry using Ba/F3 cell line that stably expresses mouse DDR2 fragment in which Discoidin-like (DSL) domain and extracellular juxtamembrane (EJXM) region were deleted from full length mouse DDR2 (mouse DDR2 delta DSL-EJXM). Names under the horizontal axis are names of the tested antibodies. IC17dK is an anti-KLH antibody used as a negative control.

[Fig. 3]

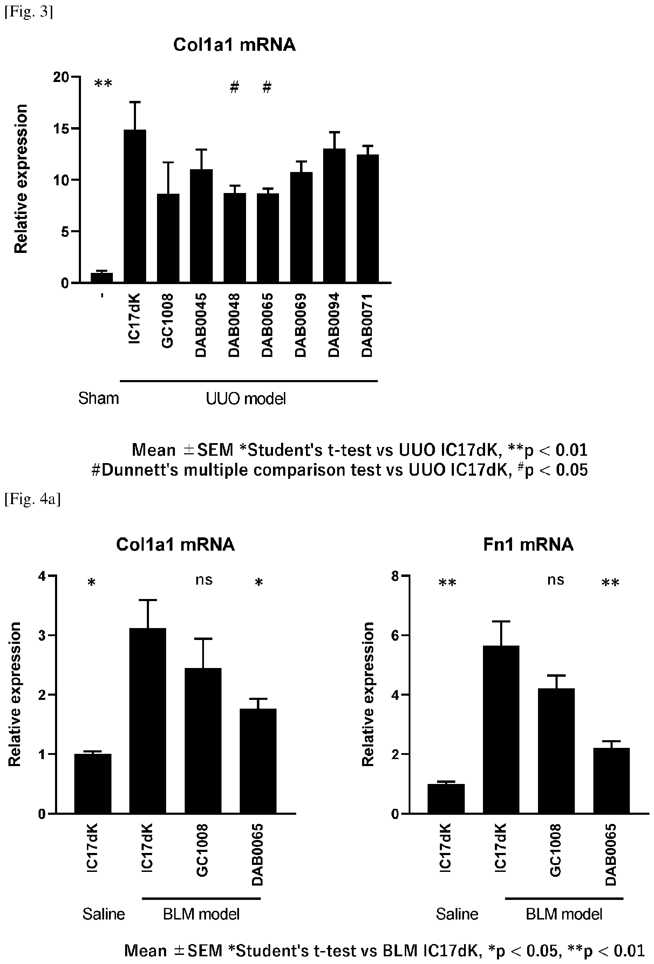

Figure 3 shows the result of evaluating collagen type 1 alpha 1 (Col1a1) mRNA levels in kidney. Efficacy of monoclonal antibodies was evaluated in Unilateral Ureteral Obstruction (UUO) induced mouse renal fibrosis model (UUO mouse model). Sham operated group represents as non-disease-induced mouse as a control. Col1a1 mRNA in UUO mouse model was suppressed by treatment with anti-DDR2 antibodies. IC17dK is an anti-KLH antibody used as a negative control. GC1008 is an anti-mature TGF-beta antibody. Names of anti-DDR2 antibodies starts from DAB.

[Fig. 4]

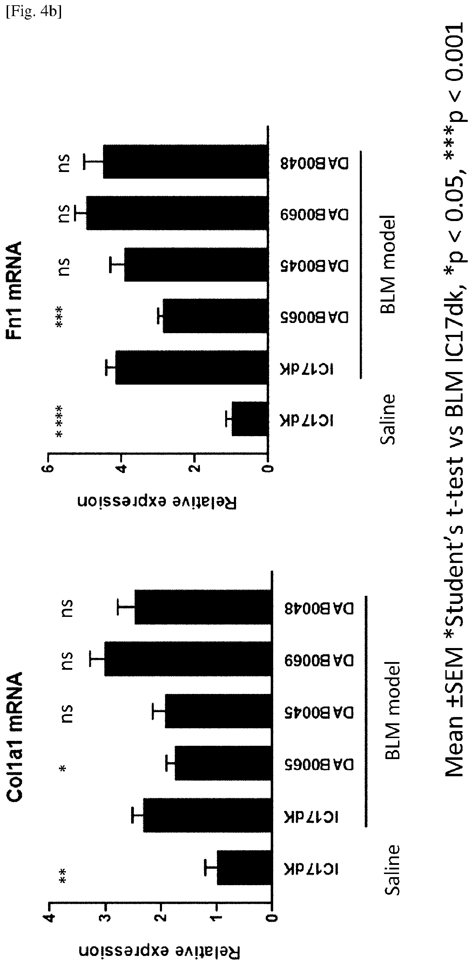

Figure 4 shows the results of evaluating collagen type 1 alpha 1 (Col1a1) mRNA and fibronectin 1 (Fnl) mRNA levels in lung. Efficacy of monoclonal antibodies were evaluated in bleomycin (BLM) induced mouse lung fibrosis model (BLM mouse model). Saline group represents as non-disease-induced mouse as a control. Col1al and Fn1 mRNA in BLM mouse model were suppressed by treatment with anti-DDR2 antibody. IC17 is an anti-KLH antibody used as a negative control. GC1008 is an anti-pan mature TGF-beta antibody. Names of anti-DDR2 antibodies starts from DAB. (A) Experiment comparing the effects of DAB0065 with IC17dk and GC1008. (B) Experiment comparing the effects of various anti-DDR2 antibodies.

[Fig. 5]

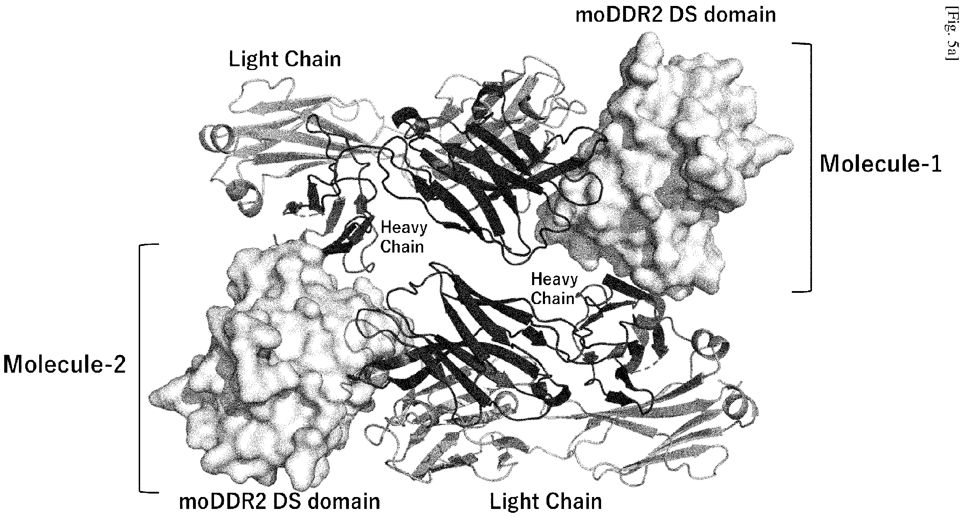

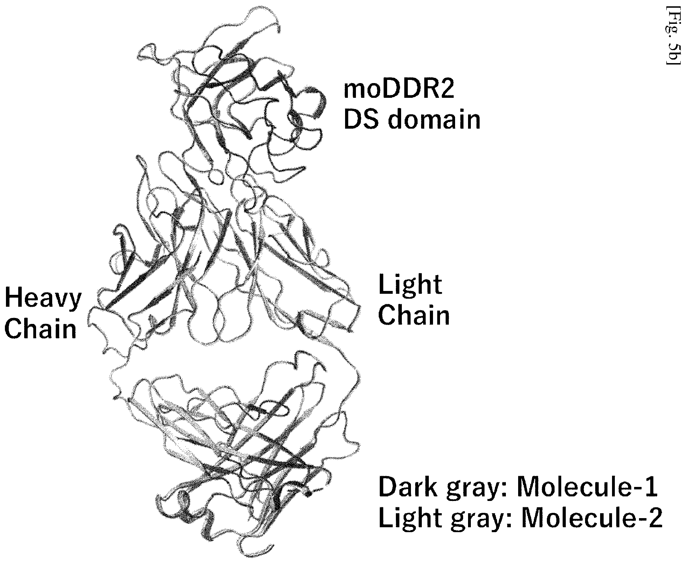

Figure 5 shows the crystal structure of the DAB0065 Fab bound to the moDDR2 DS domain. (A) An asymmetric unit is shown. MoDDR2 DS domain is shown in surface representation and the DAB0065 Fab is shown as ribbons (dark gray: heavy chain, light gray: light chain). (B) superimposed Molecule-1 and Molecule-2 is shown (dark gray: Molecule-1, light gray: Molecule-2).

[Fig. 6]

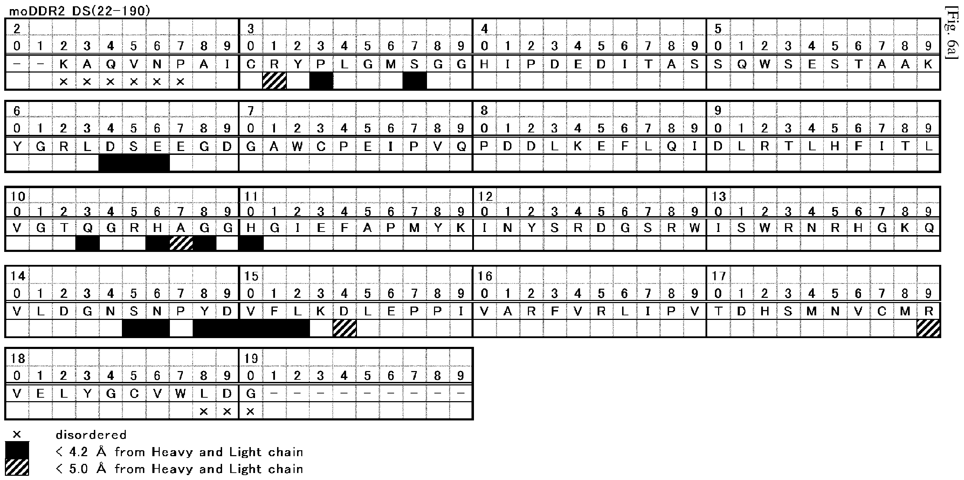

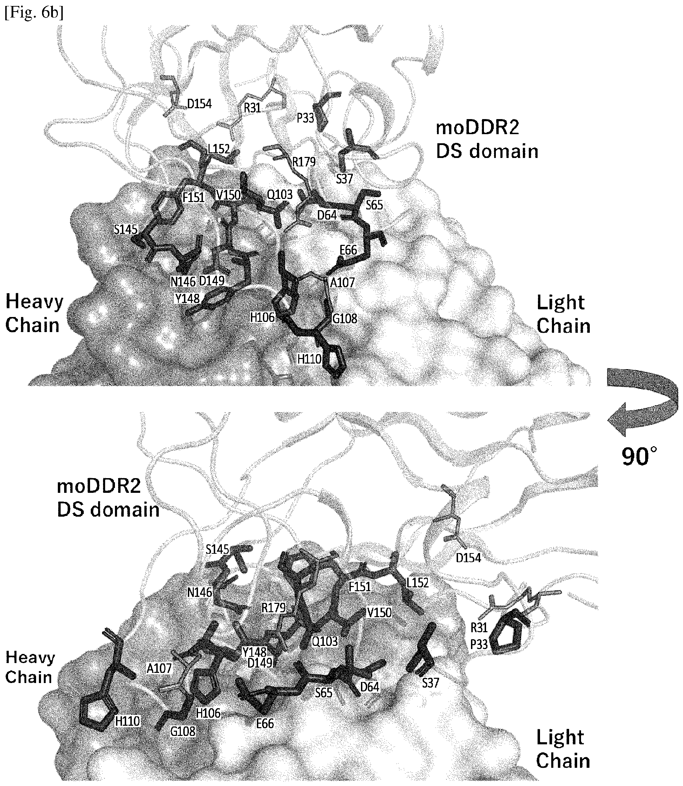

Figure 6 shows the epitope of the DAB0065 Fab contact region on the moDDR2 DS domain. (A) Epitope mapping in the moDDR2 amino acid sequence (black: closer than 4.2 Å, oblique stripes: closer than 5.0 Å). (B) Epitope mapping in the crystal structure (dark gray stick: closer than 4.2 Å, light gray thin sticks: closer than 5.0 Å from DAB0065 Fab which is shown in surface representation)

[Description of Embodiments]

## I. DEFINITIONS

### Acceptor human framework

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

### Antibody-dependent cell-mediated cytotoxicity

"Antibody-dependent cell-mediated cytotoxicity" or "ADCC" refers to a form of cytotoxicity in which secreted Ig bound onto Fc receptors (FcRs) present on certain cytotoxic cells (*e.g.* NK cells, neutrophils, and macrophages) enable these cytotoxic effector cells to bind specifically to an antigen-bearing target cell and subsequently kill the target cell with cytotoxins. The primary cells for mediating ADCC, NK cells, express Fc gamma RIII only, whereas monocytes express Fc gamma RI, Fc gamma RII, and Fc gamma RIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo, e.g.,* in an animal model such as that disclosed in Clynes et al. PNAS (USA) 95:652-656 (1998).

**Affinity**

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

**Affinity matured**

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-DDR2 antibody" and "an antibody that binds to DDR2" refer to an antibody that is capable of binding DDR2 with sufficient affinity such that the antibody is useful as a diagnostic and/or therapeutic agent in targeting DDR2. In one embodiment, the extent of binding of an anti-DDR2 antibody to an unrelated, non-DDR2 protein is less than about 10% of the binding of the antibody to DDR2 as measured, e.g., by a radioimmunoassay (RIA). In certain embodiments, an antibody that binds to DDR2 has a dissociation constant (Kd) of 1 micro M or less, 100 nM or less, 10 nM or less, 1 nM or less, 0.1 nM or less, 0.01 nM or less, or 0.001 nM or less (e.g. $10^{-8}$ M or less, e.g. from $10^{-8}$ M to $10^{-13}$ M, e.g., from $10^{-9}$ M to $10^{-13}$ M). In certain embodiments, an anti-DDR2 antibody binds to an epitope of DDR2 that is conserved among DDR2 from different species.

**Antibody**

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

**Antibody fragment**

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$; diabodies; linear antibodies; single-chain antibody molecules (e.g. scFv); and multispecific antibodies formed from antibody fragments.

**Antibody that binds to the same epitope**

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided

**Antigen-binding domain**

An "antigen-binding domain" may be of any structure as long as it binds an antigen of interest. An antigen-binding domain, include, for example, antibody heavy-chain and light-chain variable regions, "single chain Fv (scFv)", "single chain antibody", "Fv", "single chain Fv 2 (scFv2)", "Fab", "F(ab')2", and IgG.

**Chimeric antibody**

The term "chimeric" antibody refers to an antibody in which a portion of the heavy and/or light chain is derived from a particular source or species, while the remainder of the heavy and/or light chain is derived from a different source or species.

**Antibody class**

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called alpha, delta, epsilon, gamma, and mu, respectively.

**Complement dependent cytotoxicity**

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.,* as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, *e.g.,* Idusogie et al. J. Immunol. 164: 4178-4184 (2000).

**Cytotoxic agent**

The term "cytotoxic agent" as used herein refers to a substance that inhibits or prevents a cellular function and/or causes cell death or destruction. Cytotoxic agents include, but are not limited to, radioactive isotopes (e.g., $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

**DDR1**

The term "DDR1," as used herein, refers to any native DDR1 from any vertebrate source, including mammals such as primates (e.g. humans) and rodents (e.g., mice and rats), unless otherwise indicated. The term encompasses "full-length" unprocessed DDR1 as well as any form of DDR1 that results from processing in the cell. The term also encompasses naturally occurring variants of DDR1, e.g., splice variants or allelic variants.

The amino acid sequence of an exemplary human DDRla (RefSeq No. NP_001945.3) is shown in SEQ ID NO: 81. The amino acid sequence of an exemplary human DDR1b (RefSeq No. NP_054699.2) is shown in SEQ ID NO: 80. The amino acid sequence of an exemplary human DDR1c (RefSeq No. NP_054700.2) is shown in SEQ ID NO: 82. The amino acid sequence of an exemplary human DDR1d (RefSeq No. NP_001189450.1) is shown in SEQ ID NO: 83. The amino acid sequence of an exemplary human DDR1e (RefSeq No. NP_001189451.1) is shown in SEQ ID NO: 84. The amino acid sequence of an exemplary mouse DDR1 (RefSeq No. NP_031610.1) is shown in SEQ ID NO: 79.

Extracellular regions of human DDR1a, human DDR1b, human DDR1c, human DDR1d, and human DDR1e are the region of amino acid 21 to 417 of SEQ ID NO: 81, 80, 82, 83, and 84 respectively. Discoidin (DS) domain of human DDR1b is the region of amino acid 22 to 191, or the region of amino acid 31 to 185, or the region of amino acid 29 to 187 of SEQ ID NO: 80. DS-like (DSL) domain of human DDR1b is the region of amino acid 192 to 367, or the region of amino acid 188 to 367 of SEQ ID NO: 80. Extracellular juxtamembrane (EJXM) domain of human DDR1b is the region of amino acid 368 to 417 of SEQ ID NO: 80.

Extracellular region of mouse DDR1 is the region of amino acid 22 to 415 of SEQ ID NO: 79. Discoidin (DS) domain of mouse DDR1 is the region of amino acid 22 to 186, or the region of amino acid 31 to 186, or the region of amino acid 32 to 186 of SEQ ID NO: 79. DS-like (DSL) domain of mouse DDR1 is the region of amino acid 193 to 369, or the region of amino acid 188 to 367 of SEQ ID NO: 79. Extracellular juxtamembrane (EJXM) domain of mouse DDR1 is the region of amino acid 370 to 415, or the region of 368 to 417 of SEQ ID NO: 79.

**DDR2**

The term "DDR2," as used herein, refers to any native DDR2 from any vertebrate source, including mammals such as primates (e.g. humans) and rodents (e.g., mice and rats), unless otherwise indicated. The term encompasses "full-length" unprocessed DDR2 as well as any form of DDR2 that results from processing in the cell. The term also encompasses naturally occurring variants of DDR2, e.g., splice variants or allelic variants.

The amino acid sequence of an exemplary human DDR2 (RefSeq No. NP_001014796.1) is shown in SEQ ID NO: 78. The amino acid sequence of an exemplary mouse DDR2 (RefSeq No. NP_072075.2) is shown in SEQ ID NO: 77.

Extracellular region of human DDR2 is the region of amino acid 22 to 399 of SEQ ID NO: 78. Discoidin (DS) domain of human DDR2 is the region of amino acid 22 to 191, or the region of amino acid 32 to 184, or the region of amino acid 30 to 185, or the region of amino acid 28 to 187 of SEQ ID NO: 78. DS-like (DSL) domain of human DDR2 is the region of amino acid 200 to 367, or the region of amino acid 188 to 367 of SEQ ID NO: 78. Extracellular juxtamembrane (EJXM) domain of human DDR2 is the region of amino acid 368 to 399 of SEQ ID NO: 78.

Extracellular region of mouse DDR2 is the region of amino acid 24 to 399, or the region of amino acid 22 to 399 of SEQ ID NO: 77. Discoidin (DS) domain of mouse DDR2 is the region of amino acid 24 to 191, or the region of amino acid 32 to 184, or the region of amino acid 30 to 185, or the region of amino acid 28 to 187 of SEQ ID NO: 77. DS-like (DSL) domain of mouse DDR2 is the region of amino acid 200 to 367, or the region of amino acid 188 to 367 of SEQ ID NO: 77. Extracellular juxtamembrane (EJXM) domain of mouse DDR2 is the region of amino acid 368 to 399 of SEQ ID NO: 77.

**Effector functions**

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

**Effective amount**

An "effective amount" of an agent, e.g., a pharmaceutical formulation, refers to an amount effective, at dosages and for periods of time necessary, to achieve the desired therapeutic or prophylactic result.

**Epitope**

An "epitope" may be referred to as defining the site or sites of interaction between an antibody and its antigen(s). Accordingly, in Nilvebrant J, Rockberg J. An Introduction to Epitope Mapping. Methods Mol Biol. 2018;1785:1-10, "Antibody epitopes can conceptually be divided into linear and non-linear epitopes. Linear epitopes, which are also known as continuous or sequential epitopes, can be mimicked by short peptide sequences. Epitopes in which amino acids in distant parts of the sequence are brought together by protein folding are called non-linear, conformational, or discontinuous epitopes. For example, discontinuous epitopes often contain short segments of contiguous residues that can be independently bound by an antibody". As used herein, the terms "conformational epitope" or "non-linear epitope" or "discontinuous epitope" are used interchangeably to refer to an epitope which is composed of at least two amino acids which are not consecutive amino acids in a single protein chain or single polypeptide chain.

**Fc region**

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

**Fc receptor**

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.,* Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn *in vivo* and plasma half life of human FcRn high affinity binding polypeptides can be assayed, e.g., in transgenic mice or transfected human cell lines expressing human FcRn, or in primates to which the polypeptides with a variant Fc region are administered. WO 2000/42072 (Presta) describes antibody variants with increased or decreased binding to FcRs. See also, *e.g.,* Shields et al. J. Biol. Chem. 9(2):6591-6604 (2001).

## Framework

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

## Full length antibody

The terms "full length antibody," "intact antibody," and "whole antibody" are used herein interchangeably to refer to an antibody having a structure substantially similar to a native antibody structure or having heavy chains that contain an Fc region as defined herein.

## Host cell

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

## Human antibody

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

## Human consensus framework

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

## Humanized antibody

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

**HVR**

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra.*

**Immunoconjugate**

An "immunoconjugate" is an antibody conjugated to one or more heterologous molecule(s), including but not limited to a cytotoxic agent.

**Individual / Subject**

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

**Isolated antibody**

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

**Isolated nucleic acid**

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-DDR2 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

**Monoclonal antibody**

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

**Naked antibody**

A "naked antibody" refers to an antibody that is not conjugated to a heterologous moiety (e.g., a cytotoxic moiety) or radiolabel. The naked antibody may be present in a pharmaceutical formulation.

**Native antibody**

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

**Native sequence Fc region**

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

**Package insert**

The term "package insert" is used to refer to instructions customarily included in commercial packages of therapeutic products, that contain information about the indications, usage, dosage, administration, combination therapy, contraindications and/or warnings concerning the use of such therapeutic products.

**Percent (%) amino acid sequence identity**

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

**Pharmaceutical formulation**

The term "pharmaceutical formulation" refers to a preparation which is in such form as to permit the biological activity of an active ingredient contained therein to be effective, and which contains no additional components which are unacceptably toxic to a subject to which the formulation would be administered.

**Pharmaceutically acceptable carrier**

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

**Substantially similar**

The term "substantially similar" or "substantially the same," as used herein, refers to a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values (e.g., Kd values). The term "substantially similar" or "substantially the same", as used herein, also refers to a sufficiently high degree of similarity in a parameter when comparing two subjects (for example, antigen-binding property of an antibody of the invention compared to another antibody), such that one of skill in the art would consider the difference between the two to be of little or no biological significance within the context of the biological characteristic when evaluated by the same method.

**Treatment**

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

**Variable region**

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

**Vector**

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

## II. COMPOSITIONS AND METHODS

In one aspect, the invention is based, in part, on anti-DDR2 antibodies and uses of the same. In certain embodiments, antibodies that bind to DDR2 are provided. Antibodies of the invention are useful, e.g., for the diagnosis or treatment of fibrotic diseases.

### A. Exemplary Anti-DDR2 Antibodies

In one aspect, the invention provides isolated antibodies that bind to DDR2.

In certain embodiments, an anti-DDR2 antibody binds to extracellular region of DDR2. In certain embodiments, an anti-DDR2 antibody binds to discoidin domain (DS domain) of DDR2. In certain embodiments, an anti-DDR2 antibody binds to discoidin-like domain (DSL domain) of DDR2. In certain embodiments, an anti-DDR2 antibody binds to extracellular juxtamembrane domain (EJXM domain) of DDR2.

In certain embodiments, an anti-DDR2 antibody binds to human DDR2. In certain embodiments, an anti-DDR2 antibody binds to mouse DDR2. In certain embodiments, an anti-DDR2 antibody binds to both human DDR2 and mouse DDR2. In certain embodiments, an anti-DDR2 antibody binds to human DDR2 shown in SEQ ID NO: 78. In certain embodiments, an anti-DDR2 antibody binds to mouse DDR2 shown in SEQ ID NO: 77. In certain embodiments, an anti-DDR2 antibody binds to both human DDR2 shown in SEQ ID NO: 78 and mouse DDR2 shown in SEQ ID NO: 77.

In certain embodiments, an anti-DDR2 antibody binds to extracellular region of human DDR2. In certain embodiments, an anti-DDR2 antibody binds to extracellular region of mouse DDR2. In certain embodiments, an anti-DDR2 antibody binds to both extracellular region of human DDR2 and extracellular region of mouse DDR2.

In certain embodiments, an anti-DDR2 antibody binds to discoidin domain (DS domain) of human DDR2. In certain embodiments, an anti-DDR2 antibody binds to discoidin domain (DS domain) of mouse DDR2. In certain embodiments, an anti-DDR2 antibody binds to both discoidin domain (DS domain) of human DDR2 and discoidin domain (DS domain) of mouse DDR2.

In certain embodiments, an anti-DDR2 antibody binds to discoidin-like domain (DSL domain) of human DDR2. In certain embodiments, an anti-DDR2 antibody binds to discoidin-like domain (DSL domain) of mouse DDR2. In certain embodiments, an anti-DDR2 antibody binds to both discoidin-like domain (DSL domain) of human DDR2 and discoidin-like domain (DSL domain) of mouse DDR2.

In certain embodiments, an anti-DDR2 antibody binds to extracellular juxtamembrane domain (EJXM domain) of human DDR2. In certain embodiments, an anti-DDR2 antibody binds to extracellular juxtamembrane domain (EJXM domain) of mouse DDR2. In certain embodiments, an anti-DDR2 antibody binds to both extracellular juxtamembrane domain (EJXM domain) of human DDR2 and extracellular juxtamembrane domain (EJXM domain) of mouse DDR2.

In certain embodiments, an anti-DDR2 antibody does not bind to DDR1. In certain embodiments, an anti-DDR2 antibody does not bind to human DDR1. In certain embodiments, an anti-DDR2 antibody does not bind to human DDR1a. In certain embodiments, an anti-DDR2 antibody does not bind to human DDR1b. In certain embodiments, an anti-DDR2 antibody does not bind to human DDR1c. In certain embodiments, an anti-DDR2 antibody does not bind to human DDR1d. In certain embodiments, an anti-DDR2 antibody does not bind to human DDR1e. In certain embodiments, an anti-DDR2 antibody does not bind to mouse DDR1. In certain embodiments, an anti-DDR2 antibody does not bind to any one of human DDR1a, human DDR1b, human DDR1c, human DDR1d, human DDR1e, and mouse DDR1. In certain embodiments, an anti-DDR2 antibody does not bind to human DDRIa shown in SEQ ID NO: 81. In certain embodiments, an anti-DDR2 antibody does not bind to human DDR1b shown in SEQ ID NO: 80. In certain embodiments, an anti-DDR2 antibody does not bind to human DDR1c shown in SEQ ID NO: 82. In certain embodiments, an anti-DDR2 antibody does not bind to human DDR1d shown in SEQ ID NO: 83. In certain embodiments, an anti-DDR2 antibody does not bind to human DDR1e shown in SEQ ID NO: 84. In certain embodiments, an anti-DDR2 antibody does not bind to mouse DDR1 shown in SEQ ID NO: 79. In certain embodiments, an anti-DDR2 antibody does not bind to any one of human DDRIa shown in SEQ ID NO: 81, human DDR1b shown in SEQ ID NO: 80, human DDR1c shown in SEQ ID NO: 82, human DDR1d shown in SEQ ID NO: 83, human DDR1e shown in SEQ ID NO: 84 and mouse DDR1 shown in SEQ ID NO: 79.

In certain embodiments, an anti-DDR2 antibody binds to the region of amino acid 22 to 399 of SEQ ID NO: 78.

In certain embodiments, an anti-DDR2 antibody binds to the region of amino acid 22 to 191 of SEQ ID NO: 78. In certain embodiments, an anti-DDR2 antibody binds to the region of amino acid 32 to 184 of SEQ ID NO: 78. In certain embodiments, an anti-DDR2 antibody binds to the region of amino acid 30 to 185 of SEQ ID NO: 78. In certain embodiments, an anti-DDR2 antibody binds to the region of amino acid 28-187 of SEQ ID NO: 78.

In certain embodiments, an anti-DDR2 antibody binds to the region of amino acid 200 to 367 of SEQ ID NO: 78. In certain embodiments, an anti-DDR2 antibody binds to the region of amino acid 188 to 367 of SEQ ID NO: 78.

In certain embodiments, an anti-DDR2 antibody binds to the region of amino acid 368 to 399 of SEQ ID NO: 78.

In certain embodiments, an anti-DDR2 antibody binds to the region of amino acid 24 to 399 of SEQ ID NO: 77. In certain embodiments, an anti-DDR2 antibody binds to the region of amino acid 22 to 399 of SEQ ID NO: 77.

In certain embodiments, an anti-DDR2 antibody binds to the region of amino acid 24 to 191 of SEQ ID NO: 77. In certain embodiments, an anti-DDR2 antibody binds to the region of amino acid 32 to 184 of SEQ ID NO: 77. In certain embodiments, an anti-DDR2 antibody binds to the region of amino acid 30 to 185 of SEQ ID NO: 77. In certain embodiments, an anti-DDR2 antibody binds to the region of amino acid 28 to 187 of SEQ ID NO: 77.

In certain embodiments, an anti-DDR2 antibody binds to the region of amino acid 200 to 367 of SEQ ID NO: 77. In certain embodiments, an anti-DDR2 antibody binds to the region of amino acid 188 to 367 of SEQ ID NO: 77.

In certain embodiments, an anti-DDR2 antibody binds to the region of amino acid 368 to 399 of SEQ ID NO: 77.

In certain embodiments, an anti-DDR2 antibody binds to the region of amino acid 201 to 393 of SEQ ID NO: 77. In certain embodiments, an anti-DDR2 antibody binds to the region of amino acid 24 to 200 of SEQ ID NO: 77.

In certain embodiments, an anti-DDR2 antibody does not bind to the region of amino acid 22 to 415 of SEQ ID NO: 79. In certain embodiments, an anti-DDR2 antibody does not bind to the region of amino acid 21 to 417 of SEQ ID NO: 81. In certain embodiments, an anti-DDR2 antibody does not bind to the region of amino acid 21 to 417 of SEQ ID NO: 80. In certain embodiments, an anti-DDR2 antibody does not bind to the region of amino acid 21 to 417 of SEQ ID NO: 82. In certain embodiments, an anti-DDR2 antibody does not bind to the region of amino acid 21 to 417 of SEQ ID NO: 83. In certain embodiments, an anti-DDR2 antibody does not bind to the region of amino acid 21 to 417 of SEQ ID NO: 84.

In certain embodiments, an anti-DDR2 antibody binds to a fragment of DDR2 comprising amino acid 22 to 399 of SEQ ID NO: 78.

In certain embodiments, an anti-DDR2 antibody binds to a fragment of DDR2 comprising amino acid 22 to 191 of SEQ ID NO: 78. In certain embodiments, an anti-DDR2 antibody binds to a fragment of DDR2 comprising amino acid 32 to 184 of SEQ ID NO: 78. In certain embodiments, an anti-DDR2 antibody binds to a fragment of DDR2 comprising amino acid 30 to 185 of SEQ ID NO: 78. In certain embodiments, an anti-DDR2 antibody binds to a fragment of DDR2 comprising amino acid 28-187 of SEQ ID NO: 78.

In certain embodiments, an anti-DDR2 antibody binds to a fragment of DDR2 comprising amino acid 200 to 367 of SEQ ID NO: 78. In certain embodiments, an anti-DDR2 antibody binds to a fragment of DDR2 comprising amino acid 188 to 367 of SEQ ID NO: 78.

In certain embodiments, an anti-DDR2 antibody binds to a fragment of DDR2 comprising amino acid 368 to 399 of SEQ ID NO: 78.

In certain embodiments, an anti-DDR2 antibody binds to a fragment of DDR2 comprising amino acid 24 to 399 of SEQ ID NO: 77. In certain embodiments, an anti-DDR2 antibody binds to a fragment of DDR2 comprising amino acid 22 to 399 of SEQ ID NO: 77.

In certain embodiments, an anti-DDR2 antibody binds to a fragment of DDR2 comprising amino acid 24 to 191 of SEQ ID NO: 77. In certain embodiments, an anti-DDR2 antibody binds to a fragment of DDR2 comprising amino acid 32 to 184 of SEQ ID NO: 77. In certain embodiments, an anti-DDR2 antibody binds to a fragment of DDR2 comprising amino acid 30 to 185 of SEQ ID NO: 77. In certain embodiments, an anti-DDR2 antibody binds to a fragment of DDR2 comprising amino acid 28 to 187 of SEQ ID NO: 77.

In certain embodiments, an anti-DDR2 antibody binds to a fragment of DDR2 comprising amino acid 200 to 367 of SEQ ID NO: 77. In certain embodiments, an anti-DDR2 antibody binds to a fragment of DDR2 comprising amino acid 188 to 367 of SEQ ID NO: 77.

In certain embodiments, an anti-DDR2 antibody binds to a fragment of DDR2 comprising amino acid 368 to 399 of SEQ ID NO: 77.

In certain embodiments, an anti-DDR2 antibody binds to a fragment of DDR2 comprising amino acid 201 to 393 of SEQ ID NO: 77. In certain embodiments, an anti-DDR2 antibody binds to a fragment of DDR2 comprising amino acid 24 to 200 of SEQ ID NO: 77.

In certain embodiments, an anti-DDR2 antibody does not bind to a fragment of DDR1 comprising amino acid 22 to 415 of SEQ ID NO: 79. In certain embodiments, an anti-DDR2 antibody does not bind to a fragment of DDR1 comprising amino acid 21 to 417 of SEQ ID NO: 81. In certain embodiments, an anti-DDR2 antibody does not bind to a fragment of DDR1 comprising amino acid 21 to 417 of SEQ ID NO: 80. In certain embodiments, an anti-DDR2 antibody does not bind to a fragment of DDR1 comprising amino acid 21 to 417 of SEQ ID NO: 82. In certain embodiments, an anti-DDR2 antibody does not bind to a fragment of DDR1 comprising amino acid 21 to 417 of SEQ ID NO: 83. In certain embodiments, an anti-DDR2 antibody does not bind to a fragment of DDR1 comprising amino acid 21 to 417 of SEQ ID NO: 84.

In certain embodiments, an anti-DDR2 antibody binds DDR2 at an epitope comprising all or part of a sequence defined by amino acids 31 to 179 of SEQ ID NO: 77 or 78. In certain embodiments, an anti-DDR2 antibody binds to at least one or more, or all, of the amino acids R31, P33, S37, D64, S65, E66, Q103, H106, A107, G108, H110, S145, N146, Y148, D149, V150, F151, L152, D154 and R179 of SEQ ID NO: 77. In certain embodiments, an anti-DDR2 antibody binds to at least one or more, or all, of the amino acids P33, S37, D64, S65, E66, Q103, H106, G108, H110, S145, N146, Y148, D149, V150, F151, and L152 of SEQ ID NO: 77. In certain embodiments, an anti-DDR2 antibody binds to at least one or more, or all, of the amino acids R31, P33, S37, D64, S65, E66, Q103, H106, A107, G108, H110, S145, N146, Y148, D149, 1150, F151, L152, D154 and R179 of SEQ ID NO: 78. In certain embodiments, an anti-DDR2 antibody binds to at least one or more, or all, of the amino acids P33, S37, D64, S65, E66, Q103, H106, G108, H110, S145, N146, Y148, D149, 1150, F151, and L152 of SEQ ID NO: 78.

In certain embodiments, an anti-DDR2 antibody reduces collagen type 1 alpha 1 (Col1a1) mRNA level and/or fibronectin 1 (Fnl) mRNA level in a subject. In certain embodiments, an anti-DDR2 antibody reduces collagen type 1 alpha 1 (Col1a1) mRNA level and/or fibronectin 1 (Fnl) mRNA level in a subject which has fibrosis.

In one aspect, the invention provides an anti-DDR2 antibody comprising at least one, two, three, four, five, or six HVRs selected from (a) HVR-H1 comprising the amino acid sequence of SEQ ID NO: 2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO: 6; (e) HVR-L2 comprising the amino acid sequence of SEQ ID NO:7; and (f) HVR-L3 comprising the amino acid sequence of SEQ ID NO:8.

In one aspect, the invention provides an anti-DDR2 antibody comprising at least one, two, three, four, five, or six HVRs selected from (a) HVR-H1 comprising the amino acid sequence of SEQ ID NO:10; (b) HVR-H2 comprising the amino acid sequence of SEQ ID NO:11; (c) HVR-H3 comprising the amino acid sequence of SEQ ID NO:12; (d) HVR-L1 comprising the amino acid sequence of SEQ ID NO:14; (e) HVR-L2 comprising the amino acid sequence of SEQ ID NO:15; and (f) HVR-L3 comprising the amino acid sequence of SEQ ID NO:16.

In one aspect, the invention provides an anti-DDR2 antibody comprising at least one, two, three, four, five, or six HVRs selected from (a) HVR-H1 comprising the amino acid sequence of SEQ ID NO:18; (b) HVR-H2 comprising the amino acid sequence of SEQ ID NO:19; (c) HVR-H3 comprising the amino acid sequence of SEQ ID NO:20; (d) HVR-L1 comprising the amino acid sequence of SEQ ID NO:22; (e) HVR-L2 comprising the amino acid sequence of SEQ ID NO:23; and (f) HVR-L3 comprising the amino acid sequence of SEQ ID NO:24.

In one aspect, the invention provides an anti-DDR2 antibody comprising at least one, two, three, four, five, or six HVRs selected from (a) HVR-H1 comprising the amino acid sequence of SEQ ID NO:26; (b) HVR-H2 comprising the amino acid sequence of SEQ ID NO:27; (c) HVR-H3 comprising the amino acid sequence of SEQ ID NO:28; (d) HVR-L1 comprising the amino acid sequence of SEQ ID NO:30; (e) HVR-L2 comprising the amino acid sequence of SEQ ID NO:31; and (f) HVR-L3 comprising the amino acid sequence of SEQ ID NO:32.

In one aspect, the invention provides an anti-DDR2 antibody comprising at least one, two, three, four, five, or six HVRs selected from (a) HVR-H1 comprising the amino acid sequence of SEQ ID NO:34; (b) HVR-H2 comprising the amino acid sequence of SEQ ID NO:35; (c) HVR-H3 comprising the amino acid sequence of SEQ ID NO:36; (d) HVR-L1 comprising the amino acid sequence of SEQ ID NO:38; (e) HVR-L2 comprising the amino acid sequence of SEQ ID NO:39; and (f) HVR-L3 comprising the amino acid sequence of SEQ ID NO:40.

In one aspect, the invention provides an anti-DDR2 antibody comprising at least one, two, three, four, five, or six HVRs selected from (a) HVR-H1 comprising the amino acid sequence of SEQ ID NO:42; (b) HVR-H2 comprising the amino acid sequence of SEQ ID NO:43; (c) HVR-H3 comprising the amino acid sequence of SEQ ID NO:44; (d) HVR-L1 comprising the amino acid sequence of SEQ ID NO:46; (e) HVR-L2 comprising the amino acid sequence of SEQ ID NO:47; and (f) HVR-L3 comprising the amino acid sequence of SEQ ID NO:48.

In one aspect, the invention provides an anti-DDR2 antibody comprising at least one, two, three, four, five, or six HVRs selected from (a) HVR-H1 comprising the amino acid sequence of SEQ ID NO:50; (b) HVR-H2 comprising the amino acid sequence of SEQ ID NO:51; (c) HVR-H3 comprising the amino acid sequence of SEQ ID NO:52; (d) HVR-L1 comprising the amino acid sequence of SEQ ID NO:54; (e) HVR-L2 comprising the amino acid sequence of SEQ ID NO:55; and (f) HVR-L3 comprising the amino acid sequence of SEQ ID NO:56.

In one aspect, the invention provides an anti-DDR2 antibody comprising at least one, two, three, four, five, or six HVRs selected from (a) HVR-H1 comprising the amino acid sequence of SEQ ID NO:58; (b) HVR-H2 comprising the amino acid sequence of SEQ ID NO:59; (c) HVR-H3 comprising the amino acid sequence of SEQ ID NO:60; (d) HVR-L1 comprising the amino acid sequence of SEQ ID NO:62; (e) HVR-L2 comprising the amino acid sequence of SEQ ID NO:63; and (f) HVR-L3 comprising the amino acid sequence of SEQ ID NO:64.

In one aspect, the invention provides an anti-DDR2 antibody comprising at least one, two, three, four, five, or six HVRs selected from (a) HVR-H1 comprising the amino acid sequence of SEQ ID NO:66; (b) HVR-H2 comprising the amino acid sequence of SEQ ID NO:67; (c) HVR-H3 comprising the amino acid sequence of SEQ ID NO:68; (d) HVR-L1 comprising the amino acid sequence of SEQ ID NO:70; (e) HVR-L2 comprising the amino acid sequence of SEQ ID NO:71; and (f) HVR-L3 comprising the amino acid sequence of SEQ ID NO:72.

In one aspect, the invention provides an antibody comprising at least one, at least two, or all three VH HVR sequences selected from (a) HVR-H1 comprising the amino acid sequence of SEQ ID NO: 2; (b) HVR-H2 comprising the amino acid sequence of SEQ ID NO:3; and (c) HVR-H3 comprising the amino acid sequence of SEQ ID NO:4. In one embodiment, the antibody comprises HVR-H3 comprising the amino acid sequence of SEQ ID NO:4. In another embodiment, the antibody comprises HVR-H3 comprising the amino acid sequence of SEQ ID NO:4 and HVR-L3 comprising the amino acid sequence of SEQ ID NO:8. In a further embodiment, the antibody comprises HVR-H3 comprising the amino acid sequence of SEQ ID NO:4, HVR-L3 comprising the amino acid sequence of SEQ ID NO:8, and HVR-H2 comprising the amino acid sequence of SEQ ID NO:3. In a further embodiment, the antibody comprises (a) HVR-H1 comprising the amino acid sequence of SEQ ID NO:2; (b) HVR-H2 comprising the amino acid sequence of SEQ ID NO:3; and (c) HVR-H3 comprising the amino acid sequence of SEQ ID NO:4.

In one aspect, the invention provides an antibody comprising at least one, at least two, or all three VH HVR sequences selected from (a) HVR-H1 comprising the amino acid sequence of SEQ ID NO:10; (b) HVR-H2 comprising the amino acid sequence of SEQ ID NO:11; and (c) HVR-H3 comprising the amino acid sequence of SEQ ID NO:12. In one embodiment, the antibody comprises HVR-H3 comprising the amino acid sequence of SEQ ID NO:12. In another embodiment, the antibody comprises HVR-H3 comprising the amino acid sequence of SEQ ID NO:12 and HVR-L3 comprising the amino acid sequence of SEQ ID NO:16. In a further embodiment, the antibody comprises HVR-H3 comprising the amino acid sequence of SEQ ID NO:12, HVR-L3 comprising the amino acid sequence of SEQ ID NO:16, and HVR-H2 comprising the amino acid sequence of SEQ ID NO:11. In a further embodiment, the antibody comprises (a) HVR-H1 comprising the amino acid sequence of SEQ ID NO:10; (b) HVR-H2 comprising the amino acid sequence of SEQ ID NO:11; and (c) HVR-H3 comprising the amino acid sequence of SEQ ID NO:12.

In one aspect, the invention provides an antibody comprising at least one, at least two, or all three VH HVR sequences selected from (a) HVR-H1 comprising the amino acid sequence of SEQ ID NO:18; (b) HVR-H2 comprising the amino acid sequence of SEQ ID NO:19; and (c) HVR-H3 comprising the amino acid sequence of SEQ ID NO:20. In one embodiment, the antibody comprises HVR-H3 comprising the amino acid sequence of SEQ ID NO:20. In another embodiment, the antibody comprises HVR-H3 comprising the amino acid sequence of SEQ ID NO:20 and HVR-L3 comprising the amino acid sequence of SEQ ID NO:24. In a further embodiment, the antibody comprises HVR-H3 comprising the amino acid sequence of SEQ ID NO:20, HVR-L3 comprising the amino acid sequence of SEQ ID NO:24, and HVR-H2 comprising the amino acid sequence of SEQ ID NO:19. In a further embodiment, the antibody comprises (a) HVR-H1 comprising the amino acid sequence of SEQ ID NO:18; (b) HVR-H2 comprising the amino acid sequence of SEQ ID NO:19; and (c) HVR-H3 comprising the amino acid sequence of SEQ ID NO:20.

In one aspect, the invention provides an antibody comprising at least one, at least two, or all three VH HVR sequences selected from (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO:28. In one embodiment, the antibody comprises HVR-H3 comprising the amino acid sequence of SEQ ID NO:28. In another embodiment, the antibody comprises HVR-H3 comprising the amino acid sequence of SEQ ID NO:28 and HVR-L3 comprising the amino acid sequence of SEQ ID NO:32. In a further embodiment, the antibody comprises HVR-H3 comprising the amino acid sequence of SEQ ID NO:28, HVR-L3 comprising the amino acid sequence of SEQ ID NO:32, and HVR-H2 comprising the amino acid sequence of SEQ ID NO:27. In a further embodiment, the antibody comprises (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO:28.

In one aspect, the invention provides an antibody comprising at least one, at least two, or all three VH HVR sequences selected from (a) HVR-H1 comprising the amino acid sequence of SEQ ID NO:34; (b) HVR-H2 comprising the amino acid sequence of SEQ ID NO:35; and (c) HVR-H3 comprising the amino acid sequence of SEQ ID NO:36. In one embodiment, the antibody comprises HVR-H3 comprising the amino acid sequence of SEQ ID NO:36. In another embodiment, the antibody comprises HVR-H3 comprising the amino acid sequence of SEQ ID NO:36 and HVR-L3 comprising the amino acid sequence of SEQ ID NO:40. In a further embodiment, the antibody comprises HVR-H3 comprising the amino acid sequence of SEQ ID NO:36, HVR-L3 comprising the amino acid sequence of SEQ ID NO:40, and HVR-H2 comprising the amino acid sequence of SEQ ID NO:35. In a further embodiment, the antibody comprises (a) HVR-H1 comprising the amino acid sequence of SEQ ID NO:34; (b) HVR-H2 comprising the amino acid sequence of SEQ ID NO:35; and (c) HVR-H3 comprising the amino acid sequence of SEQ ID NO:36.

In one aspect, the invention provides an antibody comprising at least one, at least two, or all three VH HVR sequences selected from (a) HVR-H1 comprising the amino acid sequence of SEQ ID NO:42; (b) HVR-H2 comprising the amino acid sequence of SEQ ID NO:43; and (c) HVR-H3 comprising the amino acid sequence of SEQ ID NO:44. In one embodiment, the antibody comprises HVR-H3 comprising the amino acid sequence of SEQ ID NO:44. In another embodiment, the antibody comprises HVR-H3 comprising the amino acid sequence of SEQ ID NO:44 and HVR-L3 comprising the amino acid sequence of SEQ ID NO:48. In a further embodiment, the antibody comprises HVR-H3 comprising the amino acid sequence of SEQ ID NO:44, HVR-L3 comprising the amino acid sequence of SEQ ID NO:48, and HVR-H2 comprising the amino acid sequence of SEQ ID NO:43. In a further embodiment, the antibody comprises (a) HVR-H1 comprising the amino acid sequence of SEQ ID NO:42; (b) HVR-H2 comprising the amino acid sequence of SEQ ID NO:43; and (c) HVR-H3 comprising the amino acid sequence of SEQ ID NO:44.

In one aspect, the invention provides an antibody comprising at least one, at least two, or all three VH HVR sequences selected from (a) HVR-H1 comprising the amino acid sequence of SEQ ID NO:50; (b) HVR-H2 comprising the amino acid sequence of SEQ ID NO:51; and (c) HVR-H3 comprising the amino acid sequence of SEQ ID NO:52. In one embodiment, the antibody comprises HVR-H3 comprising the amino acid sequence of SEQ ID NO:52. In another embodiment, the antibody comprises HVR-H3 comprising the amino acid sequence of SEQ ID NO:52 and HVR-L3 comprising the amino acid sequence of SEQ ID NO:56. In a further embodiment, the antibody comprises HVR-H3 comprising the amino acid sequence of SEQ ID NO:52, HVR-L3 comprising the amino acid sequence of SEQ ID NO:56, and HVR-H2 comprising the amino acid sequence of SEQ ID NO:51. In a further embodiment, the antibody comprises (a) HVR-H1 comprising the amino acid sequence of SEQ ID NO:50, (b) HVR-H2 comprising the amino acid sequence of SEQ ID NO:51, and (c) HVR-H3 comprising the amino acid sequence of SEQ ID NO:52.

In one aspect, the invention provides an antibody comprising at least one, at least two, or all three VH HVR sequences selected from (a) HVR-H1 comprising the amino acid sequence of SEQ ID NO:58; (b) HVR-H2 comprising the amino acid sequence of SEQ ID NO:59; and (c) HVR-H3 comprising the amino acid sequence of SEQ ID NO:60. In one embodiment, the antibody comprises HVR-H3 comprising the amino acid sequence of SEQ ID NO:60. In another embodiment, the antibody comprises HVR-H3 comprising the amino acid sequence of SEQ ID NO:60 and HVR-L3 comprising the amino acid sequence of SEQ ID NO:64. In a further embodiment, the antibody comprises HVR-H3 comprising the amino acid sequence of SEQ ID NO:60, HVR-L3 comprising the amino acid sequence of SEQ ID NO:64, and HVR-H2 comprising the amino acid sequence of SEQ ID NO:59. In a further embodiment, the antibody comprises (a) HVR-H1 comprising the amino acid sequence of SEQ ID NO:58, (b) HVR-H2 comprising the amino acid sequence of SEQ ID NO:59, and (c) HVR-H3 comprising the amino acid sequence of SEQ ID NO:60.

In one aspect, the invention provides an antibody comprising at least one, at least two, or all three VH HVR sequences selected from (a) HVR-H1 comprising the amino acid sequence of SEQ ID NO:66; (b) HVR-H2 comprising the amino acid sequence of SEQ ID NO:67; and (c) HVR-H3 comprising the amino acid sequence of SEQ ID NO:68. In one embodiment, the antibody comprises HVR-H3 comprising the amino acid sequence of SEQ ID NO:68. In another embodiment, the antibody comprises HVR-H3 comprising the amino acid sequence of SEQ ID NO:68 and HVR-L3 comprising the amino acid sequence of SEQ ID NO:72. In a further embodiment, the antibody comprises HVR-H3 comprising the amino acid sequence of SEQ ID NO:68, HVR-L3 comprising the amino acid sequence of SEQ ID NO:72, and HVR-H2 comprising the amino acid sequence of SEQ ID NO:67. In a further embodiment, the antibody comprises (a) HVR-H1 comprising the amino acid sequence of SEQ ID NO:66, (b) HVR-H2 comprising the amino acid sequence of SEQ ID NO:67, and (c) HVR-H3 comprising the amino acid sequence of SEQ ID NO:68.

In another aspect, the invention provides an antibody comprising at least one, at least two, or all three VL HVR sequences selected from (a) HVR-L1 comprising the amino acid sequence of SEQ ID NO:6; (b) HVR-L2 comprising the amino acid sequence of SEQ ID NO:7; and (c) HVR-L3 comprising the amino acid sequence of SEQ ID NO:8. In one embodiment, the antibody comprises (a) HVR-L1 comprising the amino acid sequence of SEQ ID NO:6; (b) HVR-L2 comprising the amino acid sequence of SEQ ID NO:7; and (c) HVR-L3 comprising the amino acid sequence of SEQ ID NO:8.

In another aspect, the invention provides an antibody comprising at least one, at least two, or all three VL HVR sequences selected from (a) HVR-L1 comprising the amino acid sequence of SEQ ID NO:14; (b) HVR-L2 comprising the amino acid sequence of SEQ ID NO:15; and (c) HVR-L3 comprising the amino acid sequence of SEQ ID NO:16. In one embodiment, the antibody comprises (a) HVR-L1 comprising the amino acid sequence of SEQ ID NO:14; (b) HVR-L2 comprising the amino acid sequence of SEQ ID NO:15; and (c) HVR-L3 comprising the amino acid sequence of SEQ ID NO:16.

In another aspect, the invention provides an antibody comprising at least one, at least two, or all three VL HVR sequences selected from (a) HVR-L1 comprising the amino acid sequence of SEQ ID NO:22; (b) HVR-L2 comprising the amino acid sequence of SEQ ID NO:23; and (c) HVR-L3 comprising the amino acid sequence of SEQ ID NO:24. In one embodiment, the antibody comprises (a) HVR-L1 comprising the amino acid sequence of SEQ ID NO:22; (b) HVR-L2 comprising the amino acid sequence of SEQ ID NO:23; and (c) HVR-L3 comprising the amino acid sequence of SEQ ID NO:24.

In another aspect, the invention provides an antibody comprising at least one, at least two, or all three VL HVR sequences selected from (a) HVR-L1 comprising the amino acid sequence of SEQ ID NO:30; (b) HVR-L2 comprising the amino acid sequence of SEQ ID NO:31; and (c) HVR-L3 comprising the amino acid sequence of SEQ ID NO:32. In one embodiment, the antibody comprises (a) HVR-L1 comprising the amino acid sequence of SEQ ID NO:30; (b) HVR-L2 comprising the amino acid sequence of SEQ ID NO:31; and (c) HVR-L3 comprising the amino acid sequence of SEQ ID NO:32.

In another aspect, the invention provides an antibody comprising at least one, at least two, or all three VL HVR sequences selected from (a) HVR-L1 comprising the amino acid sequence of SEQ ID NO:38; (b) HVR-L2 comprising the amino acid sequence of SEQ ID NO:39; and (c) HVR-L3 comprising the amino acid sequence of SEQ ID NO:40. In one embodiment, the antibody comprises (a) HVR-L1 comprising the amino acid sequence of SEQ ID NO:38; (b) HVR-L2 comprising the amino acid sequence of SEQ ID NO:39; and (c) HVR-L3 comprising the amino acid sequence of SEQ ID NO:40.

In another aspect, the invention provides an antibody comprising at least one, at least two, or all three VL HVR sequences selected from (a) HVR-L1 comprising the amino acid sequence of SEQ ID NO:46; (b) HVR-L2 comprising the amino acid sequence of SEQ ID NO:47; and (c) HVR-L3 comprising the amino acid sequence of SEQ ID NO:48. In one embodiment, the antibody comprises (a) HVR-L1 comprising the amino acid sequence of SEQ ID NO:46; (b) HVR-L2 comprising the amino acid sequence of SEQ ID NO:47; and (c) HVR-L3 comprising the amino acid sequence of SEQ ID NO:48.

In another aspect, the invention provides an antibody comprising at least one, at least two, or all three VL HVR sequences selected from (a) HVR-L1 comprising the amino acid sequence of SEQ ID NO:54; (b) HVR-L2 comprising the amino acid sequence of SEQ ID NO:55; and (c) HVR-L3 comprising the amino acid sequence of SEQ ID NO:56. In one embodiment, the antibody comprises (a) HVR-L1 comprising the amino acid sequence of SEQ ID NO:54; (b) HVR-L2 comprising the amino acid sequence of SEQ ID NO:55; and (c) HVR-L3 comprising the amino acid sequence of SEQ ID NO:56.

In another aspect, the invention provides an antibody comprising at least one, at least two, or all three VL HVR sequences selected from (a) HVR-L1 comprising the amino acid sequence of SEQ ID NO:62; (b) HVR-L2 comprising the amino acid sequence of SEQ ID NO:63; and (c) HVR-L3 comprising the amino acid sequence of SEQ ID NO:64. In one embodiment, the antibody comprises (a) HVR-L1 comprising the amino acid sequence of SEQ ID NO:62; (b) HVR-L2 comprising the amino acid sequence of SEQ ID NO:63; and (c) HVR-L3 comprising the amino acid sequence of SEQ ID NO:64.

In another aspect, the invention provides an antibody comprising at least one, at least two, or all three VL HVR sequences selected from (a) HVR-L1 comprising the amino acid sequence of SEQ ID NO:70; (b) HVR-L2 comprising the amino acid sequence of SEQ ID NO:71; and (c) HVR-L3 comprising the amino acid sequence of SEQ ID NO:72. In one embodiment, the antibody comprises (a) HVR-L1 comprising the amino acid sequence of SEQ ID NO:70; (b) HVR-L2 comprising the amino acid sequence of SEQ ID NO:71; and (c) HVR-L3 comprising the amino acid sequence of SEQ ID NO:72.

In another aspect, an antibody of the invention comprises (a) a VH domain comprising at least one, at least two, or all three VH HVR sequences selected from (i) HVR-H1 comprising the amino acid sequence of SEQ ID NO:2, (ii) HVR-H2 comprising the amino acid sequence of SEQ ID NO:3, and (iii) HVR-H3 comprising an amino acid sequence of SEQ ID NO:4; and (b) a VL domain comprising at least one, at least two, or all three VL HVR sequences selected from (i) HVR-L1 comprising the amino acid sequence of SEQ ID NO:6, (ii) HVR-L2 comprising the amino acid sequence of SEQ ID NO:7, and (c) HVR-L3 comprising the amino acid sequence of SEQ ID NO:8.

In another aspect, an antibody of the invention comprises (a) a VH domain comprising at least one, at least two, or all three VH HVR sequences selected from (i) HVR-H1 comprising the amino acid sequence of SEQ ID NO:10, (ii) HVR-H2 comprising the amino acid sequence of SEQ ID NO:11, and (iii) HVR-H3 comprising an amino acid sequence of SEQ ID NO:12; and (b) a VL domain comprising at least one, at least two, or all three VL HVR sequences selected from (i) HVR-L1 comprising the amino acid sequence of SEQ ID NO:14, (ii) HVR-L2 comprising the amino acid sequence of SEQ ID NO:15, and (c) HVR-L3 comprising the amino acid sequence of SEQ ID NO:16.

In another aspect, an antibody of the invention comprises (a) a VH domain comprising at least one, at least two, or all three VH HVR sequences selected from (i) HVR-H1 comprising the amino acid sequence of SEQ ID NO:18, (ii) HVR-H2 comprising the amino acid sequence of SEQ ID NO:19, and (iii) HVR-H3 comprising an amino acid sequence of SEQ ID NO:20; and (b) a VL domain comprising at least one, at least two, or all three VL HVR sequences selected from (i) HVR-L1 comprising the amino acid sequence of SEQ ID NO:22, (ii) HVR-L2 comprising the amino acid sequence of SEQ ID NO:23, and (c) HVR-L3 comprising the amino acid sequence of SEQ ID NO:24.

In another aspect, an antibody of the invention comprises (a) a VH domain comprising at least one, at least two, or all three VH HVR sequences selected from (i) HVR-H1 comprising the amino acid sequence of SEQ ID NO:26, (ii) HVR-H2 comprising the amino acid sequence of SEQ ID NO:27, and (iii) HVR-H3 comprising an amino acid sequence of SEQ ID NO:28; and (b) a VL domain comprising at least one, at least two, or all three VL HVR sequences selected from (i) HVR-L1 comprising the amino acid sequence of SEQ ID NO:30, (ii) HVR-L2 comprising the amino acid sequence of SEQ ID NO:31, and (c) HVR-L3 comprising the amino acid sequence of SEQ ID NO:32.

In another aspect, an antibody of the invention comprises (a) a VH domain comprising at least one, at least two, or all three VH HVR sequences selected from (i) HVR-H1 comprising the amino acid sequence of SEQ ID NO:34, (ii) HVR-H2 comprising the amino acid sequence of SEQ ID NO:35, and (iii) HVR-H3 comprising an amino acid sequence of SEQ ID NO:36; and (b) a VL domain comprising at least one, at least two, or all three VL HVR sequences selected from (i) HVR-L1 comprising the amino acid sequence of SEQ ID NO:38, (ii) HVR-L2 comprising the amino acid sequence of SEQ ID NO:39, and (c) HVR-L3 comprising the amino acid sequence of SEQ ID NO:40.

In another aspect, an antibody of the invention comprises (a) a VH domain comprising at least one, at least two, or all three VH HVR sequences selected from (i) HVR-H1 comprising the amino acid sequence of SEQ ID NO:42, (ii) HVR-H2 comprising the amino acid sequence of SEQ ID NO:43, and (iii) HVR-H3 comprising an amino acid sequence of SEQ ID NO:44; and (b) a VL domain comprising at least one, at least two, or all three VL HVR sequences selected from (i) HVR-L1 comprising the amino acid sequence of SEQ ID NO:46, (ii) HVR-L2 comprising the amino acid sequence of SEQ ID NO:47, and (c) HVR-L3 comprising the amino acid sequence of SEQ ID NO:48.

In another aspect, an antibody of the invention comprises (a) a VH domain comprising at least one, at least two, or all three VH HVR sequences selected from (i) HVR-H1 comprising the amino acid sequence of SEQ ID NO:50, (ii) HVR-H2 comprising the amino acid sequence of SEQ ID NO:51, and (iii) HVR-H3 comprising an amino acid sequence of SEQ ID NO:52; and (b) a VL domain comprising at least one, at least two, or all three VL HVR sequences selected from (i) HVR-L1 comprising the amino acid sequence of SEQ ID NO:54, (ii) HVR-L2 comprising the amino acid sequence of SEQ ID NO:55, and (c) HVR-L3 comprising the amino acid sequence of SEQ ID NO:56.

In another aspect, an antibody of the invention comprises (a) a VH domain comprising at least one, at least two, or all three VH HVR sequences selected from (i) HVR-H1 comprising the amino acid sequence of SEQ ID NO:58, (ii) HVR-H2 comprising the amino acid sequence of SEQ ID NO:59, and (iii) HVR-H3 comprising an amino acid sequence of SEQ ID NO:60; and (b) a VL domain comprising at least one, at least two, or all three VL HVR sequences selected from (i) HVR-L1 comprising the amino acid sequence of SEQ ID NO:62, (ii) HVR-L2 comprising the amino acid sequence of SEQ ID NO:63, and (c) HVR-L3 comprising the amino acid sequence of SEQ ID NO:64.

In another aspect, an antibody of the invention comprises (a) a VH domain comprising at least one, at least two, or all three VH HVR sequences selected from (i) HVR-H1 comprising the amino acid sequence of SEQ ID NO:66, (ii) HVR-H2 comprising the amino acid sequence of SEQ ID NO:67, and (iii) HVR-H3 comprising an amino acid sequence of SEQ ID NO:68; and (b) a VL domain comprising at least one, at least two, or all three VL HVR sequences selected from (i) HVR-L1 comprising the amino acid sequence of SEQ ID NO:70, (ii) HVR-L2 comprising the amino acid sequence of SEQ ID NO:71, and (c) HVR-L3 comprising the amino acid sequence of SEQ ID NO:72.

In another aspect, the invention provides an antibody comprising HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8.

The antibody DAB0065 exemplified in the Examples comprises these hypervariable regions. DAB0065 has been shown to be particularly effective in a mouse model for fibrotic diseases (see example section). Consequently, in certain embodiments, the invention is directed to antibodies comprising the hypervariable regions, to antibodies binding to the same epitope and to medical uses of these antibodies, in particular in the treatment of fibrotic diseases.

In another aspect, the invention provides an antibody comprising HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16.

In another aspect, the invention provides an antibody comprising HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24.

In another aspect, the invention provides an antibody comprising HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32.

In another aspect, the invention provides an antibody comprising HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40.

In another aspect, the invention provides an antibody comprising HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48.

In another aspect, the invention provides an antibody comprising HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56.

In another aspect, the invention provides an antibody comprising HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64.

In another aspect, the invention provides an antibody comprising HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

In any of the above embodiments, an anti-DDR2 antibody is humanized. In one embodiment, an anti-DDR2 antibody comprises HVRs as in any of the above embodiments, and further comprises an acceptor human framework, e.g. a human immunoglobulin framework or a human consensus framework.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 1. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:1. In certain embodiments, substitutions, insertions, or deletions occur in regions outside the HVRs (i.e., in the FRs). Optionally, the anti-DDR2 antibody comprises the VH sequence in SEQ ID NO:1, including post-translational modifications of that sequence. In a particular embodiment, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:9. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:9. In certain embodiments, substitutions, insertions, or deletions occur in regions outside the HVRs (i.e., in the FRs). Optionally, the anti-DDR2 antibody comprises the VH sequence in SEQ ID NO:9, including post-translational modifications of that sequence. In a particular embodiment, the VH comprises one, two or three HVRs selected from: (a) HVR-H1 comprising the amino acid sequence of SEQ ID NO:10, (b) HVR-H2 comprising the amino acid sequence of SEQ ID NO:11, and (c) HVR-H3 comprising the amino acid sequence of SEQ ID NO:12.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:17. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:17. In certain embodiments, substitutions, insertions, or deletions occur in regions outside the HVRs (i.e., in the FRs). Optionally, the anti-DDR2 antibody comprises the VH sequence in SEQ ID NO:17, including post-translational modifications of that sequence. In a particular embodiment, the VH comprises one, two or three HVRs selected from: (a) HVR-H1 comprising the amino acid sequence of SEQ ID NO:18, (b) HVR-H2 comprising the amino acid sequence of SEQ ID NO:19, and (c) HVR-H3 comprising the amino acid sequence of SEQ ID NO:20.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:25. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:25. In certain embodiments, substitutions, insertions, or deletions occur in regions outside the HVRs (i.e., in the FRs). Optionally, the anti-DDR2 antibody comprises the VH sequence in SEQ ID NO:25, including post-translational modifications of that sequence. In a particular embodiment, the VH comprises one, two or three HVRs selected from: (a) HVR-H1 comprising the amino acid sequence of SEQ ID NO:26, (b) HVR-H2 comprising the amino acid sequence of SEQ ID NO:27, and (c) HVR-H3 comprising the amino acid sequence of SEQ ID NO:28.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:33. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:33. In certain embodiments, substitutions, insertions, or deletions occur in regions outside the HVRs (i.e., in the FRs). Optionally, the anti-DDR2 antibody comprises the VH sequence in SEQ ID NO:33, including post-translational modifications of that sequence. In a particular embodiment, the VH comprises one, two or three HVRs selected from: (a) HVR-H1 comprising the amino acid sequence of SEQ ID NO:34, (b) HVR-H2 comprising the amino acid sequence of SEQ ID NO:35, and (c) HVR-H3 comprising the amino acid sequence of SEQ ID NO:36.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:41. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:41. In certain embodiments, substitutions, insertions, or deletions occur in regions outside the HVRs (i.e., in the FRs). Optionally, the anti-DDR2 antibody comprises the VH sequence in SEQ ID NO:41, including post-translational modifications of that sequence. In a particular embodiment, the VH comprises one, two or three HVRs selected from: (a) HVR-H1 comprising the amino acid sequence of SEQ ID NO:42, (b) HVR-H2 comprising the amino acid sequence of SEQ ID NO:43, and (c) HVR-H3 comprising the amino acid sequence of SEQ ID NO:44.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:49. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:49. In certain embodiments, substitutions, insertions, or deletions occur in regions outside the HVRs (i.e., in the FRs). Optionally, the anti-DDR2 antibody comprises the VH sequence in SEQ ID NO:49, including post-translational modifications of that sequence. In a particular embodiment, the VH comprises one, two or three HVRs selected from: (a) HVR-H1 comprising the amino acid sequence of SEQ ID NO:50, (b) HVR-H2 comprising the amino acid sequence of SEQ ID NO:51, and (c) HVR-H3 comprising the amino acid sequence of SEQ ID NO:52.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:57. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:57. In certain embodiments, substitutions, insertions, or deletions occur in regions outside the HVRs (i.e., in the FRs). Optionally, the anti-DDR2 antibody comprises the VH sequence in SEQ ID NO:57, including post-translational modifications of that sequence. In a particular embodiment, the VH comprises one, two or three HVRs selected from: (a) HVR-H1 comprising the amino acid sequence of SEQ ID NO:58, (b) HVR-H2 comprising the amino acid sequence of SEQ ID NO:59, and (c) HVR-H3 comprising the amino acid sequence of SEQ ID NO:60.

In another aspect, an anti-DDR2 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:65. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:65. In certain embodiments, substitutions, insertions, or deletions occur in regions outside the HVRs (i.e., in the FRs). Optionally, the anti-DDR2 antibody comprises the VH sequence in SEQ ID NO:65, including post-translational modifications of that sequence. In a particular embodiment, the VH comprises one, two or three HVRs selected from: (a) HVR-H1 comprising the amino acid sequence of SEQ ID NO:66, (b) HVR-H2 comprising the amino acid sequence of SEQ ID NO:67, and (c) HVR-H3 comprising the amino acid sequence of SEQ ID NO:68.

Post-translational modifications include but are not limited to a modification of glutamine or glutamate in N-terminal of heavy chain or light chain to pyroglutamic acid by pyroglutamylation.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:5. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:5. In certain embodiments, the substitutions, insertions, or deletions occur in regions outside the HVRs (i.e., in the FRs). Optionally, the anti-DDR2 antibody comprises the VL sequence in SEQ ID NO:5, including post-translational modifications of that sequence. In a particular embodiment, the VL comprises one, two or three HVRs selected from (a) HVR-L1 comprising the amino acid sequence of SEQ ID NO:6, (b) HVR-L2 comprising the amino acid sequence of SEQ ID NO:7, and (c) HVR-L3 comprising the amino acid sequence of SEQ ID NO:8.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 13. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:13. In certain embodiments, the substitutions, insertions, or deletions occur in regions outside the HVRs (i.e., in the FRs). Optionally, the anti-DDR2 antibody comprises the VL sequence in SEQ ID NO: 13, including post-translational modifications of that sequence. In a particular embodiment, the VL comprises one, two or three HVRs selected from (a) HVR-L1 comprising the amino acid sequence of SEQ ID NO: 14, (b) HVR-L2 comprising the amino acid sequence of SEQ ID NO: 15, and (c) HVR-L3 comprising the amino acid sequence of SEQ ID NO: 16.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:21. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:21. In certain embodiments, the substitutions, insertions, or deletions occur in regions outside the HVRs (i.e., in the FRs). Optionally, the anti-DDR2 antibody comprises the VL sequence in SEQ ID NO:21, including post-translational modifications of that sequence. In a particular embodiment, the VL comprises one, two or three HVRs selected from (a) HVR-L1 comprising the amino acid sequence of SEQ ID NO:22, (b) HVR-L2 comprising the amino acid sequence of SEQ ID NO:23, and (c) HVR-L3 comprising the amino acid sequence of SEQ ID NO:24.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:29. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:29 In certain embodiments, the substitutions, insertions, or deletions occur in regions outside the HVRs (i.e., in the FRs). Optionally, the anti-DDR2 antibody comprises the VL sequence in SEQ ID NO:29, including post-translational modifications of that sequence. In a particular embodiment, the VL comprises one, two or three HVRs selected from (a) HVR-L1 comprising the amino acid sequence of SEQ ID NO:30, (b) HVR-L2 comprising the amino acid sequence of SEQ ID NO:31, and (c) HVR-L3 comprising the amino acid sequence of SEQ ID NO:32.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:37. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:37. In certain embodiments, the substitutions, insertions, or deletions occur in regions outside the HVRs (i.e., in the FRs). Optionally, the anti-DDR2 antibody comprises the VL sequence in SEQ ID NO:37, including post-translational modifications of that sequence. In a particular embodiment, the VL comprises one, two or three HVRs selected from (a) HVR-L1 comprising the amino acid sequence of SEQ ID NO:38, (b) HVR-L2 comprising the amino acid sequence of SEQ ID NO:39, and (c) HVR-L3 comprising the amino acid sequence of SEQ ID NO:40.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:45. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:45. In certain embodiments, the substitutions, insertions, or deletions occur in regions outside the HVRs (i.e., in the FRs). Optionally, the anti-DDR2 antibody comprises the VL sequence in SEQ ID NO:45, including post-translational modifications of that sequence. In a particular embodiment, the VL comprises one, two or three HVRs selected from (a) HVR-L1 comprising the amino acid sequence of SEQ ID NO:46, (b) HVR-L2 comprising the amino acid sequence of SEQ ID NO:47, and (c) HVR-L3 comprising the amino acid sequence of SEQ ID NO:48.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:53. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:53. In certain embodiments, the substitutions, insertions, or deletions occur in regions outside the HVRs (i.e., in the FRs). Optionally, the anti-DDR2 antibody comprises the VL sequence in SEQ ID NO:53, including post-translational modifications of that sequence. In a particular embodiment, the VL comprises one, two or three HVRs selected from (a) HVR-L1 comprising the amino acid sequence of SEQ ID NO:54, (b) HVR-L2 comprising the amino acid sequence of SEQ ID NO:55, and (c) HVR-L3 comprising the amino acid sequence of SEQ ID NO:56.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:61. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:61. In certain embodiments, the substitutions, insertions, or deletions occur in regions outside the HVRs (i.e., in the FRs). Optionally, the anti-DDR2 antibody comprises the VL sequence in SEQ ID NO:61, including post-translational modifications of that sequence. In a particular embodiment, the VL comprises one, two or three HVRs selected from (a) HVR-L1 comprising the amino acid sequence of SEQ ID NO:62, (b) HVR-L2 comprising the amino acid sequence of SEQ ID NO:63, and (c) HVR-L3 comprising the amino acid sequence of SEQ ID NO:64.

In another aspect, an anti-DDR2 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:69. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-DDR2 antibody comprising that sequence retains the ability to bind to DDR2. In certain embodiments, a total of 1 to 10 amino acids have been substituted, inserted and/or deleted in SEQ ID NO:69. In certain embodiments, the substitutions, insertions, or deletions occur in regions outside the HVRs (i.e., in the FRs). Optionally, the anti-DDR2 antibody comprises the VL sequence in SEQ ID NO:69, including post-translational modifications of that sequence. In a particular embodiment, the VL comprises one, two or three HVRs selected from (a) HVR-L1 comprising the amino acid sequence of SEQ ID NO:70, (b) HVR-L2 comprising the amino acid sequence of SEQ ID NO:71, and (c) HVR-L3 comprising the amino acid sequence of SEQ ID NO:72.

Post-translational modifications include but are not limited to a modification of glutamine or glutamate in N-terminal of heavy chain or light chain to pyroglutamic acid by pyroglutamylation.

In another aspect, an anti-DDR2 antibody is provided, wherein the antibody comprises a VH as in any of the embodiments provided above, and a VL as in any of the embodiments provided above. In one embodiment, the antibody comprises the VH and VL sequences in SEQ ID NO:1 and SEQ ID NO:5, respectively, optionally including post-translational modifications of those sequences. In one embodiment, the antibody comprises the VH and VL sequences in SEQ ID NO:9 and SEQ ID NO: 13, respectively, optionally including post-translational modifications of those sequences. In one embodiment, the antibody comprises the VH and VL sequences in SEQ ID NO: 17 and SEQ ID NO:21, respectively, optionally including post-translational modifications of those sequences. In one embodiment, the antibody comprises the VH and VL sequences in SEQ ID NO:25 and SEQ ID NO:29, respectively, optionally including post-translational modifications of those sequences. In one embodiment, the antibody comprises the VH and VL sequences in SEQ ID NO:33 and SEQ ID NO:37, respectively, optionally including post-translational modifications of those sequences. In one embodiment, the antibody comprises the VH and VL sequences in SEQ ID NO:41 and SEQ ID NO:45, respectively, optionally including post-translational modifications of those sequences. In one embodiment, the antibody comprises the VH and VL sequences in SEQ ID NO:49 and SEQ ID NO:53, respectively, optionally including post-translational modifications of those sequences. In one embodiment, the antibody comprises the VH and VL sequences in SEQ ID NO:57 and SEQ ID NO:61, respectively, optionally including post-translational modifications of those sequences. In one embodiment, the antibody comprises the VH and VL sequences in SEQ ID NO:65 and SEQ ID NO:69, respectively, optionally including post-translational modifications of those sequences. Post-translational modifications include but are not limited to a modification of glutamine or glutamate in N-terminal of heavy chain or light chain to pyroglutamic acid by pyroglutamylation.

In a further aspect, the invention provides an antibody that binds to the same epitope as an anti-DDR2 antibody provided herein.

For example, in certain embodiments, an antibody is provided that binds to the same epitope as (1) the antibody comprises the VH and VL sequences in SEQ ID NO: 1 and SEQ ID NO:5, respectively; (2) the antibody comprises the VH and VL sequences in SEQ ID NO:9 and SEQ ID NO: 13, respectively; (3) the antibody comprises the VH and VL sequences in SEQ ID NO: 17 and SEQ ID NO:21, respectively; (4) the antibody comprises the VH and VL sequences in SEQ ID NO:25 and SEQ ID NO:29, respectively; (5) the antibody comprises the VH and VL sequences in SEQ ID NO:33 and SEQ ID NO:37, respectively; (6) the antibody comprises the VH and VL sequences in SEQ ID NO:41 and SEQ ID NO:45, respectively; (7) the antibody comprises the VH and VL sequences in SEQ ID NO:49 and SEQ ID NO:53, respectively; (8) the antibody comprises the VH and VL sequences in SEQ ID NO:57 and SEQ ID NO:61, respectively; or (9) the antibody comprises the VH and VL sequences in SEQ ID NO:65 and SEQ ID NO:69, respectively.

For example, in certain embodiments, an antibody is provided that binds to the same epitope as

(1) the antibody that comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8;

(2) the antibody that comprises HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16;

(3) the antibody that comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24;

(4) the antibody that comprises HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32;

(5) the antibody that comprises HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40;

(6) the antibody that comprises HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48;

(7) the antibody that comprises HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56;

(8) the antibody that comprises HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; or

(9) the antibody that comprises HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

In a further aspect, the invention provides an antibody that competes with an anti-DDR2 antibody provided herein in binding towards DDR2.

For example, in certain embodiments, an antibody is provided that competes with (1) the antibody that comprises the VH and VL sequences in SEQ ID NO:1 and SEQ ID NO:5, respectively; (2) the antibody that comprises the VH and VL sequences in SEQ ID NO:9 and SEQ ID NO:13, respectively; (3) the antibody that comprises the VH and VL sequences in SEQ ID NO:17 and SEQ ID NO:21, respectively; (4) the antibody that comprises the VH and VL sequences in SEQ ID NO:25 and SEQ ID NO:29, respectively; (5) the antibody that comprises the VH and VL sequences in SEQ ID NO:33 and SEQ ID NO:37, respectively; (6) the antibody that comprises the VH and VL sequences in SEQ ID NO:41 and SEQ ID NO:45, respectively; (7) the antibody that comprises the VH and VL sequences in SEQ ID NO:49 and SEQ ID NO:53, respectively; (8) the antibody that comprises the VH and VL sequences in SEQ ID NO:57 and SEQ ID NO:61, respectively; or (9) the antibody that comprises the VH and VL sequences in SEQ ID NO:65 and SEQ ID NO:69, respectively; in binding towards DDR2.

For example, in certain embodiments, an antibody is provided that competes with

(1) the antibody that comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8;

(2) the antibody that comprises HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16;

(3) the antibody that comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24;

(4) the antibody that comprises HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32;

(5) the antibody that comprises HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40;

(6) the antibody that comprises HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48;

(7) the antibody that comprises HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56;

(8) the antibody that comprises HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; or

(9) the antibody that comprises HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72;

in binding towards DDR2.

In a further aspect of the invention, an anti-DDR2 antibody according to any of the above embodiments is a monoclonal antibody, including a chimeric, humanized or human antibody. In one embodiment, an anti-DDR2 antibody is an antibody fragment, e.g., a Fv, Fab, Fab', scFv, diabody, or $F(ab')_2$ fragment. In another embodiment, the antibody is a full length antibody, e.g., an intact IgG1, IgG2, IgG3 or IgG4 antibody, or other antibody class or isotype as defined herein.

In a further aspect of the invention, an anti-DDR2 antibody according to any of the above embodiments comprises human antibody constant regions, such as human heavy chain constant region SG181 (SEQ ID NO: 73) and human light chain constant region k0MC (SEQ ID NO: 74). In another embodiment, an anti-DDR2 antibody according to any of the above embodiments comprises mouse antibody constant regions, such as mouse heavy chain constant region mF18 (SEQ ID NO: 75) and mouse light chain constant region mk1 (SEQ ID NO: 76).

In a further aspect, an anti-DDR2 antibody is provided, wherein the heavy chain of the antibody has at least 90%, 91%, 92%, 93%, 94%, 95%, 96%, 97%, 98%, 99% or 100% sequence identity to the amino acid sequence of SEQ ID NO: 85, and the light chain of the antibody has at least 90%, 91%, 92%, 93%, 94%, 95%, 96%, 97%, 98%, 99% or 100% sequence identity to the amino acid sequence of SEQ ID NO: 86. In a preferred embodiment, the antibody comprises the heavy chain and light chain sequences in SEQ ID NO:85 and SEQ ID NO:86, respectively, optionally including post-translational modifications of those sequences. In a preferred embodiment, the invention provides an antibody that binds to the same epitope as an anti-DDR2 antibody that comprises the heavy chain and light chain sequences in SEQ ID NO:85 and SEQ ID NO:86, respectively. In a preferred embodiment, the invention provides an antibody that competes with an anti-DDR2 antibody that comprises the heavy chain and light chain sequences in SEQ ID NO:85 and SEQ ID NO:86, respectively.

In a further aspect, an anti-DDR2 antibody according to any of the above embodiments may incorporate any of the features, singly or in combination, as described in Sections 1-7 below:

## 1. Antibody Affinity

In certain embodiments, an antibody provided herein has a dissociation constant (Kd) of 1 micro M or less, 100 nM or less, 10 nM or less, 1 nM or less, 0.1 nM or less, 0.01 nM or less, or 0.001 nM or less (e.g. $10^{-8}$M or less, e.g. from $10^{-8}$M to $10^{-13}$M, e.g., from $10^{-9}$M to $10^{-13}$ M).

In one embodiment, Kd is measured by a radiolabeled antigen binding assay **(RIA)**. In one embodiment, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 micro g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 micro l/well of scintillant (MICROSCINT-20 ™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using a BIACORE (registered trademark) surface plasmon resonance assay. For example, an assay using a BIACORE (registered trademark)-2000 or a BIACORE(registered trademark)-3000 (BIAcore, Inc., Piscataway, NJ) is performed at 25 degrees C with immobilized antigen CM5 chips at ~ 10 response units (RU). In one embodiment, carboxymethylated dextran biosensor chips (CM5, BIACORE, Inc.) are activated with *N*-ethyl-*N'*- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 micro g/ml (~0.2 micro M) before injection at a flow rate of 5 micro l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25 degrees C at a flow rate of approximately 25 micro l/min. Association rates ($k_{on}$) and dissociation rates ($k_{off}$) are calculated using a simple one-to-one Langmuir binding model (BIACORE (registered trademark) Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 degrees C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO™ spectrophotometer (ThermoSpectronic) with a stirred cuvette.

## 2. Antibody Fragments

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. *J. Immunol.* 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMAB (registered trademark) technology; U.S. Patent No. 7,041,870 describing K-M MOUSE (registered trademark) technology, and U.S. Patent Application Publication No. US 2007/0061900, describing VELOCIMOUSE (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. *Library-Derived Antibodies*

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. *Multispecific Antibodies*

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for DDR2 and the other is for any other antigen. In certain embodiments, bispecific antibodies may bind to two different epitopes of DDR2. Bispecific antibodies may also be used to localize cytotoxic agents to cells which express DDR2. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting Fab" or "DAF" comprising an antigen binding site that binds to DDR2 as well as another, different antigen (see, US 2008/0069820, for example).

**7. Antibody Variants**

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

**a) Substitution, Insertion, and Deletion Variants**

In certain embodiments, antibody variants having one or more amino acid substitutions are provided. Sites of interest for substitutional mutagenesis include the HVRs and FRs. Conservative substitutions are shown in Table A under the heading of "preferred substitutions." More substantial changes are provided in Table A under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

[TABLE A]

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

## b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al*.).* Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'lAcad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1™ non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

#### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

#### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-DDR2 antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making an anti-DDR2 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-DDR2 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

C. Assays

Anti-DDR2 antibodies provided herein may be identified, screened for, or characterized for their physical/chemical properties and/or biological activities by various assays known in the art.

*1. Binding assays and other assays*

In one aspect, an antibody of the invention is tested for its antigen binding activity, e.g., by known methods such as ELISA, Western blot, etc.

In another aspect, competition assays may be used to identify an antibody that competes with (1) the antibody that comprises the VH and VL sequences in SEQ ID NO: 1 and SEQ ID NO:5, respectively; (2) the antibody that comprises the VH and VL sequences in SEQ ID NO:9 and SEQ ID NO: 13, respectively; (3) the antibody that comprises the VH and VL sequences in SEQ ID NO: 17 and SEQ ID NO:21, respectively; (4) the antibody that comprises the VH and VL sequences in SEQ ID NO:25 and SEQ ID NO:29, respectively; (5) the antibody that comprises the VH and VL sequences in SEQ ID NO:33 and SEQ ID NO:37, respectively; (6) the antibody that comprises the VH and VL sequences in SEQ ID NO:41 and SEQ ID NO:45, respectively; (7) the antibody that comprises the VH and VL sequences in SEQ ID NO:49 and SEQ ID NO:53, respectively; (8) the antibody that comprises the VH and VL sequences in SEQ ID NO:57 and SEQ ID NO:61, respectively; or (9) the antibody that comprises the VH and VL sequences in SEQ ID NO:65 and SEQ ID NO:69, respectively; for binding to DDR2. In certain embodiments, such a competing antibody binds to the same epitope (e.g., a linear or a conformational epitope) that is bound by (1) the antibody that comprises the VH and VL sequences in SEQ ID NO: 1 and SEQ ID NO:5, respectively; (2) the antibody that comprises the VH and VL sequences in SEQ ID NO:9 and SEQ ID NO:13, respectively; (3) the antibody that comprises the VH and VL sequences in SEQ ID NO:17 and SEQ ID NO:21, respectively; (4) the antibody that comprises the VH and VL sequences in SEQ ID NO:25 and SEQ ID NO:29, respectively; (5) the antibody that comprises the VH and VL sequences in SEQ ID NO:33 and SEQ ID NO:37, respectively; (6) the antibody that comprises the VH and VL sequences in SEQ ID NO:41 and SEQ ID NO:45, respectively; (7) the antibody that comprises the VH and VL sequences in SEQ ID NO:49 and SEQ ID NO:53, respectively; (8) the antibody that comprises the VH and VL sequences in SEQ ID NO:57 and SEQ ID NO:61, respectively; or (9) the antibody that comprises the VH and VL sequences in SEQ ID NO:65 and SEQ ID NO:69, respectively.

In another aspect, competition assays may be used to identify an antibody that competes with (1) the antibody that comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8; (2) the antibody that comprises HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16; (3) the antibody that comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24; (4) the antibody that comprises HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32; (5) the antibody that comprises HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40; (6) the antibody that comprises HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48; (7) the antibody that comprises HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56; (8) the antibody that comprises HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; or (9) the antibody that comprises HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72; for binding to DDR2. In certain embodiments, such a competing antibody binds to the same epitope (e.g., a linear or a conformational epitope) that is bound by (1) the antibody that comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8; (2) the antibody that comprises HVR-H1 comprising the amino acid sequence of SEQ ID NO:10, HVR-H2 comprising the amino acid sequence of SEQ ID NO:11, HVR-H3 comprising the amino acid sequence of SEQ ID NO:12, HVR-L1 comprising the amino acid sequence of SEQ ID NO:14, HVR-L2 comprising the amino acid sequence of SEQ ID NO:15, and HVR-L3 comprising the amino acid sequence of SEQ ID NO:16; (3) the antibody that comprises HVR-H1 comprising the amino acid sequence of SEQ ID NO:18, HVR-H2 comprising the amino acid sequence of SEQ ID NO:19, HVR-H3 comprising the amino acid sequence of SEQ ID NO:20, HVR-L1 comprising the amino acid sequence of SEQ ID NO:22, HVR-L2 comprising the amino acid sequence of SEQ ID NO:23, and HVR-L3 comprising the amino acid sequence of SEQ ID NO:24; (4) the antibody that comprises HVR-H1 comprising the amino acid sequence of SEQ ID NO:26, HVR-H2 comprising the amino acid sequence of SEQ ID NO:27, HVR-H3 comprising the amino acid sequence of SEQ ID NO:28, HVR-L1 comprising the amino acid sequence of SEQ ID NO:30, HVR-L2 comprising the amino acid sequence of SEQ ID NO:31, and HVR-L3 comprising the amino acid sequence of SEQ ID NO:32; (5) the antibody that comprises HVR-H1 comprising the amino acid sequence of SEQ ID NO:34, HVR-H2 comprising the amino acid sequence of SEQ ID NO:35, HVR-H3 comprising the amino acid sequence of SEQ ID NO:36, HVR-L1 comprising the amino acid sequence of SEQ ID NO:38, HVR-L2 comprising the amino acid sequence of SEQ ID NO:39, and HVR-L3 comprising the amino acid sequence of SEQ ID NO:40; (6) the antibody that comprises HVR-H1 comprising the amino acid sequence of SEQ ID NO:42, HVR-H2 comprising the amino acid sequence of SEQ ID NO:43, HVR-H3 comprising the amino acid sequence of SEQ ID NO:44, HVR-L1 comprising the amino acid sequence of SEQ ID NO:46, HVR-L2 comprising the amino acid sequence of SEQ ID NO:47, and HVR-L3 comprising the amino acid sequence of SEQ ID NO:48; (7) the antibody that comprises HVR-H1 comprising the amino acid sequence of SEQ ID NO:50, HVR-H2 comprising the amino acid sequence of SEQ ID NO:51, HVR-H3 comprising the amino acid sequence of SEQ ID NO:52, HVR-L1 comprising the amino acid sequence of SEQ ID NO:54, HVR-L2 comprising the amino acid sequence of SEQ ID NO:55, and HVR-L3 comprising the amino acid sequence of SEQ ID NO:56; (8) the antibody that comprises HVR-H1 comprising the amino acid sequence of SEQ ID NO:58, HVR-H2 comprising the amino acid sequence of SEQ ID NO:59, HVR-H3 comprising the amino acid sequence of SEQ ID NO:60, HVR-L1 comprising the amino acid sequence of SEQ ID NO:62, HVR-L2 comprising the amino acid sequence of SEQ ID NO:63, and HVR-L3 comprising the amino acid sequence of SEQ ID NO:64; or (9) the antibody that comprises HVR-H1 comprising the amino acid sequence of SEQ ID NO:66, HVR-H2 comprising the amino acid sequence of SEQ ID NO:67, HVR-H3 comprising the amino acid sequence of SEQ ID NO:68, HVR-L1 comprising the amino acid sequence of SEQ ID NO:70, HVR-L2 comprising the amino acid sequence of SEQ ID NO:71, and HVR-L3 comprising the amino acid sequence of SEQ ID NO:72.

Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized DDR2 is incubated in a solution comprising a first labeled antibody that binds to DDR2 (e.g., any of the antibody listed in the previous paragraph) and a second unlabeled antibody that is being tested for its ability to compete with the first antibody for binding to DDR2. The second antibody may be present in a hybridoma supernatant. As a control, immobilized DDR2 is incubated in a solution comprising the first labeled antibody but not the second unlabeled antibody. After incubation under conditions permissive for binding of the first antibody to DDR2, excess unbound antibody is removed, and the amount of label associated with immobilized DDR2 is measured. If the amount of label associated with immobilized DDR2 is substantially reduced in the test sample relative to the control sample, then that indicates that the second antibody is competing with the first antibody for binding to DDR2. See Harlow and Lane (1988) Antibodies: A Laboratory Manual ch. 14 (Cold Spring Harbor Laboratory, Cold Spring Harbor, NY).

### 2. Activity assays

In one aspect, assays are provided for identifying anti-DDR2 antibodies having biological activity. Biological activity may include, e.g., reducing collagen type 1 alpha 1 (Col1a1) mRNA level, and/or fibronectin 1 (Fn1) mRNA level, in a subject. Antibodies having such biological activity in vivo are also provided.

In certain embodiments, an antibody of the invention is tested for such biological activity.

For example, in one embodiment, antibody's biological activity to reduce collagen type 1 alpha 1 (Col1a1) mRNA level is evaluated in UUO (unilateral ureteral obstruction) mouse model which develops progressive renal fibrosis. Mice (e.g., specific pathogen-free C57BL/6NTac male mice of 6-7 weeks of age) are acclimated for about 1 week before the start of treatments. Animals are maintained at 20 - 26 degrees C with a 12:12 hour light/dark cycle and fed with a commercial standard diet (e.g., 5P75; PMI Nutrition INT'L (LabDiet), Missouri, United States) and tap water ad libitum.

UUO surgery is operated under anesthetized condition (e.g., using isoflurane). The left side of the abdomen is shaved, and a vertical incision is made through the skin. A second incision is made through the peritoneum and that skin is also retracted to reveal the kidney. Using forceps, the kidney is brought to the surface and the left ureter is tied with surgical silk, twice, below the kidney. The ligated kidney is placed gently back into its correct anatomical position then peritoneum and skin are sutured. Analgesic agent is added to reduce animal affliction. In sham operated group, peritoneum and skin is only incised and sutured.

Test antibody or negative control antibody (such as IC17, an anti-KLH antibody) is administered by intraperitoneal injection two times, for example, one day after the surgical operation and one day before the harvest. For sham operated group, vehicle is administered. The animals are weighed and then killed by exsanguination under anaesthesia. Blood samples are collected, for example, from the heart cavities or the postcaval vein and maintained at -80 degrees C until assayed. The kidney is quickly removed. Part of the kidney tissue is snap-frozen in liquid nitrogen or on dry ice for molecular analyses.

Total RNA is extracted from kidney tissues, for example, using RNeasy Mini Kit (Qiagen). Mouse mitochondrial ribosomal protein L19 (Mrpl19) or other suitable gene is used as the endogenous reference for each sample. Antibody's biological activity to reduce collagen type 1 alpha 1 (Col1a1) mRNA level is evaluated by measuring collagen type 1 alpha 1 (Col1a1) mRNA levels in kidney, comparing the level in test antibody-administered animal with that of negative control antibody-administered animal. Relative mRNA expression values are calculated using double delta Ct analysis.

In another embodiment, antibody's biological activity to reduce collagen type 1 alpha 1 (Col1a1) mRNA level and antibody's biological activity to reduce fibronectin 1 (Fn1) mRNA level is evaluated in bleomycin (BLM)-induced mouse lung fibrosis model (BLM mouse model), which is characterized by the infiltration of leukocytes, fibroblast proliferation, and an increase in collagen within the lung tissue.

Mice (e.g., specific pathogen-free C57BL/6NTac male mice of 6-7 weeks of age) are acclimated for about 1 week before the start of treatments. Animals are maintained at 20 - 26 degree C with a 12:12 hour light/dark cycle and fed with a commercial standard diet (e.g., 5P75; PMI Nutrition INT'L (LabDiet), Missouri, United States) and tap water ad libitum.

Bleomycin Sulfate (can be purchased from Gold Bio, St Louis, US (Cat.No. B-910-50)) or saline is administered, for example, through Osmotic minipump (Alzet, Cupertino, CA, USA, MODEL 1007D). On day 0, minipumps are loaded with BLM (50 mg/kg) dissolved in suitable volume of saline (e.g., 90 micro-L). Saline is administered for a non-diseased control group. Minipumps are incubated at 37 degrees C for about 3-4 hours before implantation. Pumps are implanted subcutaneously (s.c.) at the back through an incision under anesthesia (e.g., isoflurane). After a suitable interval has passed (e.g., on day 10), minipumps are removed from mice under anesthesia. Test antibody or negative control antibody (such as anti-KLH antibody IC17dK) is administered, for example, at 25 mg/kg by intravenous injection from day14 once every 4 to 3 days. The animals are weighed and then killed by exsanguination under isoflurane anaesthesia. Blood samples are collected , for example, from the heart cavity or the postcaval vein and housed at -80°C until assayed. The lung is quickly removed and weighed. Part of the lung tissue is snap-frozen in liquid nitrogen or on dry ice for molecular analyses.

Total RNA is extracted from lung tissues, for example, using RNeasy Mini Kit (Qiagen). Mouse mitochondrial ribosomal protein L19 (Mrpl19) or other suitable gene is used as the endogenous reference for each sample. Antibody's biological activity to reduce collagen type 1 alpha 1 (Col1a1) mRNA level is evaluated by measuring collagen type 1 alpha 1 (Col1a1) mRNA levels in lung, by comparing the level in test antibody-administered animal with that in negative control antibody-administered animal. Antibody's biological activity to reduce fibronectin 1 (Fn1) mRNA level is evaluated by measuring fibronectin 1 (Fn1) mRNA levels in lung, by comparing the level in test antibody-administered animal with that in negative control antibody-administered animal. Relative mRNA expression values were calculated using double delta Ct analysis.

### 3. Epitope Mapping

Epitope mapping is generally described as the process to identify or characterize the epitope on the antigen, to which an antibody specifically recognizes and binds to. Epitopes can be generally classified into two classes, linear (also known as continuous) or non-linear (also known as discontinuous) epitopes. Detailed exemplary methods for mapping an epitope to which an antibody binds are provided in Rockberg and Nilvebrant (2018) "Epitope Mapping Protocols, Third Edition", in Part of the Methods in Molecular Biology vol. 1785 (Humana Press, Totowa, NJ). Techniques to identify the amino acid residues involved in a linear epitope, i.e. linear epitope mapping, are publicly known, for example, by performing array-based oligo-peptide scanning.

In one embodiment, amino acid residues involved in the binding of an antibody and an antigen of interest can be determined by conducting array-based oligo-peptide scanning. Techniques to perform overlapping peptide scanning for epitope mapping are publicly known (Yone K et al. 1995, Geyson, H et al. 1984, Gey H et al. 1987). As an example, but without being limited thereto, to conduct oligo-peptide scanning, a library comprising oligo-peptide sequences from overlapping and non-overlapping segments of an antigen of interest may be prepared. In one embodiment, a library comprising a series of deletion fragments of the extracellular domain of an antigen of interest may be prepared. In one embodiment, a series of linear peptides non-overlapping but covering the entire extracellular domain of an antigen of interest may be prepared, and/or a series of linear peptides overlapping by one or more amino acid(s) and covering the entire extracellular domain of an antigen of interest may be prepared. In one embodiment, the series of peptides can be prepared as protein fusion peptides, such as GST-fusion, immunoglobulin-Fc fusion etc. Binding of antibody to each peptide within the library can be measured by a known method using flow cytometry, ELISA, Biacore or such, and the amino acid residues involved in binding of an antigen of interest and an antibody can be determined accordingly.

Techniques to identify the amino acid residues involved in a non-linear epitope, i.e. non-linear epitope mapping, are publicly known, for example, but without being limited thereto, by conducting crystal structure analysis or cryo-electron microscopy of the complex formed by an antibody and the antigen of interest, by introducing mutations and performing high throughput shotgun mutagenesis epitope mapping, or by performing hydrogen-deuterium exchange mass spectrometry.

In one embodiment, amino acid residues involved in the binding of an antibody and an antigen of interest can be determined by conducting crystal structure analysis of complexes formed by an antibody and the antigen of interest. Techniques to perform crystal structure analysis are publicly known (Padlan et al. 1989). As an example, but without being limited thereto, to perform crystal structure analysis, an antibody for crystallization is prepared and purified in appropriate buffer. Crystallization is carried out using various methods, such as for example, sitting drop vapor diffusion method and complexes of antibody and the antigen of interest is obtained. A single crystal of the complex is then frozen in liquid nitrogen or cryo-preserved. X-ray diffraction analysis is then performed to elucidate the structure of the complex of antibody and antigen of interest. Alternative methods such as cryo-electron microscopy, nanosecond microscopy or other methods capable of the same or substantially the same resolution may be suitably employed. Amino acids involved in the binding of an antibody to the antigen of interest is determined through analysis of the obtained antibody-antigen complex.

The phrase "involved in the binding of an antibody to the antigen of interest" as used herein refers to a condition where intermolecular interactions are taking place between the atoms of the main chain or side chains of the amino acids forming the antibody H chain or L chain and the atoms of the antigen at a distance that may have an effect on the binding activity; or a condition where certain amino acid residues are involved in the binding of the antigen, including an indirect effect of stabilizing the three-dimensional structure of the CDR loop and such to the conformation when bound to the antigen; and a condition that satisfies both of those conditions.

The "condition where intermolecular interactions are taking place" in the present specification can be determined based on the interatomic distances, for example, between non-hydrogen atoms constituting the main chain or side chains of the amino acids that form the antibody H chain or L chain and the non-hydrogen atoms constituting the antigen obtained from crystal structure analysis of the complex formed by the antigen and the antibody. For example, the above-mentioned interatomic distances are preferably 3.0 Å, 3.2 Å, 3.4 Å, 3.6 Å, 3.8 Å, 4.0 Å, 4.2 Å, 4.4 Å, 4.6 Å, 4.8 Å, or 5.0 Å or less, but are not limited thereto. More preferably, examples of the interatomic distance are 3.6 Å, 3.8 Å, 4.0 Å, or 4.2 Å or less.

In one embodiment, amino acid residues involved in the binding of an antibody and an antigen of interest can be determined by conducting high throughput shotgun mutagenesis. Techniques to perform high throughput shotgun mutagenesis are publicly known (Davidson E. et al., 2014). As an example, but without being limited thereto, to conduct high throughput shotgun mutagenesis, a library comprising of the antigen of interest and a plurality of variants of the antigen of interest may be prepared. The plurality of variants of the antigen of interest can be obtained by introducing single amino acid substitution at every amino acid residue position of the antigen of interest. Binding of each variant of the antigen of interest to an antibody can be measured by a known method using Biacore, flow cytometry or such, and the binding activity (affinity) of the variant of the antigen of interest to an antibody is calculated as a KD value. This KD value is compared to the KD value of the antigen of interest, i.e. unmodified antigen (template), and variants where the single amino acid substitution altered the binding greater than a certain standard is determined as sites that are involved in the binding of an antibody and the antigen of interest. Alternatively, rather than comparing the KD value, variants where the single amino acid substitution altered binding to an extent resulting in loss of binding, such as for example, complete loss of binding, between antibody and antigen is determined as sites that are involved in the binding of an antibody and the antigen of interest.

In the present invention, in determining amino acid residues involved in the binding of an antibody and an antigen of interest, "amino acid substitution that alter binding greater than a certain standard" has the meaning below. For example, as a result of performing measurements using known methods such as Biacore or flow cytometry, the binding activity (affinity) of each variant to the antibody is calculated as a KD value, and where alteration changes the binding capacity of the variant to the antibody by 1/100, 1/50, 1/25, 1/20, 1/10, 1/9, 1/8, 1/7, 1/6, 1/5, 1/4, 1/3, or 1/2 compared to that of the binding capacity of the antigen of interest, i.e. unmodified antigen (template), to the same antibody, the altered sites are determined as sites involved in the binding to an antibody, but the above-mentioned standards are non-limiting. Alternatively, rather than comparing the KD value of the variant to that of the antigen of interest, i.e. unmodified antigen (template), the binding activity (affinity) of each variant to the antibody is calculated as a KD value, and wherein binding capacity is lower than 10 mM, 1 mM, 100 uM, 10 uM, 1 uM, 100 nM, 10 nM, 1 nM, 100 pM, 10 pM, or 1 pM, the altered sites are determined as sites that are involved in the binding to an antibody, but the above-mentioned standards are non-limiting.

The binding activity of the antigen of interest, or plurality of variants to the antigen of interest, to an antibody can be measured by appropriately selecting methods known to those skilled in the art (Biacore, Flow cytometry, ELISA, ECL, and such).

In another embodiment, amino acid sites involved in the binding of an antibody and an antigen of interest, may be considered as amino acid sites other than any one or more amino acid sites selected from among the amino acid sites not involved in the binding of an antibody and an antigen of interest. In some instances, however, a KD value determined to be lower than a certain standard or a loss of binding observed may not indicate that the interrogated amino acid, i.e. amino acid that has been substituted, is involved in the binding of an antibody and an antigen of interest, but rather the reduced binding capacity or loss of binding is due to a loss in structural maintenance etc. Accordingly, it is considered that the person skilled in the art will readily and can reasonably determine whether the amino acid residues involved in the binding of an antibody and an antigen of interest by methods such as, for example, supplementing the analysis with crystal structure analysis of the antibody-antigen complex.

In one embodiment, amino acid residues involved in the binding of an antibody and an antigen of interest can be determined by conducting hydrogen-deuterium exchange mass spectrometry. Techniques to perform hydrogen-deuterium exchange mass spectrometry are publicly known (Houde D. et al. 2011, Karpusas M. et al. 1997). As an example, but without being limited thereto, to conduct hydrogen-deuterium exchange mass spectrometry, an antigen of interest and an antigen dimer-Fc antibody complex is incubated in solution that exposes both compounds to hydrogen-deuterium exchange. The deuterium labeling process is then quenched, and the resultant deuterium-labelled compounds are subjected to pepsin/trypsin digestion, which subsequently undergo mass spectrometry. Peptide mass fingerprints of unmodified, i.e. non-deuterium labelled (template) and deuterium-labelled antigen and antigen complexes are obtained. Amino acids involved in the binding of an antibody to the antigen of interest is determined through comparing the peptide mass fingerprints between the non-deuterium labelled (template) and deuterium-labelled antigen and antigen complexes.

In one embodiment, the amino acid residues involved in the binding of an anti-DDR2 antibody and mouse DDR2 is described in Example 6. Specific details of the methods used to determine the amino acid residues involved in the binding of the anti-DDR2 antibody and mouse DDR2 can be found in the mentioned examples, but the above-mentioned examples are non-limiting.

#### 4. Methods for obtaining or screening antibodies or antigen-binding domains whose antigen-binding properties is the same or substantially similar to an antibody of interest

The present disclosure includes methods for obtaining or screening for antibodies or antigen-binding domains whose antigen-binding properties, such as for example, epitope identity, paratope identity, antigen binding capacity etc., is the same or substantially similar to that of an antibody of interest, or without limitation thereto, an antibody as described in Example 6. Various methods such as epitope binning via competitive immunoassay may be used to sort antibodies or antigen-binding domains whose antigen-binding properties is the same or substantially similar into the same bin as described in para 0153. As used herein, it is understood by the skilled person in the art, that the methods described for obtaining or screening for antibodies whose antigen-binding properties is the same or substantially similar to that of an antibody of interest is equally applicable to a method to obtain or screen antigen-binding domains whose antigen-binding properties is the same or substantially the same as another antigen-binding domain, and the terms are used interchangeably.

In one embodiment, without limitation thereto, the present disclosure provides a method for obtaining or screening for antibodies that bind the same epitope as an antibody of interest, which involves firstly identifying the amino acid residues involved in binding between the antigen and the antibody of interest, then generating a library comprising the antigen and a plurality of variants of the antigens and lastly, screening libraries comprising antibodies and antigen-binding domains against the library of antigen and its variants, as further exemplified below.

In this method, a library comprising a plurality of variants of the antigen of interest is first prepared. Without limitation thereto, one or more amino acid mutations (insertions, deletions and/or substitutions) are introduced to one or more amino acid residues identified as sites that are involved in the antigen's binding to an antibody. In one embodiment, a single amino acid substitution is introduced to one residue identified as a site that is involved in the antigen's binding to an antibody.

In one embodiment, a library comprising a plurality of variants of the antigen, DDR2, is prepared comprising the introduction of one or more amino acid substitutions at one or more amino acid residues identified to be involved in the binding of DDR2 to an anti-DDR2 antibody. Accordingly, as described Example 6, the amino acid residues identified to be involved in the binding of mouse DDR2 to an anti-DDR2 antibody are, from the N-terminus, 31 to 179 of SEQ ID NO: 77. The amino acid residues identified to be involved in the binding of mouse DDR2 to an antibody are, from the N-terminus, R31, P33, S37, D64, S65, E66, Q103, H106, A107, G108, H110, S145, N146, Y148, D149, V150, F151, L152, D154 and R179 of SEQ ID NO: 77. SEQ ID NO: 77 corresponding to mouse DDR2 [Ref Seq No. NP_072075.2], A library comprising a plurality of variants of DDR2 is prepared by introducing one or more amino acid substitutions at one or more of amino acid positions 31 to 179 of SEQ ID NO: 77, and/or at one or more of the amino acids R31, P33, S37, D64, S65, E66, Q103, H106, A107, G108, H110, S145, N146, Y148, D149, V150, F151, L152, D154 and R179 of SEQ ID NO: 77.

In one embodiment, the present disclosure provides methods for obtaining, or screening for antibodies whose antigen-binding properties is the same or substantially similar to that of an antibody of interest, or an antibody as described in Example 6, using publicly known methods. In one embodiment, existing antibodies or antigen-binding domains or pre-existing libraries (phage libraries, etc.), or libraries prepared from hybridomas obtained by immunizing animals or from B cells of immunized animals, for example, antibodies or libraries prepared from immune cells such as B cells of animals immunized with a conjugate in which the antigen of interest, such as the extracellular domain (ECD) of mouse or human DDR2, which may be suitably linked to an adjuvant agent such as a highly immunogenic T cell epitope peptide.

In one embodiment of a method for screening antibodies whose antigen-binding properties is the same or substantially similar to that of an antibody of interest of the present disclosure, it is possible to use as a library, for example, a phage display library, a ribosome display library, an mRNA display library, a cDNA display library, a CIS display library, an E.coli display library, a Gram-positive bacterium display library, an yeast display library, a mammalian cell display library, a virus display library, and an in vitro virus display library. Methods to generate the abovementioned libraries and screen such libraries for antibodies possessing the desired binding characteristics is well known and also described in para 0114 - para 0116.

In one embodiment, in the method of panning, wherein an antigen of interest, is fixed onto a carrier such as beads. The antigen of interest, can be produced by, for example, a method of contacting the antigen of interest, synthesized to be chemically linked to biotin via a linker with beads or a plate onto which streptavidin or NeutrAvidin has been fixed, or a method of adhering the antigen of interest, covalently linked to an adjuvant such as bovine serum albumin (BSA) to beads or plates by hydrophobic interaction. These methods are already publicly known (J. Immunol. Methods. 2003 Sep, 280 (1-2): 139-55; BMC Biotechnol. 2009 Jan 29; 9: 6. doi: 10.1186/1472-6750-9-6). Antibodies having binding activity to the antigen of interest, can be prepared by collecting antibodies that have binding activity to the fixed antigen of interest.

In an alternate embodiment of the method for screening antibodies whose antigen-binding properties is the same or substantially similar to that of an antibody of interest of the present disclosure, by panning, a fluorescence-labeled antigen of interest, or a biotin-labeled antigen of interest and fluorescence-labeled streptavidin (or NeutrAvidin or avidin) may be used. Antibodies having binding activity to the antigen of interest can be prepared by contacting the fluorescence-labeled antigen of interest, or the biotin-labeled antigen of interest and fluorescence-labeled streptavidin (or NeutrAvidin or avidin), with a library presented on the cell surface or such, and then using the fluorescence-activated cell sorting (FACS) method. These methods are already publicly known (Proc Natl Acad Sci U S A. 2000 Sep 26; 97 (20): 10701-5).

The abovementioned library of antigen variants is used for panning against the abovementioned libraries of antibodies. Binding of antibodies to the antigen and/or antigen variant can be measured by a known method using Biacore, flow cytometry or such, without limitation thereto, and the binding activity (affinity) of the variant of the antigen of interest to an antibody is calculated as a KD value. For the avoidance of doubt, other suitable measurement methods may also be employed, KD value being discussed herein merely as an example.

The binding capacity (affinity) of each antibody to each antigen and/or antigen variant, such as its KD value, is determined. For example, the KD value or other suitable or equivalent measure of binding capacity (affinity) can be measured using known methods, such as Biacore or flow cytometry, and where the KD value is determined to be lower than a certain standard, such as, for example, a value lesser or smaller than 1 uM, 100 nM, 10 nM, 1 nM, 100 pM, 10 pM, or 1 pM, or $10^{-13}$ M, etc., binding of the antibody to the antigen and/or antigen variant is confirmed and can be represented by "o (circle)", and otherwise, indicated as "x (cross)". Where the antigen variant is represented by a "o (circle)", the amino acid residue that had been substituted in that antigen variant is considered an amino acid involved in the binding of an antibody and an antigen of interest, otherwise, the antigen variant is represented by a "x (cross)".

Accordingly, antibodies that share the same, or substantially the same binding capacity (affinity) to the same antigen and/or antigen variant can be identified, as illustrated in Table B below. In one example, Ab#1 and Ab#10 bind to an antigen comprising of the same amino acid modifications and are considered as antibodies that share the same antigen-binding properties. In another example, Ab#1 and Ab#7, each binding to an antigen, wherein the antigen comprises of substantially the same amino acid modifications and are considered as antibodies that share substantially the same antigen-binding properties. Antibodies Ab#1, Ab#7 and Ab#10 are considered as antibodies that share the same or substantially the same antigen-binding properties, or antibodies that bind to the same epitope, or antibodies that recognize the same epitope, or antibodies that compete for binding to the same epitope. As used herein, "antibodies that share the same or substantially the same antigen-binding properties", "antibodies that bind to the same epitope", or "antibodies that compete for binding to the same epitope" are used interchangeably.

[Table B]

| | Ab # | 1 | 2 | 3 | 4 | 5 | 6 | 7 | 8 | 9 | 10 |
|---|---|---|---|---|---|---|---|---|---|---|---|
| Amino acid modification | WT | o | o | o | o | o | o | o | o | o | o |
| | 11X | o | x | x | x | o | x | o | x | o | o |
| | 12X | x | o | x | x | x | x | x | o | o | x |
| | 13X | x | x | x | x | x | x | x | x | o | x |
| | 14X | o | x | o | x | o | o | o | x | o | o |
| | 15X | o | o | o | x | o | o | o | o | o | o |
| | 16X | x | x | o | o | o | o | x | o | o | x |
| | 17X | x | x | o | o | o | o | x | o | x | x |
| | 18X | o | x | o | x | o | o | o | o | x | o |
| | 19X | o | x | o | x | o | x | o | o | x | o |
| | 20X | x | x | x | x | o | x | o | o | o | x |

In one embodiment, the above method can be applied to identify alternative antibodies, also referred to as a "second antibody" herein, that share substantially similar antigen-binding properties, i.e. recognize and bind the same epitope on the antigen to a first antibody of interest. For example, a first antibody and a second antibody binds to one or more antigen or antigen variants, and wherein the amino acid sequence of said antigen or antigen variant(s) has 98.0%, 98.1%, 98. 2%, 98.3%, 98.4%, 98.5%, 98.6%, 98.7%, 98.8%, 98.9%, 99.0%, 99.1%, 99.2%, 99.3%, 99.4%, 99.5%, 99.6% 99.7%, 99.8%, 99.9% sequence identity to each other. For example, Ab #1 and Ab #7 in Table B.

The present disclosure provides antibodies that bind to the same epitope as or competes for binding to the same epitope of an antibody of interest, wherein such an antibody is one that share the same, or substantially similar antigen-binding properties with that of an antibody of interest obtained via the method for screening antibodies whose antigen-binding properties is the same or substantially similar to that of an antibody of interest described herein, for binding an antigen of interest. In one embodiment, the present disclosure includes anti-DDR2 antibodies that bind to the same epitope as or competes for binding to the same epitope of the anti-DDR2 antibodies identified in Example 6 for binding mouse DDR2. Without limitation, by performing the method for screening antibodies whose antigen-binding properties is the same or substantially similar described herein, the skilled person is able to identify antibodies that bind the same epitope as or competes for binding to the same epitope as an antibody of interest. In a preferred embodiment, the skilled person is able to generate and screen for anti-DDR2 antibodies that bind to the same epitope as or competed for binding to the same epitope as that of the anti-DDR2 antibodies identified in Example 6 for binding mouse DDR2.

In one embodiment, the present disclosure also includes anti-DDR2 antibodies that bind human DDR2. The skilled person following the aforementioned method will readily obtain alternative anti-DDR2 antibodies that share the same, or substantially similar antigen-binding properties with an anti-DDR2 antibody that binds human DDR2. Accordingly, considering the sequence similarity of extracellular region of mouse and human DDR2, the amino acid residues identified as involved in the binding of anti-DDR2 antibodies to mouse DDR2 as exemplified in Example 6, are considered correspondent to and are relevant for the further identification of alternative anti-DDR2 antibodies that bind to human DDR2 via the same epitope.

## D. Immunoconjugates

The invention also provides immunoconjugates comprising an anti-DDR2 antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $^{211}$At, $^{131}$I, $^{125}$I $^{90}$Y, $^{186}$Re, $^{188}$Re, $^{153}$Sm, $^{212}$Bi, $^{32}$P, $^{212}$Pb and radioactive isotopes of Lu. When the radioconjugate is used for detection, it may comprise a radioactive atom for scintigraphic studies, for example Tc-99m or $^{123}$I, or a spin label for nuclear magnetic resonance (NMR) imaging (also known as magnetic resonance imaging, MRI), such as iodine-123 again, iodine-131, indium-III, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionuclide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

## E. Methods and Compositions for Diagnostics and Detection

In certain embodiments, any of the anti-DDR2 antibodies provided herein is useful for detecting the presence of DDR2 in a biological sample. The term "detecting" as used herein encompasses quantitative or qualitative detection. In certain embodiments, a biological sample comprises a cell or tissue, such as fibrotic disease, including idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, and retroperitoneum fibrosis.

In one embodiment, an anti-DDR2 antibody for use in a method of diagnosis or detection is provided. In a further aspect, a method of detecting the presence of DDR2 in a biological sample is provided. In certain embodiments, the method comprises contacting the biological sample with an anti-DDR2 antibody as described herein under conditions permissive for binding of the anti-DDR2 antibody to DDR2, and detecting whether a complex is formed between the anti-DDR2 antibody and DDR2. Such method may be an *in vitro* or *in vivo* method. In one embodiment, an anti-DDR2 antibody is used to select subjects eligible for therapy with an anti-DDR2 antibody, e.g. where DDR2 is a biomarker for selection of patients.

Exemplary disorders that may be diagnosed using an antibody of the invention include idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, and retroperitoneum fibrosis.

In preferred embodiments, exemplary disorders that may be diagnosed using antibody of the invention include idiopathic pulmonary fibrosis (IPF), renal fibrosis, lung fibrosis/pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis.

In certain embodiments, labeled anti-DDR2 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}$P, $^{14}$C, $^{125}$I, $^{3}$H, and $^{131}$I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, those coupled with an enzyme that employs hydrogen peroxide to oxidize a dye precursor such as HRP, lactoperoxidase, or microperoxidase, biotin/avidin, spin labels, bacteriophage labels, stable free radicals, and the like.

## F. Pharmaceutical Formulations

Pharmaceutical formulations of an anti-DDR2 antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

## G. Therapeutic Uses, Methods and Compositions

Any of the anti-DDR2 antibodies provided herein may be used in therapeutic methods.

In one aspect, an anti-DDR2 antibody for use as a medicament is provided. In further aspects, an anti-DDR2 antibody for use in treating fibrotic disease is provided.

According to the present invention, the term "fibrotic disease" relates to diseases with the common feature of an uncontrolled and progressive accumulation of fibrotic tissue in affected organs causing their dysfunction and ultimate failure.

In further aspects, an anti-DDR2 antibody for use in treating idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis is provided.

In preferred embodiments, an anti-DDR2 antibody for use in treating idiopathic pulmonary fibrosis (IPF), renal fibrosis, lung fibrosis/pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis is provided.

In certain embodiments, an anti-DDR2 antibody for use in a method of treatment is provided. In certain embodiments, the invention provides an anti-DDR2 antibody for use in a method of treating an individual having fibrotic disease comprising administering to the individual an effective amount of the anti-DDR2 antibody. In certain embodiments, the invention provides an anti-DDR2 antibody for use in a method of treating an individual having idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis comprising administering to the individual an effective amount of the anti-DDR2 antibody. In one such embodiment, the method further comprises administering to the individual an effective amount of at least one additional therapeutic agent.

In certain embodiments, the invention provides an anti-DDR2 antibody for use in a method of reducing collagen type 1 alpha 1 (Col1a1) mRNA level in an individual comprising administering to the individual an effective of the anti-DDR2 antibody to reduce collagen type 1 alpha 1 (Col1a1) mRNA level. In certain embodiments, the invention provides an anti-DDR2 antibody for use in a method of reducing fibronectin 1 (Fn1) mRNA level in an individual comprising administering to the individual an effective of the anti-DDR2 antibody to reduce fibronectin 1 (Fn1) mRNA level.

An "individual" according to any of the above embodiments is preferably a human, and the present invention is particularly directed to the treatment of fibrotic disease, and particularly of the fibrotic diseases specified above, in human patients.

In a further aspect, the invention provides for the use of an anti-DDR2 antibody in the manufacture or preparation of a medicament. In one embodiment, the medicament is for treatment of fibrotic disease. In a further embodiment, the medicament is for use in a method of treating fibrotic disease comprising administering to an individual having fibrotic disease an effective amount of the medicament. In one embodiment, the medicament is for treatment of idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis. In a preferred embodiment, the medication is for treatment of treating idiopathic pulmonary fibrosis (IPF), renal fibrosis, lung fibrosis/pulmonary fibrosis, scleroderma lung disease, moderate-to-severe asthma, chronic obstructive pulmonary disease (COPD), acute respiratory distress syndrome (ARDS), rheumatoid arthritis-associated interstitial lung disease (RA-ILD), nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, cystic fibrosis, inflammatory bowel disease (IBD), or retroperitoneum fibrosis. In a further embodiment, the medicament is for use in a method of treating idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis comprising administering to an individual having corresponding disease an effective amount of the medicament.

In one such embodiment, the method further comprises administering to the individual an effective amount of at least one additional therapeutic agent. In a further embodiment, the medicament is for reducing collagen type 1 alpha 1 (Col1a1) mRNA level. In a further embodiment, the medicament is for use in a method of reducing collagen type 1 alpha 1 (Col1a1) mRNA level in an individual comprising administering to the individual an amount effective of the medicament to reduce collagen type 1 alpha 1 (Col1a1) mRNA level. In a further embodiment, the medicament is for reducing fibronectin 1 (Fn1) mRNA level. In a further embodiment, the medicament is for use in a method of reducing fibronectin 1 (Fn1) mRNA level in an individual comprising administering to the individual an amount effective of the medicament to reduce fibronectin 1 (Fn1) mRNA level. An "individual" according to any of the above embodiments may be a human.

In a further aspect, the invention provides a method for treating a fibrotic disease. In one embodiment, the method comprises administering to an individual having such fibrotic disease an effective amount of an anti-DDR2 antibody. In one such embodiment, the method further comprises administering to the individual an effective amount of at least one additional therapeutic agent. In a further aspect, the invention provides a method for treating idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis. In one embodiment, the method comprises administering to an individual having idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis an effective amount of an anti-DDR2 antibody. In one such embodiment, the method further comprises administering to the individual an effective amount of at least one additional therapeutic agent. An "individual" according to any of the above embodiments may be a human.

In a further aspect, the invention provides a method for reducing collagen type 1 alpha 1 (Col1a1) mRNA level in an individual. In one embodiment, the method comprises administering to the individual an effective amount of an anti-DDR2 antibody to reduce collagen type 1 alpha 1 (Col1a1) mRNA level. In a further aspect, the invention provides a method for reducing fibronectin 1 (Fn1) mRNA level in an individual. In one embodiment, the method comprises administering to the individual an effective amount of an anti-DDR2 antibody to reduce fibronectin 1 (Fn1) mRNA level. In one embodiment, an "individual" is a human.

In a further aspect, the invention provides pharmaceutical formulations comprising any of the anti-DDR2 antibodies provided herein, e.g., for use in any of the above therapeutic methods. In one embodiment, a pharmaceutical formulation comprises any of the anti-DDR2 antibodies provided herein and a pharmaceutically acceptable carrier. In another embodiment, a pharmaceutical formulation comprises any of the anti-DDR2 antibodies provided herein and at least one additional therapeutic agent, e.g., as described below.

Antibodies of the invention can be used either alone or in combination with other agents in a therapy. For instance, an antibody of the invention may be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-DDR2 antibody and administration of an additional therapeutic agent occur within about one month, or within about one, two or three weeks, or within about one, two, three, four, five, or six days, of each other.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 micro g/kg to 15 mg/kg (e.g. 0.1 mg/kg to 10 mg/kg) of antibody can be an initial candidate dosage for administration to the patient, whether, for example, by one or more separate administrations, or by continuous infusion. One typical daily dosage might range from about 1 micro g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the antibody). An initial higher loading dose, followed by one or more lower doses may be administered. The progress of this therapy is easily monitored by conventional techniques and assays.

It is understood that any of the above formulations or therapeutic methods may be carried out using an immunoconjugate of the invention in place of or in addition to an anti-DDR2 antibody.

## H. Articles of Manufacture

In another aspect of the invention, an article of manufacture (or a kit) containing materials useful for the treatment, prevention, and/or diagnosis of the disorders described above is provided. The article of manufacture comprises a container and a label on or a package insert associated with the container. Suitable containers include, for example, bottles, vials, syringes, IV solution bags, etc. The containers may be formed from a variety of materials such as glass or plastic. The container holds a composition which is by itself or combined with another composition effective for treating, preventing, and/or diagnosing the condition and may have a sterile access port (for example the container may be an intravenous solution bag or a vial having a stopper pierceable by a hypodermic injection needle). At least one active ingredient in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-DDR2 antibody.

Idiopathic Pulmonary Fibrosis (IPF) is a chronic disease characterized by loss of lung function due to lung fibrosis, which is hypothesized to be a result of abnormal extracellular matrix (ECM) remodeling and fibroblast proliferation. There is currently no cure for IPF and the current standard of care typically involves slowing down the progression of IPF using any of the only 2 approved drugs - Pirfenidone (Esbriet®) or Nintedanib (Ofev®). These medications are anti-fibrotic agents that slow down the rate of fibrosis or scarring in the lungs and are approved for patients with mild, moderate and severe IPF (NPL 12). Pirfenidone inhibits TGFbeta stimulated collagen production and reduces fibroblast proliferation (NPL 13), while Nintedanib binds PDGFR, FGFR and VEGFR, blocks autophosphorylation of these receptors, and prevents their downstream signaling cascades, resulting in the reduction of fibroblast proliferation (NPL 14). Meanwhile, Discoidin domain receptors (DDRs) is a lesser known family of the receptor tyrosine kinases (RTKs), which unlike other RTKs that respond to diffusible cytokines or growth factors such as TGFbeta, PDGF, FGF and VEGF, respond to the binding of collagens that are part of the extracellular matrices of cells. The DDRs are known to be involved in both physiological (growth, reproduction, cell microenvironment signaling), and pathological processes (tumor, bone malignancies, atherosclerosis, and fibrosis) (NPL 15). There have been reports that DDR2 play a role in fibrotic diseases (NPL 16), including a study using siRNA against DDR2 that exhibited therapeutic efficacy against lung fibrosis (NPL 11) and another study where DDR2 knockout led to reduced collagen production and decreased proliferation of fibroblasts but restoration of DDR2 signaling reversed the phenotype (NPL 10).

While there are some approved DDR2 targeting drugs approved to date, these are small molecule inhibitors that bind to the kinase domain, and not monoclonal antibodies which bind to the extracellular domains. Further, these small molecule inhibitors are not specific to DDR2 and are also effective inhibitors for DDR1 and other kinases due to the high sequence and structural homology between DDR2 and DDR1 kinase domains and other kinases (NPL 17). In addition, although there has been a report of a monoclonal antibody that specifically binds DDR2 that showed efficacy in the diagnosis and treatment of cancer (PTL1), there is no suggestion that said antibody would also be effective for the treatment of fibrosis. The inventors have developed monoclonal antibodies that bind to the extracellular domains of DDR2 and does not bind to DDR1 and additionally shown that these antibodies showed efficacy to reduce fibrosis in the unilateral ureteral obstruction (UUO) model and bleomycin (BLM)-induced mouse lung fibrosis model as exemplified below.

[Examples]

The following are examples of methods and compositions of the invention. It is understood that various other embodiments may be practiced, given the general description provided above.

[Example 1]

**Anti-DDR2 antibody generation**

Anti-DDR2 antibodies were prepared, selected and assayed as follows:

Three NZW rabbits were immunized with mixture of mouse DDR1 (NCBI Reference Sequence: NP_031610.1, SEQ ID NO: 79) plasmid and mouse DDR2 (NCBI Reference Sequence: NP_072075.2, SEQ ID NO: 77) plasmid coated gold particles (1 microgram (micro g) DNA per shot for total 40 shots) by Helios Gene Gun system (Bio-Rad) according to the manufacturer's instruction (Yuxin Chen et al. J. Virol. (2013) 87(18):10232). In addition, combination of intradermal administration (200 microgram DNA per site for total 2 sites at shoulders) and intramuscular administration (400 microgram DNA per site for total 2 sites at two legs) with mixture of mouse DDR1 plasmid and mouse DDR2 plasmid were carried out. The immunization was repeated weekly for 8 times followed by blood and spleen collection. For B cell selection, IgG positive and mouse DDR2 binding B cells were collected. B cells were sorted using a cell sorter and then plated and cultured according to the procedure described in WO2016098356A1. After cultivation, B cell culture supernatants were collected for further analysis and B cell pellets were cryopreserved.

Specific binding to mouse DDR2 (binding to mouse DDR2 but not to mouse DDR1) was checked by flow cytometry using selected B cell supernatant with mouse-DDR1-stably-expressing Ba/F3 cell line and mouse-DDR2-stably-expressing Ba/F3 cell line.

The RNAs of selected B cell lines were purified from the cryopreserved cell pellets using the ZR-96 Quick-RNA kits (ZYMO RESEARCH, Cat No. R1053). DNAs encoding antibody heavy-chain variable regions in the selected lines were amplified by reverse transcription PCR and recombined with DNA encoding antibody heavy chain constant region SG181 (SEQ ID NO:73). DNAs encoding antibody light-chain variable regions were also amplified by reverse transcription PCR and recombined with DNA encoding the antibody light-chain constant region k0MC (SEQ ID NO:74). Cloned antibodies were expressed in Expi293™ Expression System (Thermo Fisher Scientific) and purified from culture supernatants.

Table 1 shows the names and SED ID NOs of variable regions (including VH, VL, and HVR/CDR) of selected identified antibody for further evaluation.

[Table 1]

| Antibody Name | SEQ ID NO | | | | | | | |
|---|---|---|---|---|---|---|---|---|
| | VH | HVR-H1 | HVR-H2 | HVR-H3 | VL | HVR-L1 | HVR-L2 | HVR-L3 |
| DAB0065 | 1 | 2 | 3 | 4 | 5 | 6 | 7 | 8 |
| DAB0048 | 9 | 10 | 11 | 12 | 13 | 14 | 15 | 16 |
| DAB0060 | 17 | 18 | 19 | 20 | 21 | 22 | 23 | 24 |
| DAB0045 | 25 | 26 | 27 | 28 | 29 | 30 | 31 | 32 |
| DAB0069 | 33 | 34 | 35 | 36 | 37 | 38 | 39 | 40 |
| DAB0094 | 41 | 42 | 43 | 44 | 45 | 46 | 47 | 48 |
| DAB0071 | 49 | 50 | 51 | 52 | 53 | 54 | 55 | 56 |
| DAB0009 | 57 | 58 | 59 | 60 | 61 | 62 | 63 | 64 |
| DAB0052 | 65 | 66 | 67 | 68 | 69 | 70 | 71 | 72 |

[Example 2]

**Binding analysis of the anti-DDR2 monoclonal antibodies**

To determine antibody binding to mouse DDR2, flow cytometry analysis was carried out by using mouse-DDR2-stably-expressing Ba/F3 cell line. One-arm format antibodies, which contains one antibody variable region in each molecule, were prepared to evaluate the binding by affinity mode. Mean Fluorescent Intensity (MFI) of anti-DDR2 antibodies was measured and the result is showed in Figure 1.

To map more detailed binding epitope of anti-DDR2 antibodies, fragment of mouse DDR2, in which discoidin-like (DSL) domain and extracellular juxtamembrane (EJXM) region (Gln201-Val393) are deleted from full length mouse DDR2 (mouse DDR2 delta DSL-EJXM) was made. Mouse DDR2 delta DSL-EJXM stably-expressing Ba/F3 cell line was prepared. Binding of the antibodies to this cell line was measured by flow cytometry and MFI value was acquired. The result is shown in Figure 2. Epitope of antibodies which can bind to full length mouse DDR2 but not to mouse DDR2 delta DSL-EJXM would be DSL-EJXM region. Epitope of antibodies which can bind to both full length mouse DDR2 and mouse DDR2 delta DSL-EJXM would be discoidin (DS) domain. The binding results and estimated epitope location from this study were summarized in Table 2. In Table2, "Yes" means that tested antibody can bind to the cell line and "No" means that antibody cannot bind to the cell line.

Among the antibodies shown in Table 2, binding of DAB0009, DAB0045, DAB0065, DAB0069 and DAB0071 to human DDR2 were also evaluated and all of these antibodies showed binding to human DDR2. Binding of DAB0048, DAB0052, DAB0060 and DAB0094 to human DDR2 has not been evaluated yet, but they are likely to bind to human DDR2, according to the sequence similarity of extracellular region of mouse and human DDR2. Furthermore, considering the sequence similarity of extracellular region of mouse and human DDR2, the binding regions of the antibodies shown in Table 2 are considered to be correspondent in mouse and human DDR2.

[Table 2]

| Antibody Name | Binding to full length mouse DDR2 | Binding to mouse DDR2 delta DSL-EJXM | Binding region (epitope) |
|---|---|---|---|
| DAB0009 | Yes | No | DSL-EJXM |
| DAB0045 | Yes | Yes | DS |
| DAB0048 | Yes | Yes | DS |
| DAB0052 | Yes | No | DSL-EJXM |
| DAB0060 | Yes | Yes | DS |
| DAB0065 | Yes | Yes | DS |
| DAB0069 | Yes | Yes | DS |
| DAB0071 | Yes | No | DSL-EJXM |
| DAB0094 | Yes | Yes | DS |

[Example 3]

**Competitive binding analysis of the anti-DDR2 monoclonal antibodies**

Competitive epitope binning experiment was performed by real-time binding assay using Octet (Pall ForteBio). Recombinant mouse DDR2 ECD-Fc (R&D systems, Cat. No. 7479-DR-050) was biotinylated and captured onto streptavidin biosensor tips (Pall ForteBio) as antigen. The antigen captured tips were dipped into 15 or 40 microgram (micro g)/mL of a first set of antibodies for 300 seconds. Then the tips were incubated with 15 or 40 micro g/mL of a second set of antibodies for 300 seconds. To eliminate an effect of antibody dissociation, the antibody concentration in the second set was the same with that of the first set. Result was analyzed by DATA Analysis HT software (Pall ForteBio, Version 10.0.1.7). Positive response of the second set of antibodies indicate that the antibody in the second set does not compete with the antibody in the first set, in another word, the antibody in the second set binds to an epitope that is different from that of the antibody in the first set; and negative response of the second set of antibodies shows that the antibodies in the first and second sets compete with each other, in another word, the antibodies in the first and second set bind to the same epitope.

Antibodies that bind to DS domain of mouse DDR2 were tested and the results are shown in Table 3. In Table 3, "Ab1" indicates antibody in the first set and "Ab2" indicates antibody in the second set. Bold letter means that Ab1 and Ab2 of the corresponding cell compete with each other and do not bind to the antigen "in tandem", and thus they are located in the same epitope bin. As shown in Table 3, the tested antibodies were classified in to 3 different epitope bins: Bin 1 including DAB0048 and DAB0093, Bin 2 including DAB0060 and DAB0094, Bin 3 including DAB0045, DAB0065 and DAB0069.

[Table 3]

| | | Ab2 | | | | | | |
|---|---|---|---|---|---|---|---|---|
| | | DAB0048 | DAB0093 | DAB0060 | DAB0094 | DAB0045 | DAB0065 | DAB0069 |
| Ab1 | DAB0048 | **0** | **0.0114** | 0.82 | 1.4162 | 1.2047 | 1.648 | 1.5679 |
| | DAB0093 | **-0.0129** | **0** | 0.8565 | 1.4787 | 1.1299 | 1.6621 | 1.4959 |
| | DAB0060 | 1.3094 | 1.4529 | **0** | **0.0985** | 1.09 | 1.5112 | 1.3465 |
| | DAB0094 | 1.4965 | 1.492 | **-0.054** | **0** | 1.0685 | 1.552 | *1.3398 |
| | DAB0045 | 1.3242 | 1.4332 | 0.5914 | 1.2221 | **0** | **0.0398** | **0.0335** |
| | DAB0065 | 1.4924 | 1.4955 | 0.7657 | 1.3713 | **-0.0102** | **0** | **-0.0058** |
| | DAB0069 | 1.4697 | 1.4606 | 0.6147 | 1.3047 | **-0.001** | **0.0322** | **0** |

| Bin1 | | Bin2 | | Bin3 | | |
|---|---|---|---|---|---|---|

Table 3 shows the result of competitive binding analysis of the anti-DDR2 antibodies. Bold letter indicates that Ab1 and Ab2 correspond to the cell compete with each other. Antibodies compete with each other were classified into the same epitope bin. Data with * was obtained under 40 microgram (micro g)/mL of first and second set of antibodies

[Example 4]

## Anti-DDR2 antibodies prevented kidney fibrosis in UUO mouse model

The in vivo efficacy of the monoclonal anti-DDR2 antibodies was evaluated in UUO (unilateral ureteral obstruction) mouse model which develops progressive renal fibrosis.

Specific pathogen-free C57BL/6NTac male mice of 6-7 weeks of age were purchased from Invivos Pte Ltd (Singapore) and were acclimated for 1 weeks before the start of treatments. Animals were maintained at 20 - 26 degrees C with a 12:12 hour light/dark cycle and fed with a commercial standard diet (5P75; PMI Nutrition INT'L (LabDiet), Missouri, United States) and tap water ad libitum.

UUO surgery was operated under isoflurane anesthetized condition. The left side of the abdomen was shaved, and a vertical incision was made through the skin. A second incision was made through the peritoneum and that skin was also retracted to reveal the kidney. Using forceps, the kidney was brought to the surface and the left ureter was tied with surgical silk, twice, below the kidney. The ligated kidney was placed gently back into its correct anatomical position then peritoneum and skin were sutured. Analgesic agent was added to reduce animal affliction. In sham operated group, peritoneum and skin were only incised and sutured.

All monoclonal antibodies were administered by intraperitoneal injection two times, one day after the surgical operation and one day before the harvest. For sham operated group, vehicle was administered. The animals were weighed and then killed by exsanguination under isoflurane anaesthesia on day 4. Blood samples were collected from the heart cavities or the postcaval vein and maintained at -80 degrees C until assayed. The kidney was quickly removed. Part of the kidney tissue was snap-frozen in liquid nitrogen or on dry ice for molecular analyses.

Total RNA was extracted from kidney tissues using RNeasy Mini Kit (Qiagen). Mouse mitochondrial ribosomal protein L19 (Mrpl19) was used as the endogenous reference for each sample. Antibodies' efficacy was evaluated by measuring collagen type 1 alpha 1 (Col1a1) mRNA levels in kidney. Relative mRNA expression values were calculated using double delta Ct analysis.

As shown in Figure 3, UUO mice showed significant increase in Col1a1 mRNA level. DAB0048 and DAB0065 showed significant efficacy in reducing Col1a1 mRNA in UUO mice.

Data are presented as mean $\pm$ standard error of the mean (SEM). Statistical analysis was performed using Student's t-test and Dunnett's multiple comparison test. When P values were of <0.05, differences were considered significant.

[Example 5]

**Anti-DDR2 antibodies prevented lung fibrosis in BLM mouse model**

In vivo efficacy of anti-DDR2 monoclonal antibodies (DABs) and anti-mature TGFbeta antibody (GC1008) were evaluated in bleomycin (BLM)-induced mouse lung fibrosis model (BLM mouse model), which is characterized by the infiltration of leukocytes, fibroblast proliferation, and an increase in collagen within the lung tissue. This model is the best characterized and most widely used pre-clinical model which reproduces many aspects of idiopathic pulmonary fibrosis (NPL 18, NPL 19, NPL 20).

Specific pathogen-free C57BL/6NTac male mice of 6-7 weeks of age were purchased from Invivos Pte Ltd (Singapore) and were acclimated for 1 weeks before the start of treatments. Animals were maintained at 20 - 26 degrees C with a 12:12 hour light/dark cycle and fed with a commercial standard diet (5P75; PMI Nutrition INT'L (LabDiet), Missouri, United States) and tap water ad libitum.

Bleomycin Sulfate (Gold Bio, St Louis, US, Cat.No. B-910-50) or saline were administered through Osmotic minipump (Alzet, Cupertino, CA, USA, MODEL 1007D). On day 0, minipumps were loaded with BLM (50 mg/kg) dissolved in 90 microliter (micro-L) of saline. Saline was administered for a non-diseased control group. Minipumps were incubated at 37 degrees C for 3-4 hours before implantation. Pumps were implanted subcutaneously (s.c.) at the back through an incision under isoflurane anesthesia. On day 10, minipumps were removed from mice under isoflurane anesthesia. All monoclonal antibodies were administered 25 mg/kg by intravenous injection from day14 once every 4 to 3 days. Anti-KLH antibody IC17dK was used as a negative control in this study. The animals were weighed and then killed by exsanguination under isoflurane anaesthesia on Day 28. Blood samples were collected from the heart cavity or the postcaval vein and housed at -80°C until assayed. The lung was quickly removed and weighed. Part of the lung tissue was snap-frozen in liquid nitrogen or on dry ice for molecular analyses.

Total RNA was extracted from lung tissues using RNeasy Mini Kit (Qiagen). Mouse mitochondrial ribosomal protein L19 (Mrpl19) or glyceraldehyde-3-phosphate dehydrogenase (Gapdh) was used as the endogenous reference for each sample. Antibody's efficacy was evaluated by measuring collagen type 1 alpha 1 (Col1a1) and fibronectin 1 (Fn1) mRNA levels in the lung. Relative mRNA expression values were calculated using double delta Ct analysis.

As shown in Figure 4A, BLM mice showed significant increase in Col1a1 and Fn1 mRNA level. DAB0065 showed significant efficacy in reducing Col1a1 and Fn1 mRNA in BLM mice. On the other hand, the rest of the antibodies DAB0045, DAB0069 and DAB0048 did not show significant efficacy in reducing Col1a1 and Fn1 mRNA in BLM mice as shown in Figure 4B.

Data are presented as mean $\pm$ standard error of the mean (SEM). Statistical analysis was performed using Student's t-test. When P values were of <0.05, differences were considered significant. "ns": not significant.

[Example 6]

**Epitope Analysis for the DAB0065 antibody**

6-1 X-ray crystal structure analysis of mouse DDR2 in complex with DAB0065 antibody

The three-dimensional structure of the complex of mouse DDR2 and monoclonal antibody DAB0065 was revealed by X-ray crystallographic analysis.

6-2. Expression and purification of discoidin domain (22-190) of mouse DDR2 (moDDR2 DS domain)

Extracellular domain of mouse DDR2 (22-190) with N-terminal His tag and FLAG tag (His-FLAG-moDDR2) was transiently expressed using Expi293 expression system (Thermo Fisher Scientific). The His-FLAG-moDDR2 from culture medium was purified by affinity chromatography (HisTrap™ excel, Cytiva) followed by size exclusion chromatography (HiLoad 16/600 Superdex 200 pg, Cytiva). His tag and FLAG tag fused at the N-terminus were cleaved with Enterokinase (Roche) and the resultant moDDR2 DS domain was further purified with Anti-FLAG M2 Affinity Gel (Sigma-Aldrich) to remove uncleaved protein and gel filtration column (Superdex™ 75 Increase 10/300 GL, Cytiva). Fractions containing moDDR2 DS domain were pooled and stored at -80°C.

6-3. Preparation of Fab fragment of DAB0065 (DAB0065 Fab)

DAB0065 for crystal structure analysis was transiently transfected and expressed using an Expi293 Expression system (Thermo Fisher Scientific). The culture supernatant was harvested, and DAB0065 was purified from the supernatant using MabSelect SuRe affinity chromatography (Cytiva) followed by gel filtration chromatography using (HiLoad 26/600 Superdex 200 pg Cytiva). Fab fragment of DAB0065 was prepared by the conventional method using limited digestion with FabULOUS® (Genovis), followed by loading onto a Protein A column (MabSlect SuRe, Cytiva) to remove Fc fragments, a cation exchange column (HiTrap SP HP, Cytiva), and a gel filtration column (Superdex™ 75 Increase 10/300 GL, Cytiva). Fractions containing DAB0065 Fab were pooled and stored at -80°C.

6-4. Crystallization, data collection and structure determination of DAB0065 Fab and moDDR2 DS domain complex

Purified moDDR2 DS domain was mixed with DAB0065 Fab. The complex was purified by gel filtration column (Superdex™ 75 Increase 10/300 GL, Cytiva) with 20 mM HEPES pH 7.1, 100 mM NaCl. The purified complex was concentrated to about 8.49 mg/mL, and crystallization was carried out by the sitting drop vapor diffusion method at 4°C. The reservoir solution consisted of 27.0 %w/v EDO_P8K, 90 mM Morpheus buffer 3 (pH 8.5), 81 mM Morpheus Halogens (Molecular Dimensions).

X-ray diffraction data were collected by BL45XU at SPring-8. During the measurement, the crystal was constantly placed in a nitrogen stream at 100K to maintain it in a frozen state, and a total of 3600 X-ray diffraction images were collected using a PILATUS3 6M (DECTRIS) attached to a beam line, while rotating the crystal 0.1° at a time. Determining the cell parameters, indexing the diffraction spots, and processing the diffraction data obtained from the diffraction images were performed using the autoPROC program (Acta. Cryst. 2011, D67: 293-302), XDS Package (Acta. Cryst. 2010, D66: 125-132), and AIMLESS (Acta. Cryst. 2013, D69: 1204-1214), and finally the diffraction intensity data up to 1.437 Å resolution was obtained. The crystallography data statistics are shown in Table 4.

The structure was determined by molecular replacement with the program Phaser (J. Appl. Cryst. 2007, 40: 658-674) using homology model of DAB0065 built by MOE (Chemical Computing Group) and predicted structure of moDDR2 DS domain built by AlphaFold from UniProt (AF-Q62371-F1) as search models. A model was built with the Coot program (Acta Cryst. 2010, D66: 486-501) and refined with the program PHENIX (Acta Cryst. 2010, D66: 213-221). The structure refinement statistics are shown in Table 4.

[Table 4] X-ray data collection and refinement statistics

| Data collection and refinement statistics. | |
|---|---|
| **Data collection** | |
| X-ray source | SPring-8 / BL45XU |
| Wavelength (Å) | 1.00000 |
| Space group | $P2_1 2_1 2_1$ |
| Cell dimensions $a, b, c$ (Å) | 88.245, 112.178, 139.634 |
| Cell dimensions $\alpha, \beta, \gamma$ (°) | 90.0, 90.0, 90.0 |
| Resolution (Å) | 87.452 − 1.437 (1.602 − 1.437)* |
| $R_{pim}$ | 0.030 (0.401)* |
| $CC(1/2)$ | 0.999 (0.796)* |
| mean $I/\sigma(I)$ | 14.6 (1.8)* |
| Total observations | 1057696 (53468)* |
| Unique observations | 155813 (7792)* |
| Completeness, spherical (%) | 62.1 (11.3)* |
| Completeness, ellipsoidal (%) | 96.1 (77.5)* |
| Redundancy | 6.8 (6.9)* |
| **Refinement** | |
| Resolution (Å) | 69.82 − 1.44 |
| No. of reflections | 155759 |
| $R_{work}/R_{free}^{a}$ | 20.36 / 23.71 |
| r.m.s.d. Bond lengths (Å) | 0.006 |
| r.m.s.d. Bond angles (°) | 0.963 |
| Ramachandran plot[b] | |
| Outliers (%) | 0.00 |
| Allowed (%) | 2.82 |
| Favored (%) | 97.18 |
| Rotamer Outliers (%)[b] | 0.20 |
| * Values in parentheses are for the highest-resolution shell. [a] $R_{free}$ is calculated with 5% of the reflection randomly set aside. [b] Calculated with the program MolProbity. | |

6-5. Identification of the interaction sites of DAB0065 Fab and moDDR2 DS domain

The crystal structure of DAB0065 Fab and moDDR2 DS domain complex was determined at 1.44 Å resolution. The Fab fragment of DAB0065 bound to the DS domain in 1:1 ratio, and the asymmetric unit of the crystal structure contained two complexes, Molecule-1 and Molecule-2, as depicted in Fig. 5A. Molecule-1 and Molecule-2 can be aligned well at 0.313 Å RMSD with Calpha atom position in all the residues, as shown in Fig. 5B. Figures 5 and 6 discussed here were prepared using Molecule-1.

In Fig. 6A and 6B, the epitope of the DAB0065 Fab contact region is mapped in the moDDR2 DS domain amino acid sequence and in the crystal structure respectively. The epitope includes the amino acid residues of moDDR2 DS domain that contain one or more non-hydrogen atoms located within 5.0 Å distance from any part of the DAB0065 Fab in the crystal structure.

As shown in Fig. 6B, the crystal structure analysis revealed that the flat surface of moDDR2 DS domain is mainly recognized by the flat surface formed by HCDR3, LCDR1 and LCDR2 of DAB0065. In addition, the interactions between small convex which is formed by Y148 and D149 of DDR2 and shallow concave, which is formed by HCDR1, 2 and 3 are also observed and considered to be required for antibody and antigen recognition.

Accordingly, considering the very high sequence similarity of the DS domain of extracellular region of mouse and human DDR2 (see SEQ ID NO: 77 and 78), the amino acid residues identified as involved in the binding of anti-DDR2 antibodies to mouse DDR2 as exemplified in this example, are considered correspondent for the binding of anti-DDR2 antibodies to human DDR2 via the same epitope.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

**Claims**

1. An isolated antibody that binds to Discoidin Domain Receptor 2 (DDR2).

2. The isolated antibody of claim 1, wherein the antibody binds to the extracellular region of DDR2, in particular wherein the antibody binds to discoidin domain (DS domain), discoidin-like domain (DSL domain) or extracellular juxtamembrane domain (EJXM domain) of DDR2.

3. The antibody of any one of claims 1 or 2, wherein the antibody does not bind to DDR1.

4. The antibody of any one of claims 1 to 3, wherein the antibody binds DDR2 at an epitope comprising all or part of a sequence defined by amino acids 31 to 179 of SEQ ID NO: 77 or 78.

5. The antibody of claim 4, wherein the antibody binds to a discontinuous epitope comprising amino acids R31, P33, S37, D64, S65, E66, Q103, H106, A107, G108, H110, S145, N146, Y148, D149, V150, F151, L152, D154 and R179 of SEQ ID NO: 77, or R31, P33, S37, D64, S65, E66, Q103, H106, A107, G108, H110, S145, N146, Y148, D149, 1150, F151, L152, D154 and R179 of SEQ ID NO: 78, in particular wherein the antibody binds to a discontinuous epitope comprising amino acids P33, S37, D64, S65, E66, Q103, H106, G108, H110, S145, N146, Y148, D149, V150, F151, and L152 of SEQ ID NO: 77, or P33, S37, D64, S65, E66, Q103, H106, G108, H110, S145, N146, Y148, D149, 1150, F151, and L152 of SEQ ID NO: 78.

6. The antibody of any one of claims 1 to 5, wherein the antibody comprises HVR-H1 comprising the amino acid sequence of SEQ ID NO: 2, HVR-H2 comprising the amino acid sequence of SEQ ID NO:3, HVR-H3 comprising the amino acid sequence of SEQ ID NO:4, HVR-L1 comprising the amino acid sequence of SEQ ID NO: 6, HVR-L2 comprising the amino acid sequence of SEQ ID NO:7, and HVR-L3 comprising the amino acid sequence of SEQ ID NO:8.

7. The antibody of claim 6, comprising a VH sequence having the amino acid sequence of SEQ ID NO:1 and a VL sequence having the amino acid sequence of SEQ ID NO:5.

8. An antibody binding to the same epitope as the antibody of any of claims 6 or 7 or which competes with the antibody of any of claims 6 or 7 in binding towards DDR2.

9. The antibody of any one of claims 1 to 8, which is a human, humanized, or chimeric antibody.

10. An isolated nucleic acid encoding the antibody of any one of claims 1 to 9.

11. A host cell comprising the nucleic acid of claim 10.

12. A method of producing an antibody comprising culturing the host cell of claim 11 so that the antibody is produced.

13. The antibody of any one of claims 1 to 9 for use as a medicament.

14. The antibody of any one of claims 1 to 9 for use in reducing collagen type 1 alpha 1 (Col1a1) mRNA level and/or fibronectin 1 (Fn1) mRNA level in a subject.

15. The antibody of any one of claims 1 to 9 for use in treating fibrotic disease.

16. The antibody for use of claim 15, wherein the fibrotic disease is idiopathic pulmonary fibrosis (IPF), renal fibrosis, liver fibrosis, skin fibrosis, hepatic cirrhosis, lung fibrosis/pulmonary fibrosis, myocardial fibrosis, ocular fibrosis, myelofibrosis, age-related macular degeneration (AMD), diabetic retinopathy, proliferative vitreoretinopathy (PVR), glaucoma, diabetic macular edema (DME), dry eye disease, scleroderma lung disease, moderate-to-severe asthma, chronic obstructive pulmonary disease (COPD), acute respiratory distress syndrome (ARDS), rheumatoid arthritis-associated interstitial lung disease (RA-ILD), cardiac scarring post-MI, cardiomyopathy, congestive heart failure, Alcoholic cirrhosis, hepatitis B (HBV), hepatitis C (HCV), schistosomiasis, non-alcoholic steatohepatitis (NASH), primary biliary cholangitis (PBC), primary sclerosing cholangitis (PSC), transplant nephropathy, diabetic nephropathy, lupus nephritis, focal segmental glomerulosclerosis, Alport's syndrome, interstitial fibrosis, hypertrophic scarring, keloids, scleroderma, nephrogenic systemic fibrosis, chronic graft vs. host disease, atherosclerosis, cystic fibrosis, surgical-induced fibrosis, burn-induced scarring & contraction, radiation-induced fibrosis, muscular dystrophy, inflammatory bowel disease (IBD), mediastinal fibrosis, or retroperitoneum fibrosis, in particular wherein the fibrotic disease is a lung fibrotic disease, more in particular idiopathic pulmonary fibrosis (IPF).

[Fig. 1]

mouse DDR2 binding

[Fig. 2]

mouse DDR2 delta DSL-EJXM

[Fig. 3]

**Col1a1 mRNA**

Mean ±SEM *Student's t-test vs UUO IC17dK, **p < 0.01
#Dunnett's multiple comparison test vs UUO IC17dK, #p < 0.05

[Fig. 4a]

**Col1a1 mRNA**

**Fn1 mRNA**

Mean ±SEM *Student's t-test vs BLM IC17dK, *p < 0.05, **p < 0.01

**Col1a1 mRNA**

**Fn1 mRNA**

Mean ±SEM *Student's t-test vs BLM IC17dk, *p < 0.05, ***p < 0.001

[Fig. 4b]

EP 4 155 321 A1

[Fig. 5a]

[Fig. 5b]

moDDR2 DS(22-190)

[Fig. 6a]

| 2 | | | | | | | | | | 3 | | | | | | | | | | 4 | | | | | | | | | | 5 | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 |
| – | – | K | A | Q | V | N | P | A | I | C | R | Y | P | L | G | M | S | G | G | H | I | P | D | E | D | I | T | A | S | S | Q | W | S | E | S | T | A | A | K |
|  |  | × | × | × | × | × | × |  |  | ▨ |  | ■ |  |  | ■ |  |  |  |  |  |  |  |  |  |  |  |  |  |  |  |  |  |  |  |  |  |  |  |  |

| 6 | | | | | | | | | | 7 | | | | | | | | | | 8 | | | | | | | | | | 9 | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 |
| Y | G | R | L | D | S | E | E | G | D | G | A | W | C | P | E | I | P | V | Q | P | D | D | L | K | E | F | L | Q | I | D | L | R | T | L | H | F | I | T | L |
|  |  |  |  | ■ | ■ | ■ |  |  |  |  |  |  |  |  |  |  |  |  |  |  |  |  |  |  |  |  |  |  |  |  |  |  |  |  |  |  |  |  |  |

| 10 | | | | | | | | | | 11 | | | | | | | | | | 12 | | | | | | | | | | 13 | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 |
| V | G | T | Q | G | R | H | A | G | G | H | G | I | E | F | A | P | M | Y | K | I | N | Y | S | R | D | G | S | R | W | I | S | W | R | N | R | H | G | K | Q |
|  |  |  | ■ |  |  | ■ | ▨ |  | ■ |  |  |  |  |  |  |  |  |  |  |  |  |  |  |  |  |  |  |  |  |  |  |  |  |  |  |  |  |  |  |

| 14 | | | | | | | | | | 15 | | | | | | | | | | 16 | | | | | | | | | | 17 | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|
| 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 | 0 | 1 | 2 | 3 | 4 | 5 | 6 | 7 | 8 | 9 |
| V | L | D | G | N | S | N | P | Y | D | V | F | L | K | D | L | E | P | P | I | V | A | R | F | V | R | L | I | P | V | T | D | H | S | M | N | V | C | M | R |
|  |  |  |  |  | ■ | ■ |  | ■ | ■ | ■ | ■ | ■ |  | ▨ |  |  |  |  |  |  |  |  |  |  |  |  |  |  |  |  |  |  |  |  |  |  |  |  | ▨ |

| 18 | | | | | | | | | | 19 | | | | | | | | | |
|---|---|---|---|---|---|---|---|---|---|---|---|---|---|---|---|---|---|---|---|
| 0 | 1 | 2 | 3 | 4 | 5 | 6 | 7 | 8 | 9 | 0 | 1 | 2 | 3 | 4 | 5 | 6 | 7 | 8 | 9 |
| V | E | L | Y | G | C | V | W | L | D | G | – | – | – | – | – | – | – | – | – |
|  |  |  |  |  |  |  | × | × | × |  |  |  |  |  |  |  |  |  |  |

× disordered
■ < 4.2 Å from Heavy and Light chain
▨ < 5.0 Å from Heavy and Light chain

[Fig. 6b]

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

**EP 22 17 6993**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/070090 A2 (ONCOMED PHARM INC [US]; LEWICKI JOHN [US] ET AL.) 12 June 2008 (2008-06-12) | 1-7,9-13 | INV. C07K16/28 A61K39/395 |
| Y | * abstract * <br> * example 1 * <br> * example 3 * <br> * figure 2 * | 14-16 | |
| X | BIAN HUAN ET AL: "The pronounced high expression of discoidin domain receptor 2 in human interstitial lung diseases", ERJ OPEN RESEARCH, vol. 4, no. 1, 16 January 2018 (2018-01-16), pages 00138-2016, XP093023685, DOI: 10.1183/23120541.00138-2016 * abstract * <br> * page 3, paragraph 4 * | 1-5 | |

-----

-----

-/--

**TECHNICAL FIELDS SEARCHED (IPC)**

A61K
C07K

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 14 February 2023 | Malamoussi, A |

EPO FORM 1503 03.82 (P04E07)

Europäisches
Patentamt

European
Patent Office

Office européen
des brevets

# PARTIAL EUROPEAN SEARCH REPORT

**Application Number**

EP 22 17 6993

## DOCUMENTS CONSIDERED TO BE RELEVANT

| Category | Citation of document with indication, where appropriate, of relevant passages | Relevant to claim |
|---|---|---|
| X | HU ZHAO ET AL: "Targeting of Discoidin Domain Receptor 2 (DDR2) Prevents Myofibroblast Activation and Neovessel Formation During Pulmonary Fibrosis", MOLECULAR THERAPY, 27 May 2016 (2016-05-27), XP055299676, US ISSN: 1525-0016, DOI: 10.1038/mt.2016.109 | 1-5 |
| Y | * abstract * * page 10, left-hand column, paragraph 5 * | 14-16 |
| A | M. BARDELLI ET AL: "Epitope mapping by solution NMR spectroscopy : EPITOPE MAPPING BY SOLUTION NMR SPECTROSCOPY", JOURNAL OF MOLECULAR RECOGNITION., vol. 28, no. 6, 27 February 2015 (2015-02-27), pages 393-400, XP055684054, GB ISSN: 0952-3499, DOI: 10.1002/jmr.2454 * page 393, right-hand column - page 394, left-hand column, paragraph 1 * | 1-7,9-16 |
| A | Lilit Yeon Simonyan: "Conformational Epitope Mapping by Cross-link Mass Spectrometry: Analysis of Ipilimumab, Nivolumab and Pembrolizumab", , 15 November 2017 (2017-11-15), XP055593280, DOI: 10.1038/ncomms13354 Retrieved from the Internet: URL:https://covalx.com/pdf/171113-CovalX-X LMS%20Xray%20Comparions-PEGSEU17.pdf [retrieved on 2019-06-03] * figure "Comparison with X-ray crystallographic data" * | 1-7,9-16 |

**CLASSIFICATION OF THE APPLICATION (IPC)**

**TECHNICAL FIELDS SEARCHED (IPC)**

EPO FORM 1503 03.82 (P04C10)

1

Europäisches
Patentamt

European
Patent Office

Office européen
des brevets

**INCOMPLETE SEARCH
SHEET C**

Application Number

EP 22 17 6993

Claim(s) completely searchable:
        1-7, 9-16

Claim(s) not searched:
        8

Reason for the limitation of the search:

Claim 8 aims at providing an antibody binding to the same epitope as the antibody of any of claims 6 or 7 or which competes with the antibody of any of claims 6 or 7 in binding towards DDR2 (emphasis added).
Claim 8 tries to define the antibody by unusual parameters and is therefore not searchable for following reasons:
In order to fall within (or outside) the claimed scope a given antibody must bind (or must not bind) to the exact same epitope as the antibody of claim 6 or 7, and in order to fulfill the requirements of Art. 84 EPC the skilled person has to be able to unambiguously determine whether said exact same epitope is being bound or not.
In the underlying case, the application/description does not provide an unambiguous definition of the term "epitope", and does not explain which assay is to be used to determine such epitope-specificity. The functional property of "binding the same epitope" and indeed determining which concrete epitope is bound by a given antibody may be established in multiple different manners, e.g. by alanine-scanning, peptide-mapping or protein-crystallography, all of which lead to different conclusions of which minimal amino acids must at least be present to form the given epitope. It follows that as long as it is not clear, by which assay the functional parameter of "binding the same epitope" is to be determined the skilled person cannot unambiguously determine the scope presently claimed (GL F-IV, 4.11 & 4.18). The subject-matter of claim 8 does not meet the requirements of Art. 84 EPC.
Moreover, the feature of "competes for binding" is a functional feature which could never have been disclosed in the prior art, since it relates to competition to an antibody which is for the first time disclosed in present application. Therefore, it is considered to be an unusual parameter since the applicant's choice of said parameter to define the invention renders a meaningful comparison with the prior art impossible, because the unusual parameter measures a property of the antibody that was not measured before in the field of the invention. For this reason, the parameter chosen by the applicant lacks clarity (see GL F-IV, 4.11.1). The onus of proof that the unusual parameter is a genuine distinctive feature vis-à-vis the prior art lies with the applicant. No benefit of doubt can be accorded in this respect (see F-IV, 4.11.1 and G-VI, 6). The lack of clarity and support and disclosure of such antibodies is to such an extent, that a search of  claim 8 is meaningless.
The applicant replied that  claim 8 is to to be interpreted as being directed at a competing antibody, which binds the epitope as defined in claim 4 or 5. Moreover, the applicant submitted that the description, at least in paragraphs [0152] to [0156] and [0167] to [0192], provides all the necessary disclosure for the skilled person to determine if an antibody in the prior art would compete with the antibody of the present invention.
The search division does not agree with this interpretation of the claim,

**Europäisches Patentamt**
**European Patent Office**
**Office européen des brevets**

## INCOMPLETE SEARCH
## SHEET C

**Application Number**

**EP 22 17 6993**

since 1) claim 8 is dependent on claims 6 and 7, which are themselves dependent also on claims 1-4, where no concrete epitope is defined. It is noted that different epitope mapping techniques will provide for different epitopes. Moreover, claim 4 does not define an epitope, it only discloses that the epitope comprises all or part of a sequence defined by amino acids 31-179 of SEQ ID No 77 or 78. This is not considered to be a clear definition of a defined epitope (also in view of the term "comprises" in claim 4). In addition, it is considered that competition to binding is not equivalent to binding to the same epitope as the competed antibody. In fact, an antibody binding to an adjacent or even a distant epitope may also exert competition.
Consequently, the present search has been restricted to the subject matter of claims 1-7 and 9-16.

## ANNEX TO THE EUROPEAN SEARCH REPORT
## ON EUROPEAN PATENT APPLICATION NO.

EP 22 17 6993

This annex lists the patent family members relating to the patent documents cited in  the above-mentioned European search report.
The members are as contained in the European Patent Office EDP file on
The European Patent Office is in no way liable for these particulars which are merely given for the purpose of information.

14-02-2023

| Patent document cited in search report | | Publication date | Patent family member(s) | | Publication date |
|---|---|---|---|---|---|
| WO 2008070090 | A2 | 12-06-2008 | EP | 2099491 A2 | 16-09-2009 |
| | | | US | 2008171045 A1 | 17-07-2008 |
| | | | US | 2014186342 A1 | 03-07-2014 |
| | | | WO | 2008070090 A2 | 12-06-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008070090 A
- US 5500362 A
- US 5821337 A
- US 6737056 B, Presta
- US 6194551 B1
- WO 199951642 A
- WO 200492219 A, Hinton
- WO 200042072 A, Presta
- WO 9316185 A
- US 5571894 A
- US 5587458 A
- US 5869046 A
- EP 404097 A
- WO 199301161 A
- US 6248516 B1
- US 4816567 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- WO 5821337 A
- WO 2006029879 A
- WO 2005100402 A
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A

- US 5770710 A
- US 5773001 A
- US 5877296 A
- US 6630579 B
- WO 9411026 A
- US 4737456 A
- US 20050260186 A

- US 20060104968 A
- US 6267958 B
- US 6171586 B
- WO 2006044908 A
- WO 2016098356 A1
- DD 222190

**Non-patent literature cited in the description**

- **W. VOGEL ; G. D. GISH ; F. ALVES ; T. PAWSON.** The discoidin domain receptor tyrosine kinases are activated by collagen. *Molecular cell,* 1997, vol. 1, 13-23
- **SHRIVASTAVA et al.** An orphan receptor tyrosine kinase family whose members serve as nonintegrin collagen receptors. *Molecular cell,* 1997, vol. 1, 25-34
- **S. BAUMGARTNER ; K. HOFMANN ; R. CHIQUET-EHRISMANN ; P. BUCHER.** The discoidin domain family revisited: new members from prokaryotes and a homology-based fold prediction. *Protein science : a publication of the Protein Society,* 1998, vol. 7, 1626-1631
- **M. A. LEMMON ; J. SCHLESSINGER.** Cell signaling by receptor tyrosine kinases. *Cell,* 2010, vol. 141, 1117-1134
- **B. LEITINGER.** Molecular analysis of collagen binding by the human discoidin domain receptors, DDR1 and DDR2. Identification of collagen binding sites in DDR2. *The Journal of biological chemistry,* 2003, vol. 278, 16761-16769
- **C. M. BORZA ; A. POZZI.** Discoidin domain receptors in disease. *Matrix biology : journal of the International Society for Matrix Biology,* 2014, vol. 34, 185-192
- **B. LEITINGER.** Discoidin domain receptor functions in physiological and pathological conditions. *Int Rev Cell Mol Biol,* 2014, vol. 310, 39-87
- **T. A. WYNN ; T. R. RAMALINGAM.** Mechanisms of fibrosis: therapeutic translation for fibrotic disease. *Nat Med,* 2012, vol. 18, 1028-1040
- **F. ALVES et al.** Distinct structural characteristics of discoidin I subfamily receptor tyrosine kinases and complementary expression in human cancer. *Oncogene,* 1995, vol. 10, 609-618
- **E. OLASO et al.** Discoidin Domain Receptor 2 Regulates Fibroblast Proliferation and Migration through the Extracellular Matrix in Association with Transcriptional Activation of Matrix Metalloproteinase-2. *Journal of Biological Chemistry,* 2002, vol. 277, 3606-3613
- **H. ZHAO et al.** Targeting of Discoidin Domain Receptor 2 (DDR2) Prevents Myofibroblast Activation and Neovessel Formation During Pulmonary Fibrosis. *Mol Ther,* 2016, vol. 24, 1734-1744

- **K. ROACH et al.** Evaluation of Pirfenidone and Nintedanib in a Human Lung Model of Fibrogenesis. *Front. Pharmacol.,* 2021, vol. 12, 679388
- **E. CONTE et al.** Molecular mechanisms of pirfenidone activity in human lung fibroblasts. *European Respiratory Journal,* 2014, vol. 44, 825
- **L. WOLLIN et al.** Mode of action of nintedanib in the treatment of idiopathic pulmonary fibrosis. *European Respiratory Journal,* 2015, vol. 45, 1434-1445
- **L. CHEN et al.** Recent Advances in the Role of Discoidin Domain Receptor Tyrosine Kinase 1 and Discoidin Domain Receptor Tyrosine Kinase 2 in Breast and Ovarian Cancer. *Front. Cell Dev. Biol,* 2021, vol. 9, 747314
- **S. JIA et al.** Discoidin Domain Receptor 2 Signaling Regulates Fibroblast Apoptosis through PDK1/Akt. *American Journal of Respiratory Cell and Molecular Biology,* 2018, vol. 59 (3), 295-305
- **WA DENNY et al.** Inhibitors of Discoidin Domain Receptor (DDR) Kinases for Cancer and Inflammation. *Biomolecules,* 2021, vol. 11 (11), 1671
- **J TASHIRO et al.** Exploring Animal Models that Resemble Idiopathic Pulmonary Fibrosis. *Front Med,* 2017, vol. 4, 118
- **T LIU et al.** The Bleomycin Model of Pulmonary Fibrosis. *Methods Mol Biol,* 2017, vol. 1627, 27-42
- **D TOREN et al.** Systems biology analysis of lung fibrosis-related genes in the bleomycin mouse model. *Sci Rep,* 2021, vol. 11, 19269
- **RAVETCH ; KINET.** *Annu. Rev. Immunol,* 1991, vol. 9, 457-92
- **CLYNES et al.** *PNAS (USA),* 1998, vol. 95, 652-656
- **GAZZANO-SANTORO et al.** *J. Immunol. Methods,* 1996, vol. 202, 163
- **IDUSOGIE et al.** *J. Immunol.,* 2000, vol. 164, 4178-4184
- **NILVEBRANT J ; ROCKBERG J.** An Introduction to Epitope Mapping. *Methods Mol Biol.,* 2018, vol. 1785, 1-10
- **KABAT et al.** Sequences of Proteins of Immunological Interest. Public Health Service, National Institutes of Health, 1991
- **DAËRON.** *Annu. Rev. Immunol.,* 1997, vol. 15, 203-234

- **CAPEL et al.** *Immunomethods,* 1994, vol. 4, 25-34
- **HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- **KABAT et al.** Sequences of Proteins of Immunological Interest. NIH Publication, 1991, vol. 1-3, 91-3242
- **CHOTHIA ; LESK.** *J. Mol. Biol.,* 1987, vol. 196, 901-917
- **MACCALLUM et al.** *J. Mol. Biol.,* 1996, vol. 262, 732-745
- **FLATMAN et al.** *J. Chromatogr. B,* 2007, vol. 848, 79-87
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537

- **TRAUNECKER et al.** *EMBO J,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Epitope Mapping Protocols, Third Edition. **ROCKBERG ; NILVEBRANT.** Part of the Methods in Molecular Biology. Humana Press, 2018, vol. 1785
- *J. Immunol. Methods,* September 2003, vol. 280 (1-2), 139-55
- *BMC Biotechnol.,* January 2009, vol. 29 (9), 6
- *Proc Natl Acad Sci U S A.,* 26 September 2000, vol. 97 (20), 10701-5
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- Remington's Pharmaceutical Sciences. 1980
- *Acta. Cryst.,* 2011, vol. D67, 293-302
- *Acta. Cryst.,* 2010, vol. D66, 125-132
- *Acta. Cryst.,* 2013, vol. D69, 1204-1214
- *J. Appl. Cryst.,* 2007, vol. 40, 658-674